# EUROPEAN PATENT SPECIFICATION

(11) **EP 0 290 565 B1**
(45) Date of publication and mention of the grant of the patent: **17.04.1996**
(21) Application number: 87907920.0
(22) Date of filing: 27.11.1987
(51) Int. Cl.: C07K 2/00, C07K 7/06, C07K 7/08, C07K 14/00, C07H 21/04, C12N 1/20, C12N 15/00, C12P 21/02

(54) **VACCINE CONTAINING TICK ANTIGENS**
IMPFSTOFF, DER ZECKEN-ANTIGENE ENTHÄLT
VACCIN CONTENANT DES ANTIGENES DE TIQUES

(30) Priority: 27.11.1986 AU 9196/86; 19.06.1987 AU 2570/87; 16.10.1987 AU 4912/87
(43) Date of publication of application: 17.11.1988
(73) Proprietor: BIOTECHNOLOGY AUSTRALIA PTY. LTD., East Roseville, NSW 2069 (AU); COMMONWEALTH SCIENTIFIC AND INDUSTRIAL RESEARCH ORGANISATION, Campbell, Australian Capital Territory 2601 (AU)
(72) Inventor: COBON, Gary, Stewart, Frenchs Forest, NSW 2086 (AU); MOORE, Joanna, Terry, Mosman, NSW 2088 (AU); JOHNSTON, Law, Anthony, York, River Hills, QLD 4074 (AU); WILLADSEN, Peter, Chapel Hill, QLD 4069 (AU); KEMP, David, Harold, Upper Brookfield, QLD 4069 (AU); SRISKANTHA, Alagacone, Chatswood, NSW 2067 (AU); RIDING, George, Alfred, Indooroopilly, QLD 4068 (AU); RAND, Keith, Norman, Frenchs Forest, NSW 2068 (AU)
(74) Representative: Bannerman, David Gardner
(86) International application number: AU8700401
(87) International publication number: WO8803929

(56) References cited:
- AU-A- 4 593 685
- AU-A- 5 970 786
- AU-B- 1 645 983
- GB-A- 2 142 334

## Description

This invention relates to an antigen isolated from the cattle tick *Boophilus microplus* and to the gene coding for that antigen and to the protein product of that gene. The antigen when used in part or in entirety as an immunogen administered to cattle as a vaccine induces immunity to infestation of a mammalian host to which it has been administered, characterized in that the immunity results in the mammalian host producing an immune response which damages the plasma membrane of the gut cells of ticks feeding on said host to such an extent that the majority of said ticks fail to survive to adult stage, or surviving ticks become red in colour and the reproductive capacity of said surviving ticks is substantially decreased or the engorgement weight of said surviving ticks is decreased.

### BACKGROUND ART

On first infestation with ticks such as the cattle tick, *Boophilus microplus,* animals such as cattle are very susceptible to the parasite. Typically about 50% of the tick larvae which attach, complete the full life cycle to eventually drop off as engorged adults. On prolonged exposure to the parasite, cattle acquire some degree of immunological resistance to it, but this resistance reaches a relatively stable level at which economically important losses to cattle production still occur. The losses to production are largely due to losses of blood and tissue fluid taken up by the parasite during feeding. Additional losses are due to the hypersensitive or allergic response which animals develop to tick salivary and cement antigens in conjunction with natural immunity, a condition known as tick worry.

A large number of approaches are used to control ticks. The most widely used is treatment of cattle with acaracides - chemicals which kill ticks. This approach has several shortcomings. For example resistance to the chemicals arises in the tick population and new classes of chemicals must be introduced frequently. The chemicals have little residual effect so cattle must be treated frequently in order to control the ticks effectively. The chemicals may have detrimental effects on the cattle, personnel and the environment. A second method for control of ticks is to breed for host resistance. Zebu breeds and Zebu cross breeds are more resistant to ticks than the highly susceptible British breeds. However, Zebu crosses have behavioural problems, are less. productive than pure British breeds and, even with the use of chemicals, the degree of resistance to ticks is far from ideal. Other methods of tick management such as pasture spelling and tick eradication present practical problems in most cattle producing areas throughout the world. An effective vaccine against ticks would provide a highly attractive alternative to the currently available methods of tick control.

Intermittent attempts have been made in the past to immunise animals against ticks. (1-5, see 13 for review) The majority of these studies have used tick-host systems in which strong immunity seems to develop naturally, and have usually used laboratory animals as hosts. Usually the effects observed have been some reduction in engorgement weights and egg masses of adult ticks and some decrease in the pliability of those eggs (1-5) although in two reports some decrease in the viability of engorging adults has been reported (3,4). Many of these studies have used antigens derived from salivary glands in order to attempt to mimic natural immunity. However it is unlikely that a vaccine which mimics natural immunity would be of great commercial benefit due to the economic losses which still occur once natural immunity has been expressed and the deleterious effect of hypersensitivity responses to ticks.

The alternative approach is to vaccinate animals with "concealed" or "novel" antigens, "Concealed" or "novel" antigens are, in this context, components of the parasite which can be used to raise a protective immune response in animals when used (in a partially or fully purified form) to vaccinate those animals, but are antigens which are not involved in naturally acquired immunity.

The successful vaccination against ticks using concealed or novel antigens has been reported (2,5). Animals were immunised with extracts of whole ticks or tick midgut. Immunization led to reductions in tick engorgement weights, feeding period, egg masses and egg viability but no significant increase in tick mortality was observed. However, the antigen fractions used in these experiments were so complex that it was not possible to identify the individual tick antigens which were responsible for the effects noted and the reasons for the effects were not investigated in detail.

In a recent patent application (Australian Patent Application No. 59707/86), claims are made that antigens derived from the synganglia of ticks can act as effective vaccines against tick infestation. However, there is no evidence presented in that patent that synganglia antigens can be effective alone. In this work dissected guts and synganglia were isolated, the gut cells were lysed, centrifuged and both the supernatant and pellet were used to vaccinate the same animals together, in some cases, with a cell suspension of synganglia. All cattle in the experiments reported were vaccinated with tick gut components and some received synganglia in addition. Therefore, it is clearly implicit in the experimental design that gut damage as a result of an immune response against gut components of ticks such as the gut cell antigens described herein and in the CSIRO patent application (45936/85), is an essential prerequisite for any secondary protective effects which may possibly result from an immune response against synganglia-specific antigens.

In all of the examples cited above, the tick extracts which were used to vaccinate the animals were extremely complex. In the majority of the reports the fractions used were homogenates of tick organs and in some cases, the pellets derived therefrom by centrifugation. In this and the other studies, no data on the complexity of the fractions is presented but it is certain that they must contain many hundreds and probably thousands of components. In the one study where any purification and characterization of the protective fraction was carried out (Australian patent application No. 45936/85) the most highly purified fraction, GF 5/6 was still very complex as will be shown below and it was not possible from this work to identify the individual component(s) of this fraction which were responsible for the protective immune response. In the present invention one such antigen is purified and characterized.

Boophilus microplus presents a particularly challenging problem. Since the naturally-acquired immunity is only partially effective, duplication of natural immunity by artificial immunization would be of comparatively little commercial value. Boophilus microplus is a parasite of cattle and does not feed readily on laboratory animals. The possibility of inducing "unnatural immunity" to Boophilus microplus has been examined and shown to be possible (6, 7, 8, Australian Patent Application No. 45936/85). The practical exploitation of this, however, would require as a first step the isolation of the antigen or antigens responsible, and as a second step, the development of means by which the effective antigens could be produced in quantities which would be sufficient for commercial uses.

The initial steps in the purification of the antigens in question and the demonstration of the efficacy of these antigens has been described previously (Australian Patent Application No. 45936/85). Briefly, ticks removed from cattle were disrupted, and sonicated-, the cuticles and debris removed by low speed centrifugation, the supernatant was subjected to high speed centrifugation at 100 000 x g for 1 hour, the membrane enriched pellet was extracted with a non-ionic detergent, the extract was subjected sequentially to chromatography on Sephacryl S-300 columns, broad range isoelectric focussing, narrow range isoelectric focussing and gel filtration chromatography on HPLC. At each step, fractions obtained were tested for efficacy as immunogens and the most highly protective fractions subjected to the next purification step. The most highly protective antigens were thus identified as being membranous, possessing an isoelectric point (pI) of between 5.05 and 5.65 and molecular weights in the range 205 to 79 kilodaltons. Other less highly protective fractions were also described and are of interest in both this and the preceding Australian Patent Application 45936/85.

Further development of the purification procedure as described herein has enabled the most highly protective antigens to be more clearly defined and characterised more precisely and has enabled animals to be vaccinated with more highly purified immunogen preparations. One such antigen has been purified to near homogeneity and it has been shown that when cattle are vaccinated with this tick component an immune response is generated in those cattle which results in the death of the majority of ticks used to challenge those vaccinated animals. The antigen isolated from ticks has been shown to be a glycoprotein with a molecular weight of approximately 89 kilodaltons and an isoelectric point in the range of 5.30 to 5.67. The method for the purification of this glycoprotein (referred to hereafter as the WGL⁺ antigen or WGL⁺) has been improved and a method is disclosed herein which results in a much larger yield of the antigen than could be obtained by the method previously described (Aust. Patent Application No. 45936/85). During this and previous work, other fractions which give protection have been identified.

Having devised means by which the WGL⁺ antigen can be obtained in larger amounts (not sufficient for commercial uses), experiments have been performed to analyse the structure of parts of the protein portion of the antigen. The purified preparation was reduced and carboxy-methylated and digested with endoproteinase lys-C. The peptide fragments so produced were purified and the partial amino acid sequence determined for some peptides. This amino acid sequence data has enabled the design of oligonucleotides which have been used to isolate bacterial cells containing cDNA coding for the WGL⁺ antigen.

Analysis of the DNA from these bacterial cells leads to the unambiguous identification of the gene coding for one protective antigen and the production of recombinant proteins which can be used as effective vaccines against ticks. These developments are the subject of the present invention.

### DEFINITIONS

Whilst the invention provides products and processes suitable for the protection of cattle against tick infestation, it is to be understood that the principles of the invention can be equally applied to the protection of other animals such as horses, deer, goats, sheep, dogs, cats and pigs against tick infestation.

It is recognised that the tick population worldwide is genetically diverse as is the case for all organisms which reproduce sexually. Each individual of a population differs subtly from the others in the population and these differences are a consequence of differences in the sequence of the DNA which each individual inherits from its parents.

Further, random mutational events which can occur in either sexually or asexually reproducing organisms are a furtner source of genetic variation.

Thus for each gene encoding a particular protein, there are likely to be differences in tne sequence among the population of individuals.

Such related molecules are referred to herein as homologues of antigens according to the invention and to the extent that they fulfill the functions of immunogens as defined herein they are included within the scope of the invention.

Homologous antigens may be defined as antigens related by evolution but not necessarily by function. Similar but not necessarily identical DNA or protein sequences may be provided. It should be noted however that function in this sense relates to the natural in vivo function of the protein.

Illustration of this point is provided by considering:
1. WGL⁺ from Boophilus microplus and other tick species
2. WGL⁺ from variants or different individuals of the Boophilus microplus population
3. WGL⁺ and related gut cell plasma membrane glycoproteins from ticks which are homologues of the WGL⁺ antigen defined herein.

It is stressed that for the purposes of this invention, homologues include only those WGL⁺ related plasma membrane glycoproteins which function as immunogens as defined herein.

Such homologous WGL⁺ related plasma membrane glycoproteins may exist in the tick population worldwide and will be capable, when incorporated into a vaccine, of eliciting in animals vaccinated with those antigens an immune response which is capable of killing ticks, by damaging tick gut cells and which additionally results in a reduction in tick engorgement weights or otherwise damaging the surviving ticks in such a way that for example egg production by those ticks is decreased to such an extent that the vaccine can be used commercially agains infestation by tick species such as Boophilus spp, Haemaphysalis spp, Otobius spp, Rhiphicephalus spp, Ambylomma spp, Dermacentor spp, Ixodes spp and Hyalomma spp, and especially from B. annulatus, B. decoloratus, Otobius megnini, Rhiphicephalus appendiculatus, Dermacentor andersoni, D. variabilis, Haemaphysalis longicornis, Ambylomma variegatum and Ixodes holocyclus.

Further, it should be recognised that it is possible to generate chemicals which are not related to the WGL⁺ antigen by evolution or necessarily by structure but which may serve as immunogens to generate an immune response against protective epitopes on the WGL⁺ antigen and thereby act as effective vaccines. These molecules are referred to herein as analogues and to the extent that they fulfill the functions of immunogens as defined herein, they are included within the scope of the invention. Such analogues include chemically synthesized oligopeptide molecules with sequences corresponding to portions of the amino acid backbone of the WGL⁺ molecule, oligopeptides which when used as immunogens elicit an immune response which recognises native WGL⁺ antigen in ticks, carbohydrate structures from whatever source which when used as antigens elicit an immune response which recognises the WGL⁺ antigen in ticks, and anti-idiotype antibodies raised against the variable region of antibodies which recognise the epitope(s) of the WGL⁺ antigen.

### DISCLOSURE OF THE INVENTION

In a first embodiment the invention provides an isolated antigen, WGL⁺, having substantially the amino acid sequence 
10 20 30 40 50
MRGIALFVAA VSLIVEGTAE SSICSDFGNE FCRNAECEVV PGAEDDFVCK
60 70 80 90 100
CPRDNMYFNA AEKQCEYKDT CKTRECSYGR CVESNPSKAS CVCEASDDLT
110 120 130 140 150
LQCKIKNDYA TDCRNRGGTA KLRTDGFIGA TCDCGEWGAM NMTTRNCVPT
160 170 180 190 200
TCLRPDLTCK DLCEKNLLQR DSRCCQGWNT ANCSAAPPAD SYCSPGSPKG
 
210 220 230 240 250
PDGQCINACK TKEAGFVCKH GCRSTGKAYE CTCPSGSTVA EDGITCKSIS
260 270 280 290 300
HTVSCTAEQK QTCRPTEDCR VHKGSVLCEC PWNQHLVGDT CISDCVDKKC
310 320 330 340 350
HEEFMDCGVY MNRQSCYCPW KSRKPGPNVN INECLLNEYY YTVSFTPNIS
360 370 380 390 400
FDSDHCKWYE DRVLEAIRTS IGKEVFKVEI LNCTQDIKAR LIAEKPLSKH
410 420 430 440 450
VLRKLQACEH PIGEWCMMYP KLLIKKNSAT EIEEENLCDS LLKDQEAAYK
460 470 480 490 500
GQNKCVKVDN LFWFQCADGY TTTYEMTRGR LRRSVCKAGV SCNENEQSEC
510 520 530 540 550
ADKGQIFVYE NGKANCQCPP DTKPGEIGCI ERTTCNPKEI QECQDKKLEC
560 570 580 590 600
VYKNHKAECE CPDDHECYRE PAKDSCSEED NGKCQSSGQR CVIENGKAVC
610 620 630 640 650
KEKSEATTAA TTTTKAKDKD PDPGKSSAAA VSATGLLLLL AATSVTAASL
660 670
 *GRCPT*IRN SLNMYIYITL TSNT*LGF and parts and homologues thereof, which antigen parts and homologues induce immunity to infestation of mammalian host to which said parts or homologues have been administered, characterized in that said immunity results in the mammalian host producing an immune response which damages the plasma membrane of the gut cells of ticks feeding on said host to such an extent that the majority of said ticks fail to survive to adult stage, or surviving ticks become red in colour and the reproductive capacity of said surviving ticks is substantially decreased or the engorgement weight of said surviving ticks is decreased.

Preferably the antigen is derived from *Boophilus microplus.*

In a preferred embodiment the immunity induced is immunity to infestation by a *Boophilus* species.

More preferably the immunity induced is immunity to *B. microplus* infestation.

However, immunity may also be induced to other species of ticks, including *Haemaphysalis* spp, *Otobius* spp, *Rhiphicephalus* spp, *Ambylomma* spp, *Dermacentor* spp, *Ixodes* spp and *Hyalomma* spp and especially to other species of Boophilus such as *B. annulatus* or *B. decoloratus.*

Of other species of ticks against which immunity can be induced, preferred species include *Otobius megnini, Rhiphicephalus appendiculatus, Dermacentor andersoni, D. variabilis, Haemaphysalis longicornis, Ambylomma variegatum* and *Ixodes holocyclus.*

By immunization with related antigens isolated from other species of ticks including *Boophilus* spp, *Haemaphysalis* spp, *Otobius* spp, *Rhiphicephalus* spp, *Ambylomma* spp, *Dermacentor* spp, *Ixodes* spp and *Hyalomma* spp, immunity to infestation by other ticks may also be induced. Preferred species from which the related antigens are isolated include *B. annulatus* or *B. decoloratus, Otobius megnini, Rhiphicephalus appendiculatus, Dermacentor andersoni, D. variabilis, Haemaphysalis longicornis, Ambylomma variegatum* and *Ixodes holocyclus.* By protecting against infestation with ticks, the antigen may also provide protection against diseases caused by agents such as *Babesia bovis, Babesia bigemina, Anaplasma marginale, Cowdria ruminantium, Theileria parva parva, T. parva lawrencii, T. annulata* and *T. hirci.*

In a second embodiment the invention provides a polynucleotide sequence which acts as a coding sequence for amino acid sequences of an antigen of the first embodiment or a part or homologue thereof of the first embodiment with the proviso that the polynucleotide sequence does not include a naturally occurring polynucleotide sequence as it exists in its native environment.

Preferably the polynucleotide sequence is a DNA sequence.

In a further preferred form of the invention the DNA sequence is a cDNA sequence.

The DNA sequence coding for part or all of the protective antigen isolated from *Boophilus microplus* can be used in DNA hybridization experiments to identify related DNA sequences from other species of ticks. These latter DNA sequences can be constructed by genetic engineering techniques to obtain the expression by bacterial or eukaryote cells such as yeast, plant, insect, tick or mammalian cell lines of all or parts of the antigen from other species of ticks and provide an effective vaccine against those tick species which are responsible for morbidity or economic losses to man or morbidity and productivity losses to animals.

The invention also provides a recombinant DNA molecule which comprises at least one DNA sequence according to the invention and vector DNA.

In a preferred form the vector DNA comprises plasmid, phage or viral DNA.

Preferred vectors include lambda gt11, pUR290, pUR291, pUR282, pUK270, pUC8, pUC9, baculovirus, pZipNeo, an SV40 based vector, lambda gt10, an EMBL vector, pBR327, pBR329, or pBR329 containing a par locus.

The invention further provides a transformant cell line, said transformant carrying at least one recombinant DNA molecule according to the invention.

In a further embodiment the invention provides a vaccine comprising at least one antigen according to the invention together with a pharmaceutically acceptable carrier, adjuvant, immunopotentiator or diluent.

In accordance with the present invention an antigen derived from a tick species which antigen is capable of inducing a highly significant degree of immunity to tick challenge when used to vaccinate cattle has been purified and characterised. Further, bacterial cells which contain DNA sequences derived from a tick species have been produced and those bacterial cells which contain DNA sequences encoding portions of the tick protective antigen have been identified. The DNA sequence of the tick gene encoding that antigen has been determined, the resulting DNA sequence has been used to identify further bacterial cells containing related genes from other species of ticks. Expression of the antigen or portions of the antigen by bacteria or other microrganisms or by eukaryotic cells such as yeast, insect, tick, plant and mammaliam cells grown in vitro provides a large amount of the antigen effective as an immunogen for the protection of cattle and other domestic animals against infestation by Boophilis microolus and other tick species.

The invention also includes within its scope the epitope or the epitopes of antigens of the invention which are responsible for the protective immune response. These epitopes may be created artificially by the synthetic production of oligopeptides which contain sequences of portions of the protective antigen which can be predicted from the results of immunochemical tests on fragments of the protective antigen produced in bacteria or generated as a result of chemical or enzymatic cleavage of the native or recombinant peptides and includes relevant epitopes from those protective antigens, oligopeptides, idiotypes and anti-idiotypes which resemble or recognise those epitopes which may have protective effects when used to actively or passively immunise animals.

In a further embodiment the invention provides methods for the purification of antigens according to the invention and particularly protective antigens derived from ticks.

The invention provides a process for the preparation of an antigen according to the invention which process comprises a chromatographic step performed on wheat germ lectin or on a lectin having the same or similar terminal sugar specificity as wheat germ lectin.

Preferably the invention provides a process for the preparation of an antigen according to the invention said process comprising extracting memorane enriched fractions obtained from homogenised ticks with detergent and subjecting the solubilised material to wheat germ lectin sepharose chromatography and elution with N-acetylglucosamine or chromatography using a lectin having the same or similar terminal sugar specificity to wheat germ lectin.

Preferably said detergent is selected from NP40, an NP40 derivative, Ewittergent 3-14 or SDS.

The process may further comprise Concanavalin-A sepharose chromatography and elution with methyl-α-D-,mannopyranoside, a preparative isoelectrofocussing step or size exclusion chromatography.

In a preferred form said methods include preparation of an homogenate of ticks centrifugation to produce membrane enriched fractions, treatment of those membranes with detergents such as Zwittergent 3-14, chromatography of the detergent soluble material on lectin affinity columns such as wheat germ lectin-Sepharose 68 columns, separation of the lectin binding antigens by isoelectric focusing in buffers containing detergent such as Zwittergent 3-14, chromatography of these antigens by size exclusion HPLC on columns such as Bio-Sil TSK 4,000 and PP 300 SW columns in series in buffers containing detergents and analysis of various fractions produced by SDS-polyacrylamide gel electrophoresis.

The invention also provides an antigen produced by a process according to the invention. Included within the scope of an antigen produced by a process according to the invention are those antigens produced as a result of purification schemes performed on native materials and recombinant or synthetic immunogens produced as a result of recombinant DNA or chemical synthetic methods respectively.

In a further embodiment, the invention provides examples of methods for the treatment of the purified antigens with proteolytic enzymes such as endo lys-C, the purification of oligopeptide fragments produced as a result of proteinase digestion by HPLC chromatography on columns such as Aquapore RP-300 C-8 or Aquapore RP-318 columns and determination of the amino acid sequence of some of the oligopeptides so produced and purified.

The invention further provides the peptide sequence information for such peptide fragments including:

### FRAGMENT NUMBER

F1 (K) D P D P G K

F2 (K) W Y E D (G) V L E A I (X) T S I G K

F3 (K) (X) Q A C E (H) P I G E (W) C M M Y P K

(C)
F4 (K) E A G F V Q K

(S) (V) (V) (I)
F5 (K) G (P) (D) G Q (C) I N (A) (C) K

(G)
F6 (K) A (D) V S T N E N E Q L E Q A D K

(G)
F7 (K) S (D) T Q (X) I D H I S K

N (A) (A)
F8 (K) D Q E (Y) (Y) Y

F9 [(K) C P C D N M Y F N A A E E I G C I E ]
[(K) A N R Q C P P D T R R G K ]

F10 [(K) A P R Q N M Y F N A A E E I G C I E ]
[(K) C N C D C P P D T R P G K ]

F11 (K) W Y E D R V L E A I R T S I G K

F12 (K) E S S I C X D F G N E F C R N A E C E V V P

F13 (K) T R E C S Y G R C V E S N P S K

F14 (K) A Y E C T C P R A F T V A E D G I (S/H) C K

[(K) D E V D N A (S/H) L V C Q N A]
F15 [(K) N V L Q S D G C G P Y]
[(K) C L N P R(P/L) R L K (H/S)]

S
F16 (K) A X V L C E X P
C
G

F17 (K) L Q A C E H P I

NOTE: Amino acids which were ascribed with low confidence are bracketed. X indicates no amino acid could be ascribed to this position; [] denotes mixed sequences.

In a preferred embodiment of the invention, these peptide sequences are:

F1 K D P D P G K
F2, F11 K W Y E D R V L E A I R T S I G K
F3, F17 K L Q A C E H P I G E W C M M Y P K
F4 K E A G F V C K
F5 K G P D G Q C I N A C K
F6 K A G V S C N E N E Q S E C A D K
F8 K D Q E A A Y K
F9, F10 K C P R D N M Y F N A A E K
K A N C Q C P P D T K P G E I G C I E
K A N C Q C P P D T R P G E I G C I E
F12 A E S S I C S D F G N E F C R N A E C E V V P G
F13 K T R E C S Y G R C V E S N P S K
F14 K A Y E C T C P S G S T V A E D G I T C K
K A Y E C T C P R A F T V A E D G I T C K
F15 K N L L Q R D S R C C Q
F16 K G T V L C E C P

The invention also provides examples of methods which can be used to design from the amino acid sequence data oligonucleotide sequences which are suitable for use as Oybridization probes to identify nucleic acid sequences (DNA or RNA) coding for the polypeptide containing those amino acid sequences, methods for the construction of bacterial cells containing complimentary DNA and genomic DNA fragments from ticks, the use of the oligonucleotides to identify bacterial cells containing complementary and genomic DNA fragments coding for that antigen, the DNA sequence of one such cDNA fragment, methods by which recombinant DNA technology can be used to produce bacterial or eukaryote cells which synthesize the protein or parts of that protein an methods for culturing those cells and for purification of the tick antigen or parts thereof to be incorporated into effective vaccines against ticks.

In a preferred model, the mechanism of action of the vaccine is one in which an immune response is generated in vaccinated animals which results in ticks feeding on those animals ingesting components of the host immune system such as antibodies which interact with the surface of tick gut cells and either alone, or together with other factors in the host blood such as components of complement result in damage occuring such as lysis of the tick gut cells which in turn results in the ticks becoming unable to effectively digest blood, the tick gut becoming permeable to host blood components, to such an extent that host blood components such as albumin, haemoglobin, immunoglobulin and blood cells can be identified in the haemoloymph of the ticks and the ticks appear red in colour. This gut damage in turn results in the death of the majority of the ticks feeding on vaccinated animals before they reach engorgement stage and those few which may survive are so badly damaged that their engorgement weight is decreased and/or reproductive capacity is impaired (6,7,8).

The invention also relates to antibodies generated against epitopes on the antigens according to the invention (so called idiotype antibodies) and to antibodies generated against the variable region of those first antibodies, (so called anti-idiotype antibodies) which mimic the protective epitopes on the antigen and may be used as effective vaccines in either passive protection of animals (idiotypes) or active immunization of animals (anti-idiotypes) and thereby result in effective protection.

### BRIEF DESCRIPTION OF THE DRAWINGS

Figure 1 is a schematic representation of the method for the isolation of the "WGL post IEF pool" and "LL Post IEF pool".

Figure 2 is a schematic representation of the fractionation procedure for the isolation of "LL⁺ antigen".

Figure 3 is a schematic representation of the fractionation procedure for the isolation of "WGL⁺ antigen".

Figure 4 is a simplified schematic representation of the purification procedure for the isolation of WGL⁺ and LL⁺ antigens.

Figure 5 shows purity of the WGL⁺ antigen.

Figure 6 shows schematic flow diagram for the synthesis of cDNA.

Figure 7 shows the DNA sequence for the WGL⁺ gene.

Figure 8 shows the translated amino acid sequence for the WGL⁺ antigen deduced from the DNA sequence.

Figure 9 shows a restriction enzyme map for part of the WGL⁺ gene showing an example of the expression strategy.

Figure 10 shows a SDS polyacrylamide gel and immunoblot demonstrating expression of WGL⁺ by bacteria.

Figure 11 shows hybridization of the Boophilus microplus DNA coding for WGL⁺ to DNA from other tick species.

### BEST MODE OF CARRYING OUT THE INVENTION

The invention is further described in the following examples which are illustrative of the invention but in no way limiting on its scope.

### SOURCE OF REAGENTS

- Sephacryl: Pharmacia
- Sepharose 6 MB: Pharmacia
- Zwittergent 3-14: Calbiochem
- Sephadex: Pharmacia
- Brij 35: Sigma
- Bio-gel: Bio Rad
- Cyanogen Bromide: Sigma or Ajax
- Sarkosyl: Sigma
- Endoproteinase lys-c: Boehringer
- Triflouroacetic acid: Pierce
- HFBA: Pierce
- Acetonitrile: Mallinckrodt
- Columns for HPLC: Waters, BioRad, Beckman
- Poly U Sepharose: Collaborative research
- Oligo dT cellulose: Collaborative research
- dATP, dCTP, dGTP and dTTP: Boehringer
- ³P-labelled deoxynucleic acid triphosphates: Amersham
- Spermidine: Calbiochem
- PEI cellulose: Merck
- Concanavlin A-Sepharose: Pharm
- CNBr-Sepharose: Pharmacia
Other chemicals used were of reagent grade.

### ABBREVIATIONS

- HPLC: High performance liquid chromatography
- SDS: Sodium dodecylsulfate
- EDTA: Ethylenediaminetetraacetic acid
- WGL: Wheat germ lectin
- WGL 1: Wheat germ lectin bound antigen pool 1
- WGL 2: Wheat germ lectin bound antigen pool 2
- WGL⁺: Wheat germ lectin bound antigen
- WGL⁻: Wheat germ lectin unbound
- IEF: Iso electric focussing
- LL: Lentil lectin
- LL⁺: Lentil lectin bound antigen
- LL⁻: Lentil lectin unbound
- HEPES: N-2-Hydroxyethylpiperazine-N¹-2-ethane-sulfonic acid
- Endo lys C: Endoproteinase lys C
- DTT: dithiothreitol
- pI: isoelectric point
- HFBA: heptafluorobutytic acid
- BSA: bovine serum albumin
- MMLV: murine maloney leukemia virus
- dNTP: deoxy nucleotide triphosphate
- dATP: deoxy adenosine triphosphate
- dCTP: deoxy cytidine triphosphate
- dGTP: deoxy guanidine triphosphate
- dTTP: deoxy thymidine triphosphate
- d(GCT)TP: a mixture of dGTP, dCTP, and dTTP
- NAD: nicatinamide adenine dinucleotide
- ATP: adenosine triphosphate
- PEI: polyethyleneimine
- BRL: Bethesda Research Laboratories
- IBI: International Biotechnologies Inc.
- A260,A280: Absorbance at 260 or 280 nm
- cDNA: Complementary DNA
- ds: double stranded
- g: gram
- gₐᵥ: average gravity units
- m,µ,n,p (prefixes): milli,micro, nano, pico
- M: Molar
- 1: litre
- U: Units of activity (restriction enzymes)
- bp: base pairs
- Kb: Kilobase pairs (thousand base pairs)
- TLC: Thin layer chromatograph
- ELISA: enzyme linked immunoabsorbent assay

### BUFFERS

- 10 x 1st strand: 0.5 M Tris pH 7.5
0.75 M KCl
0.03 M MgCl₂
- 5 x 2nd strand (RNase H): 0.2 M Tris pH 7.5
0.05 M MgCl₂
0.1 M (NH₄)₂SO₄
1 M KCl
1.5 mm B-NAD
- 10 x Methylase Buffer: 0.5 M Tris pH 7.5
0.01 M EDTA
- 10 x TA Buffer: 0.33 Tris-Acetate pH 7.9
0.66 M K-Acetate
0.1 M Mg-Acetate
- 5 x Kinase Buffer: 0.05 M Tris pH 7.5
0.05 M Mg Cl₂
0.05 M DTT
0.5 mM Spermidine
- 10 x Ligation Buffer: 0.3 M Tris pH 8
17 mM EDTA
70 mM MgCl₂
10 mM ATP
0.1 M DTT
20 ug/ml BSA
1 mM Spermidine
- 10 x High Salt Buffer: 1 M NaCl
0.5 M Tris pH 7.5
0.1 M MgCl₂
10 mM DTT
- 10 x S1 Buffer: 0.3 M NaAcetate pH4.4
2.5 M NaCl
10 mM ZnCl₂
- TE: 10 mM Tris pH 7.5
lmM EDTA
- PEI cellulose buffer: 0.75M KH₂ PO₄ pH3.5
- Buffer A: 0.05M Tris
0.03M acetic acid
0.1M NaCl

### TEAB buffer

TEAB is a solution of triethylamine equilibrated to pH7 by bubbling CO₂ through the solution. It is prepared as a 1M stock solution which is stored at 4°C. The pH is checked before use, the solution being re-equilibrated with a CO₂ pellet if required.

### EXAMPLE 1

### (a) Demonstration that at least some of the protective antigens are glycoproteins.

Since the majority of plasma membrane proteins are glycoproteins, inital attempts at further characterization of the protective antigen(s) focussed on lectin affinity.

It was found that wheat germ lectin and Concanavalin A bound to several components of tick antigen preparation B4/B5. Thus it appeared that a number of antigens in the tick preparation bear terminal N-acetylglucosamine residues and it is recognised that wheat germ lectin could be replaced in the purification scheme by other lectins with the same or similar terminal sugar specificity.

Approximately 2.lmg of antigen B4/B5 purified by the narrow range isoelectric focussing procedure (described in Australian Patent Application No. 45936/85) was applied to a column of WGL-Sepharose 6 MB (14) in 0.05M Tris chloride buffer, 1% Zwittergent 3-14, pH8 and washed with the same buffer. Bound glycoproteins were then eluted with 100mg/ml N-acetylglucosamine in the same buffer. Bound and unbound material was used to immunize sheep (Two vaccinations in Freunds incomplete adjuvant using five sheep per group). Induced immunity was estimated by applying freshly moulted adult ticks to the sheep and measuring the success of engorgement by the proportion of female ticks which finally engorged, relative to the number attached to the sheep skin three days after initial application of the young adults (Table 1).

**TABLE 1**

| Immunization of Sheep with Glycoprotein Preparations | | | | | |
|---|---|---|---|---|---|
| Group | Percentage of Ticks Engorging | | | | |
| Controls | 100, | 100, | 100, | 100, | 100, |
| Material not binding to WGL | 100, | 6, | 93, | 100, | 100 |
| Material binding to WGL | 0, | 93, | 0, | 83, | 28 |

It is clear that some animals in each vaccinated group were highly protected from tick challenge. Serum was obtained from each sheep in this experiment after vaccination but before tick challenge and the antibody titres of each serum sample against the antigens used in the vaccine were measured by radioimmunoassays. The animals in each group which showed tick damage had high antibody titres against the antigen preparation injected whereas those which had low titres allowed large numbers of ticks to engorge without any visible signs of damage (data not shown). It appears that protective antigens were present in both fractions used in this experiment but failure to observe tick damage with some animals was due to the failure of those animals to respond vigorously to vaccination for reasons which are currently unclear. (b) In a subsequent experiment with sheep, fraction GF5 and 6, the more highly purified gel filtration fractions (Australian Patent Application No. 45936/85) were chromatographed on a WGL-sepharose affinity column and the specifically bound and the unbound material was used to vaccinate sheep in a similar way to that described above. Again, for some animals in each group, the immune response generated by vaccination with either fraction was capable of producing damage to ticks feeding on those animals as demonstrated by the lower numbers of viable ticks recovered from the sheep (% surviving), the percentage of those ticks which were red in colour (% damage) and the lower weight of those ticks which survived or engorged (Table 2).

**Table 2**

| Group | Animal No. | Number of Ticks Surviving/Number Applied | % Surviving Ticks | % Damage | Mean Weight |
|---|---|---|---|---|---|
| Controls | 181 | 36/40 | 90% | 0 | 254 |
| | 182 | 45/50 | 90% | 4 | 224 |
| | 183 | 32/40 | 80% | 3 | 214 |
| | | | | | |
| WGL unbound | 121 | 27/40 | 67.5% | 19 | 182 |
| | 122 | 27/40 | 67.5% | 41 | 179 |
| | 123 | 30/40 | 75% | 3 | 223 |
| | | | | | |
| WGL bound | 124 | 27/40 | 67.5% | 52 | 156 |
| | 125 | 7/40 | 17.5% | 100 | 11 |
| | 180 | 9/40 | 22.5% | 100 | 11 |

In particular the material which was specifically bound to the affinity column is to be characterized herein but the protective antigens in the unbound fraction are also clearly capable of giving protection.
(c) An experiment similar to that described above was performed in which cattle were vaccinated with material which had specifically bound and material which failed to bind to a WGL-Sepharose column (Table 3). Again, the immune response generated by both fractions following vaccination gave indications of damage to ticks feeding on vaccinated cattle. The material which failed to bind to the WGL-Sepharose column was particularly effective in this experiment.

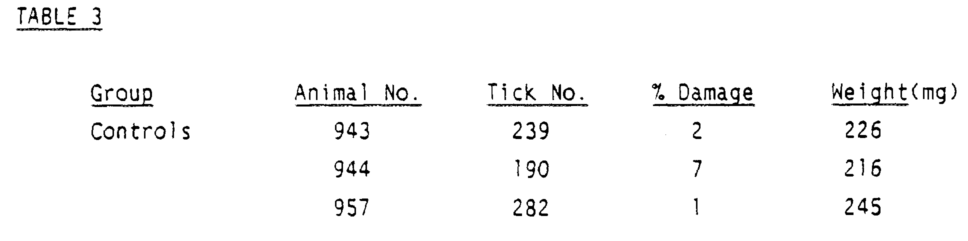

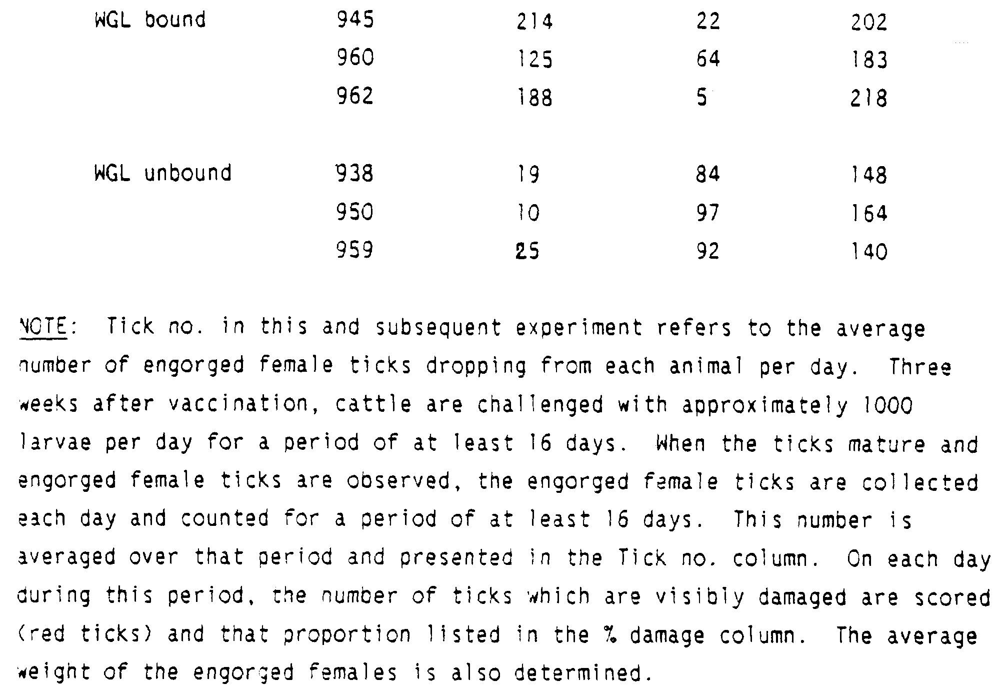

(d) Concurrently with this experiment, the material which had specifically bound to the WGL-sepharose column was fractionated on SDS polyacrylamide gels. Silver stains of these gels showed two major staining components which were excised (fractions S2 and S4) and these, as well as the intermediate portions of the gels (fractions S1, S3, S5 and S6) were used to vaccinate cattle. The most highly protective fraction was S2 (Table 4) which corresponds to one of the bands observed in stained gels which has an apparent molecular weight of approximately 80-90 kilodaltons in this gel system compared with Pharmacia and BRL molecular weight markers.

In this experiment, the number of ticks surviving on the cattle vaccinated with S2 was reduced compared with the other groups (Tick No column - the average number of engorged adult female ticks dropping from each animal per day over the 21 day period studied). In addition, the majority of the surviving ticks were red or appeared to be otherwise abnormal when examined visually (% damage) and the weight of those surviving ticks in the S2 group was reduced compared to the ticks from the animals in the other groups (Table 4).

**TABLE 4**

| Group | Animal No. | Tick No. | % Damage | Weight |
|---|---|---|---|---|
| S1 | 947 | 196 | 10 | 215 |
| | 951 | 194 | 1 | 230 |
| | 963 | 243 | 30 | 198 |
| | | | | |
| S2 | 941 | 115 | 66 | 147 |
| | 942 | 86 | 87 | 150 |
| | 953 | 173 | 32 | 192 |
| | | | | |
| S3 | 961 | 166 | 3 | 212 |
| | 967 | 240 | 4 | 243 |
| | 968 | 193 | 4 | 233 |
| | | | | |
| S4 | 939 | 163 | 1 | 229 |
| | 940 | 155 | 4 | 229 |
| | 952 | 149 | 9 | 258 |
| | | | | |
| S5 | 937 | 276 | 3 | 248 |
| | 955 | 232 | 2 | 225 |
| | 956 | 160 | 5 | 221 |
| | | | | |
| S6 | 946 | 269 | 12 | 222 |
| | 954 | 157 | 19 | 297 |
| | 958 | 281 | 1 | 245 |

(e) In vitro experiments were conducted in which a range of lectins were tested to determine which were capable of reacting with the material not retained on the WGL-sepharose column. Lentil lectin was found to be reactive and therefore the material not bound to the WGL-Sepharose was fractionated on a lentil lectin column(15). Cattle were vaccinated with these fractions, and the immune response generated against the material not bound to WGL-sepharose but bound to the LL-sepharose was found to result in some small indication of damage to ticks feeding on vaccinated cattle (Table 5). SDS gel analysis of this fraction shows a band which has a molecular weight which is in the same range as the S2 antigen identified in the previous experiment.

**TABLE 5**

| Group | Animal No. | Tick No. | % Damage | Weight |
|---|---|---|---|---|
| Controls | 990 | 195 | 0.7 | 252 |
| | 980 | 220 | 0.7 | 243 |
| | 979 | 248 | 0.6 | 252 |
| | | | | |
| WGL unbound | 1006 | 183 | 6.2 | 196 |
| LL unbound | 1002 | 188 | 0.3 | 233 |
| | 988 | 185 | 30.8 | 197 |
| | | | | |
| WGL unbound | 1001 | 270 | 3.9 | 244 |
| LL bound | 996 | 267 | 1.1 | 252 |
| | 994 | 249 | 16.1 | 206 |

Both fractions used in this experiment were capable of generating an immune response which was capable of giving some indication of protection in this experiment.

The lentil lectin chromatography step produced a far greater yield of material having a similar molecular weight to the S2 antigen than was produced by the wheat germ lectin chromatography step.

This similarity in molecular weight and difference in lectin affinity suggested that the molecules may have been related by a common peptide backbone but differed in glycosylation.

This was later disproved (Example 2g).

Due to the presumed similarity to 52 and greater abundance of the LL bound material it was proposed that this material be used as a starting material for further purification.

However, subsequent poor vaccination results with this material in the light of good vaccination results with WGL bound material (Example 3) and demonstrated differences in amino acid composition have led to further purification schemes and cloning schemes being developed for the S2 or WGL⁺ material.

### EXAMPLE 2

Knowing from the above results the iso-electric point, molecular weight and lectin binding characteristics of the major protective antigen (referred to above as S2), a number of experiments were performed in order to improve the efficiency of the isolation procedure. The following method has been devised which yields at least 10 times more of the S2 antigen (later referred to as the wheat germ lectin bound antigen, WGL⁺ antigen or WGL⁺) and lentil lectin bound antigen (later referred to as the LL⁺ antigen or LL⁺) than the methods described in Australian Patent Application No. 45936/85.

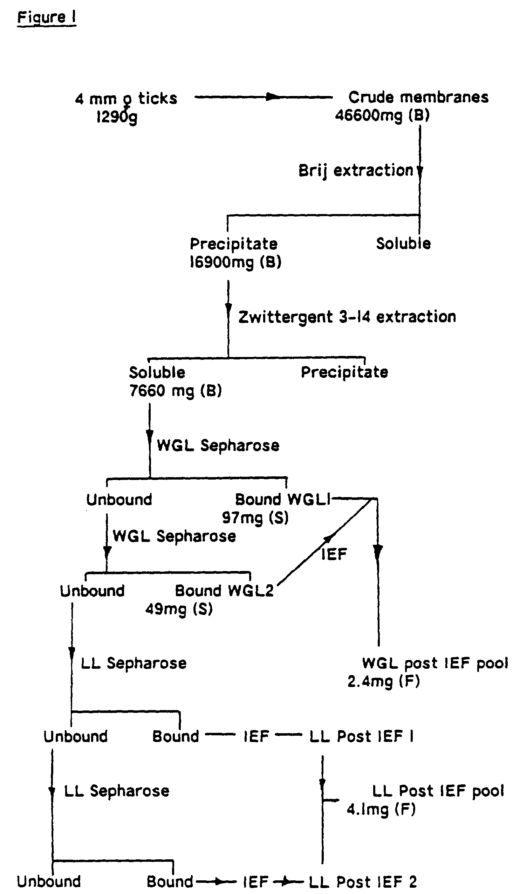

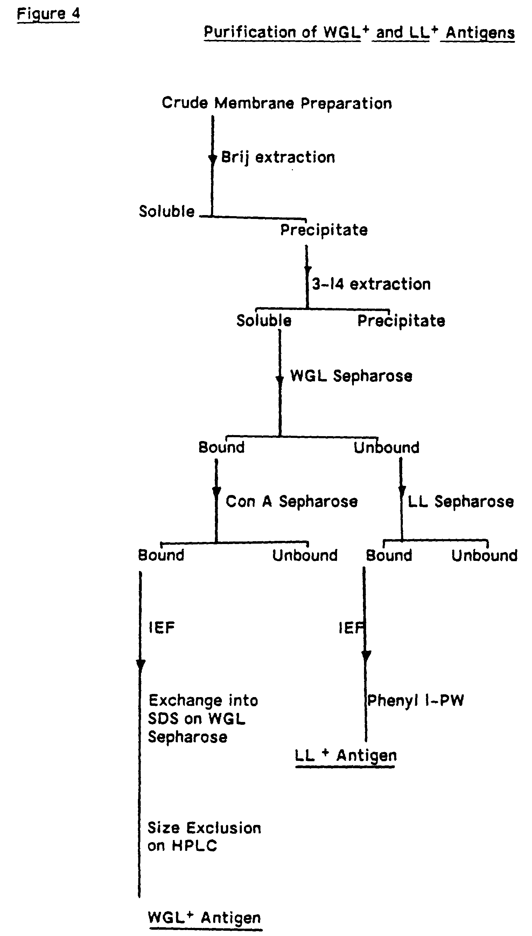

The procedure is outlined in the flow charts (Figs. 1, 2, 3, and 4).

### IMPROVEMENT OF THE PROCEDURES FOR ISOLATION OF THE MAJOR PROTECTIVE ANTIGEN

### (a) Isolation and Extraction of Tick Membrane and Particulate Material

1290 grams of semi-engorged adult female Boophilus microplus were picked from cattle on the day prior to the completion of engorgement. They were homogenised in 0.05M Tris, 0.025M acetic acid, 01M sodium chloride, 1mM EDTA, the homogenate strained through fine gauze and the retained material, which was mostly cuticle fragments, was rinsed with buffer. A total of 3ml of buffer per gram of ticks was used in the extraction. The suspension of tick material was then mixed with 350mg phenylmethanesulfonyl fluoride per litre and centrifuged at 600xgₐᵥ for 15min. The supernatant was then centrifuged at 20,000xgₐᵥ for 30min and the supernatant from that, centrifuged for 100,000xgₐᵥ for lh. Precipitates were collected from each of these centrifugation steps and frozen at -20°C until used.

The 600xg, 20,000xg and 100,000xg precipitates were thawed, suspended in buffer A (0.05M Tris, 0.03M acetic acid) and the protein concentration was measured. The suspension was diluted in buffer A containing Brij 35 to final protein and detergent concentrations of 5 and 10mg/ml respectively. The tick material was extracted at 37°C for lh then centrifuged at 3,300xgₐᵥ for 30min at 20°C. The precipitate was resuspended in buffer A and the protein concentration re-assayed. Extraction was repeated at the protein and detergent concentrations used before, substituting Zwittergent 3-14 for Brij 35, while the extraction time was lengthened to 90min. The suspension was centrifuged as before and the supernatant was retained.

### (b) Lectin Affinity Chromatography and Isoelectric Focussin (Fig.1)

The supernatant from the Zwittergent 3-14 extraction, (3255 ml), was stirred with 90ml of WGL Sepharose for 16h at 20°C, filtered and the WGL-Sepharose conjugate was poured into an 18 x 2.5cm column, washed with buffer A containing 1% Zwittergent 3-14 then eluted in buffer A containing 1% Zwittergent 3-14 and 100mg/ml N-acetylglucosamine. Fractions were pooled on the basis of the A280 absorption of specifically eluted material to give wheat germ lectin bound pool 1 (WGL1).

The adsorption of the detergent supernatant with WGL-Sepharose and subsequent elution of bound material was repeated as described above to give WGL2. The two eluates were then pooled (WGL pool).

The WGL pool was dialysed against 2 x 2.5 litres of water, then against 0.05M Tris-chloride buffer pH7.5 containing 0.1M ammonium thiocyanate. Concanavalin A-Sepharose (Pharmacia) was poured as a 2.5 x 11cm column and washed in buffer containing 0.05M Tris, 1% Zwittergent 3-14, 0.1mM calcium chloride, 0.1mM manganese chloride, 0.1M ammonium thiocyanate, adjusted to pH7.5 with hydrochloric acid. The WGL pool was loaded on this column, washed and the specifically bound material was eluted in the same buffer to which had been added 50mg/ml methyl-α-D-mannopyranoside. Fractions were pooled, dialysed against water then subjected to preparative isoelectricfocussing.

Isoelectricfocussing was carried out in a flat bed of IEF Sephadex containing 1% (w/v) Zwittergent 3-14 and Pharmalyte 4-6.5 diluted 1 to 15 (v/v) for 10,000Vhr. Individual fractions were analysed by SDS gel electrophoresis. The required protein appeared to be present in fractions with pI's of 5.3-to 5.7 though, for the sake of better purification, only those fractions with pI's of 5.4 to 5.6 were pooled to give "WGL post IEF pool".

The Zwittergent 3-14 soluble material left after the second extraction with WGL-Sepharose was mixed with 70ml of LL-Sepharose and stirred for 24h at 20°C, the suspension was filtered and the collected Sepharose conjugate was poured as a 2.5 x 14cm column. This was then washed with Tris-acetate, 1% Zwittergent 3-14 buffer and eluted in the same buffer containing 50mg/ml methyl-α-D-manno-pyranoside. Fractions were pooled on the basis of their A280 and dialysed against water. Further fractionation was carried out by preparative isoelectric focussing using the conditions already described for material which bound to WGL. Fractions were analysed by SDS polyacrylamide gel electrophoresis. The protein being isolated focussed over a pI range of 4.8 to 5.2 though the fractions which were pooled for further purification covered the range of 4.3 to 5.0.

The LL unbound material from the first affinity chromatography was readsorbed to LL-Sepharose and the material specifically eluted with methyl-α-D-mannopyranoside was separated by IEF. Material with the same pI range of 4.8 to 5.0 was pooled, then the products of the two experiments mixed to give "LL post IEF pool".

The method for the isolation of "WGL post IEF pool" and "LL post IEF pool" is shown schematically in Figure 1.

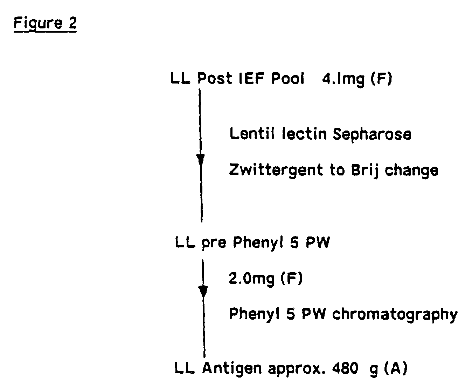

### (c) Hydrophobic Chromatography of LL Post IEF Pool (Fig.2)

A 1.6 x 6.5 cm column of LL-Sepharose was equilibrated in 0.1M Tris-acetate buffer, 1% Zwittergent 3-14 pH8.0. The "LL post IEF pool" was adjusted to pH7.1 and applied to this column which was subsequently washed with buffer, then with 0.1M Tris-acetate buffer, 0.1% Brij pH7.5. Bound material was then eluted with 0.1M Tris-acetate-Brij buffer containing SOmg/ml methyl-α-D-mannopyranoside.

Eluted material was dialysed against 0.1M Tris-acetate-Brij buffer then ammonium sulfate,was added to a final concentration of 0.5M. The sample was applied to a 7.5 x 75 mm TSK phenyl-5-PW column which had been equilibrated in 0.1M Tris-acetate, 0.5M ammonium sulfate, 0.1% Brij, pH7.5 and, after washing, the column was resolved with a linear gradient from this starting buffer to a buffer containing 0.1M Tris-acetate, 0.1% Brij pH7.5. Fractions were analysed by SDS gel electrophoresis and those containing the required protein pooled to give "LL⁺ antigen" or LL⁺.

This procedure is shown schematically in Figure 2.

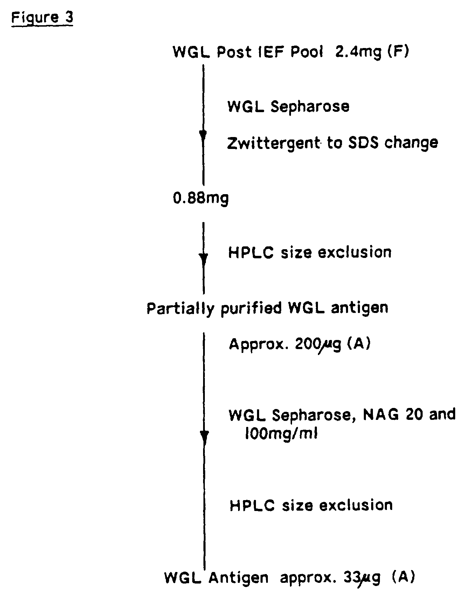

### (d) Size Exclusion Chromatography of WGL Post IEF Pool (Fig.3)

The pH of the "WGL post IEF pool" was increased to 7.3 and the material then loaded on a column of WGL-Sepharose equilibrated in 0.05M Tris-chloride, 0.2% Zwittergent 3-14 pH7.5. The column was washed with 0.05M Tris-chloride, 0.1% SDS, then bound material eluted in Tris-chloride-SDS buffer containing 100mg/ml N-acetylglucosamine. Fractions were analysed by SDS electrophoresis and those containing the required protein pooled, dialysed against 0.05M Tris-chloride buffer pH7.5 and concentrated on a Savant Speedvac.

Size exclusion chromatography was carried out using a Waters HPLC system and, in sequence, an Si200 Polyol guard column (Serva, Heidelberg), a 7.5 x 30cm Bio-Sil TSK 4000 and a 7.5mmx30cm PP 300 SW (Waters). Chromatography was carried out in a buffer containing 0.05M HEPES, 0.1M sodium thiocyanate, 0.1% SDS, the pH adjusted to 7.0 with sodium hydroxide, at a flow rate of lml/min and a column temperature of 37°C. In this system, bovine serum albumin had an elution time of 13.8 min and ribonuclease A of 17.7 min. Fractions were analysed by SDS gel electrophoresis. The material of interest was found to elute from the HPLC column at between 14.0 and 15.0 min and these fractions were pooled.

The product of this step still contained some impurity of lower molecular weight. It was therefore loaded on a 0.6 x 10cm column of WGL-Sepharose in 0.05M Tris-chloride, 0.1% SDS pH7.5, washed in this buffer, then tne bound material was eluted in the same buffer containing firstly 20mg/ml then 100mg/ml N-acetylglucosamine. Fractions were analysed by SDS gel electrophoresis and pooled on a basis of the amount and purity of the desired protein in each. They were concentrated and re-chromatographed on HPLC size exclusion chromatography as described above. The final pool of frections containing the desired antigen ("WGL⁺ antigen") was made after analysis by SDS gel electrophoresis as described above.

This procedure is shown schematically in Figure 3.

### (e) Protein Determination

Four methods of protein determination were used during antigen isolation, the methods being chosen on a basis of sensitivity required and the nature of expected interfering substances. These methods, and the abbreviations used for them in Figures 1, 2 and 3 were:
1. Biuret method; abbreviated (B)
2. Spectrophotometric method, from A280 and A260 measurements; abbreviated (S).
3. Fluorescence method, from the integrated fluorescence of high molecular weight material after derivatization with o-phthalaldehyde; abbreviated (F).
4. Absorbance method, based on the integrated A280 from HPLC chromatographic runs, assuming that a lmg/ml solution of the protein in a 1cm light path had an absorbance at 280nm of 1; abbreviated (A).

### (f) Comments on the Isolation Procedure

The major residual problem with the procedure described above is that in some preparations of the WGL⁺ antigen, a contaminant of lower molecular weight was observed as judged by SDS polyacrylamide gel electrophoresis. This contaminant could be partially, though not entirely, removed by repeating the affinity chromatography on WGL-Sepharose in SDS buffer and elution at two concentrations of N-acetylglucosamine.

The amounts of this impurity are variable from preparation to preparation. In a subsequent antigen isolation it was present in minor amounts and good antigen purity was obtained after pooling fractions with pI's in the range 5.30 to 5.67 on preparative isoelectricfocussing, followed by a single HPLC size exclusion chromatography. The yield of WGL⁺ antigen was thus higher (approximately 300µg from 1.3kg of ticks).

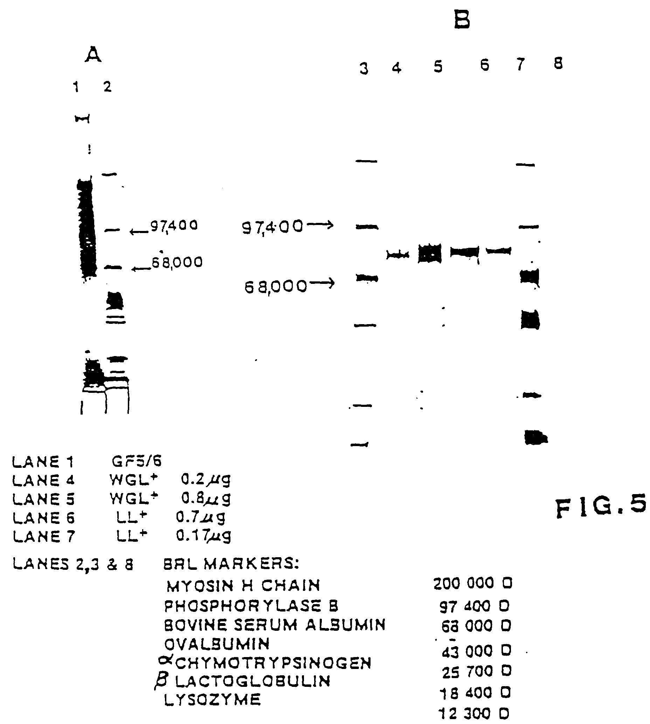

Fig. 5 shows SDS-polyacrylamide gel profiles of fraction GF5/6, the starting material in this work, (lane 2) and of the purified WGL⁺ antigen (lanes 4 & 5) and LL⁺ antigen (lanes 6 & 7) together with appropriate molecular weight markers (lanes 1, 3 & 8). It is clear from these gels that the GF 5/6 fraction is very impure and contains a large number of components in addition to the WGL⁺ antigen which is in fact such a minor component that it can not be distinguished from the other components in the fraction. The WGL⁺ and LL⁺ antigens are highly purified. In lane 5 which is an overloaded sample of WGL⁺ antigen, a small amount of the contaminating material at lower molecular weight can just be seen.

### (g) Amino Acid Composition of WGL⁺, and LL⁺ Antigens

Samples of the WGL⁺ and LL⁺ antigens isolated by the new purification procedure were analysed by amino acid analysis. The HPLC plots and calculated amino acid compositions derived from the HPLC printout by integration of the areas under each peak (Table 6) indicate that the antigens have different amino acid compositions. In addition the antigens clearly have different terminal sugar residues accounting for the different lectin binding characteristics.

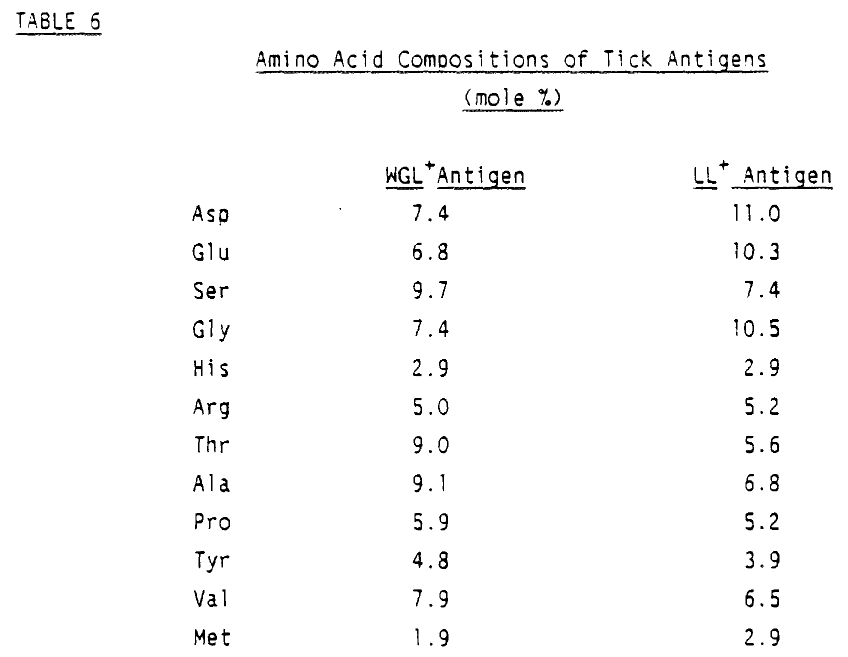

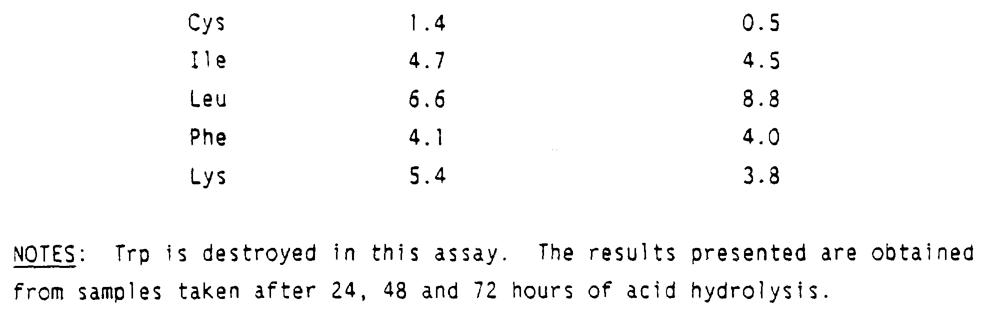

### EXAMPLE 3

### VACCINAL ACTIVITIES OF WGL⁺ AND LL⁺ ANTIGENS

Samples of WGL⁺ antigen (21µg) and LL⁺ antigen (400µg) were homogenised in Freunds Complete adjuvant and used to vaccinate cattle (1/10 of each preparation per animal per vaccination) as described in Australian Patent Application No. 45936/85. Vaccinated animals, together with control cattle were challenged with ticks and the numbers of engorged female ticks dropping from the experimental animals was monitored over a 16 day period (Table 7). It is clear that cattle vaccinated with very small amounts of WGL⁺ antigen were strongly protected from infestation in that the number of ticks dropping from each animal per day was reduced, the weight of the surviving ticks was lower and a high proportion of the surviving ticks were visibly damaged as a result of gut damage allowing cattle blood components to pass into the haemolymph of the ticks (% Red column). In addition, the ticks which survived on the cattle vaccinated with the WGL⁺ antigen had a greatly reduced capacity to produce eggs compared to the control animals.

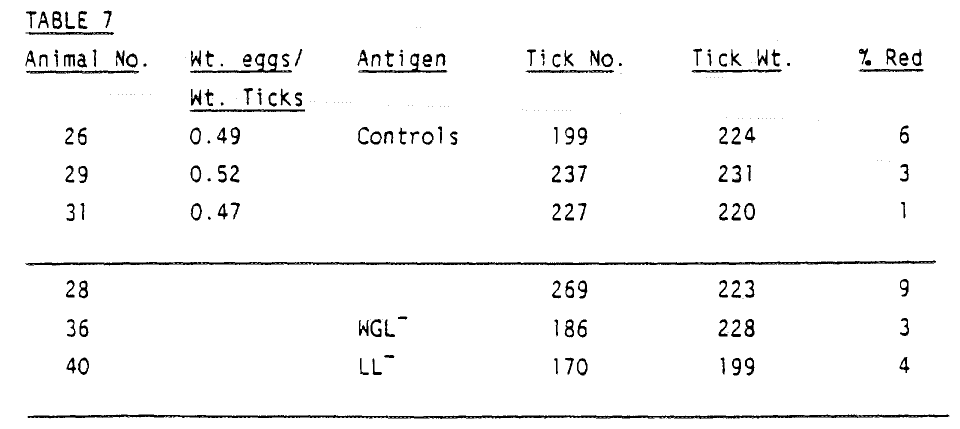

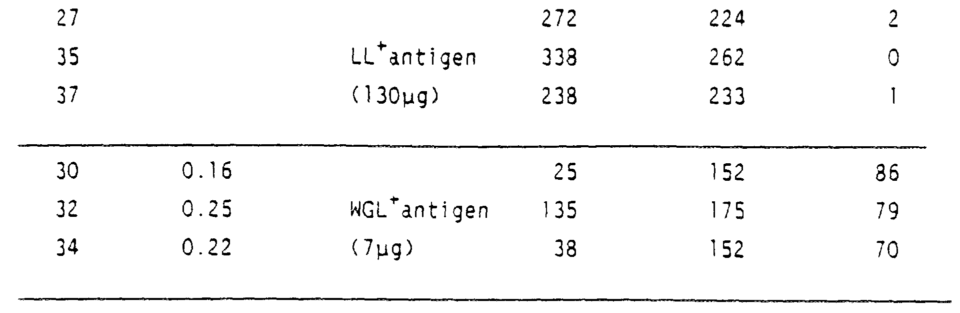

The LL⁺ antigen at a higher dose failed to give significant protection to the cattle despite the fact that the cattle had mounted a strong immune response to the vaccine as determined by ELISA [data not shown].

Both WGL⁺ and LL⁺ antigens appeared to be largely pure by SDS gel electrophoresis (Fig. 5) and both have similar molecular weights of approximately 89 kd in the gel system used compared to the BRL molecular weight standards used.

The new purification procedure outlined above is an improvement over that used previously giving a yield of 33-300µg WGL⁺ antigen compared with approximately 3µg of "52" antigen per 1.29kg tick starting material. It is asserted that these two antigens (WGL⁺ and S2) are the same glycoprotein based on similar molecular weight, isoelectric point, lectin binding properties, amino acid composition and vaccinal efficacy.

### EXAMPLE 4

### Digestion of WGL⁺ antigen with endoproteinase lys-C, separation of oligpeptides, determination of the amino acid sequence of oligopeptides and design of oligonucleotide sequences suitable as hybridization probes to detect recombinant organisms containing DNA sequences coding for the WGL⁺ peptide.

Approximately 40µg of WGL⁺ antigen purified as described in Example 2 was mixed with 100µl of 0.1M Tris-chloride buffer pH 8.3 containing 20mM dithiothreitol and 2% (^{w} /v) SDS, then incubated at 56°C for 30 min. The solution was then cooled to room temperature and sodium iodoacetate added to a final concentration of 0.14M. After 45 min. in the dark, cold methanol was added in a ratio 9:1 methanol : sample (^{v}/v). The sample was stored at -20°C overnight, centrifuged, the supernatant removed and the precipitate dried.

The precipitate was then dissolved in 76µl of 0.1M Tris-chloride buffer containing 4M urea, pH 8.5, then 4 µl of endo lys C (6 units per ml) was added. After 2 hrs at 37°C, another 4µl of enzyme was added and the digestion was continued for a further 17 hrs.

The digest was applied directly to an Aquapore RP-300 C-8 column in 0.1% trifluoroacetic acid and peptides were eluted in a linear gradient from 0-60% ^{v}/v acetonitrile/water in 0.1% trifluoroacetic acid. If necessary, peptides were rechromatographed in the same solvent system using an Aquapore 318 column. Peptides were collected, concentrated to 50-100µl by rotary dessication in a rotary evaporator. The amino acid sequences of the oligopeptides were determined using an Applied Biosystems amino acid sequencer. The following peptide sequences were obtained. The one letter and 3 letter codes used for amino acids are shown in Table 8.

### FRAGMENT NUMBER

F1(K)¹ D P D P G K (20-mer oligonucleotide)

F2(K)¹ W Y E D (G)² V L E A I (X)³ T S I G K (50-mer oligonucleotide)

F3(K)¹ (X) ⁴Q A C E (H)² P I G E (W)² C M M Y P K
(53-mer oligonucleotide)

(C)⁵
F4(K)¹ E A G F V Q K (23-mer oligonucleotide)

In addition, the following peptide sequences were deduced from mixed sequences which may assist in the characterization of the clones although there is a great deal of uncertainty in some of these sequences (especially F7).

             (S)     (V)     (V) (I)
F5(K)1 G (P) (D) G Q (C) I N (A) (C) K

         (G)
F6(K)1 A (D) V S T N E N E Q L E Q A D K

         (G)
F7(K)1 S (D) T Q (X)5 I D H I S K

      (N)     (A) (A)
F8(K)¹ (D) Q E (Y) (Y) Y

F9[(K)¹ C P C D N M Y F N A A E K          ]⁶
  [(K)¹ A N R Q C P P D T R R G E I G C I E]⁶

Oligonucleotides may be prepared using these amino acid sequences. For example the following could be used.⁷ 
20-mer 5'T T A C C T G G A T C T G G A T C C T T3'

50-mer TTA CCA ATG GAT GTA CAA ATA GCT TCA AGG ACA CCA TCT TCG TAC CAC TT

T

53-mer TTTGGGTACATCATACACCATTCACCAATTGGGTGTTCACAAGCCTGAGGCTT

AC
 NOTES: The following assumptions were made in interpreting the peptide sequences and in designing oligonucleotide probes:
Numbers 1-6 refer to superscripts in the peptide sequence listed above.
1. It was assumed that a lysine (K) preceded the first amino acid which was determined for each peptide based on the specificity of the endo lys-C.
2. These amino acids were assumed to be correct although they were detected at lower molar ratios than expected.
3. No amino acid could be confidently ascribed to the positions shown as X.
4. This position contained a number of amino acids. For the design of oligonucleotides, the correct amino acid was assumed to be either D, A or L but may be another.
5. More than one amino acid was detected in some sequences. The uncertainty is denoted by brackets.
6. These sequences were mixed (square brackets) and the relative molar abundance of the amino acids detected was approximately the same in each cycle.
7. A number of approaches known in the art can be used to design oligonucleotides suitable for use as hybridization probes. For example inosine base can be incorporated in positions where a number of deoxyribonucleotides are used in the third positions of redundant codons. The reverse complementary sequences to those presented can also be used equally well as hybridization probes. In the examples shown the codon usage was based on the sequence for the mRNA coding for the brine shrimp elongation factor (12).

**TABLE 8**

| amino acid | three letter code | one letter code |
|---|---|---|
| alanine | ala | A |
| arginine | arg | R |
| asparagine | asn | N |
| aspartic acid | asp | D |
| cysteine | cys | C |
| glutamic acid | glu | E |
| glutamine | gln | Q |
| glycine | gly | G |
| histidine | his | H |
| isoleucine | ile | I |
| leucine | leu | L |
| lysine | lys | K |
| methionine | met | M |
| phenylalanine | phe | F |
| proline | pro | P |
| serine | ser | S |
| thrionine | thr | T |
| tryptophan | trp | W |
| tyrosine | tyr | Y |
| valine | val | V |

### EXAMPLE 5

Approximately 40µg of WGL⁺ antigen was digested with endo lys-C as described in Example 4. The digest products were applied to an Aquapore RP-300 C-8 column in 0.1%, heptafluorobutyric acid (HFBA) and peptides were eluted in a linear gradient from 0-60% acetonitrile/water in 0.1% HFBA. Selected fractions were then re-chromatographed on Aquapore RP-300 C-8 or C-18 columns using trifluoroacetic acid in place of HFBA. The most symmetrical fractions were analysed for the presence of amino acids by hydrolysis of one tenth of the sample in hydrochloric acid vapour, derivatization with O-phthalaldehyde followed by reverse phase separation on HPLC and detection by fluorescence. The remaining portions of the samples were dessicated to 50-100µl volumes in a rotary evaporator and the amino acid sequence was determined using an Applied Biosystems amino acid sequencer.

The following peptide sequences were obtained.

### FRAGMENT NUMBER

F10 [(K) A P R Q N M Y F N A A E K ]
[(K) C N C D C P P D T R P G E I G C I E ]

F11 (K) W Y E D R V L E A I R T S I G K

F12 (K) E S S I C X D F G N E F C R N A E C E V V P (K) 72-mer
64 x 17 - mers

F13 (K) T R E C S Y G R C V E S N P S K 51-mer

F14 (K) A Y E C T C P R A F T V A E D G I S/H C K 63-mer
64 x 17-mers

[(K) D E V D N A S/H L V C Q N - A ]
F15 [(K) N V L Q S D G C G P Y ]
[(K) C L N P R P/L R L K H/S ]

S
F16 (K) A X V L C E X P
C
G

F17 (K) L Q A C E H P I

NOTES: It was assumed that a lysine precedes each fragment (K). X indicates that no amino acid could be confidently ascribed to the position during the peptide sequencing. F10 and F15 were mixtures of two and three peptide fragments respectively (denoted by []).

F10 is the sequence of the same mixture of two peptides as analysed for F9. It is surprising that these two oligopeptides co-purified on both occasions as the peptide fractionation procedure was different in the two examples.

F11 and F2 are likely to be the same fragment as the only differences are that the two uncertain amino acids in the F2 sequence are both R in the F11 sequence. A larger amount of material was present in F11 so this sequence is likely to be correct.

F17 and F3 appear to be sequences of the same peptide. F3 could be read further as more material was present but F17 contained less impurities so the first residue could be identified.

From these amino acid sequences, oligonucleotides can be prepared which would be suitable for screening cDNA and genomic ONA banks to identify the gene coding for the WGL⁺ antigen. The following examples could be used (see note 7 in Example 4. In the following examples, the third position in the codons was chosen to minimise secondary structure, not on brine shrimp usage as used in Example 4).

72-mer 5' TTT AGG TAC AAC CTC ACA TTC AGC ATT CCT ACA AAA TTC ATT ACC GAA ATC
AAA ACA AAT ACT ACT CTC CTT 3'

51-mer 5' CTT CGA CGG ATT GGA TTC GAC GCA TCT GCC ATA GCT ACA TTC CCT C[?]T
CTT 3'

63-mer 5' CTT GCA ATG GAT TCC ATC CTC GGC GAC AGT GAA AGC TCT AGG GCA AGT GCA
CTC ATA AGC CTT 3'

In addition, degenerate shorter oligonucleotides could be synthesized. For example 64 fold degenerate 17-mer oligonucleotides could be designed using the sequence DFGNEF from the F12 sequence and from the sequences KAYECT and YECTCP from the F14 sequence. 16 fold degenerate 17-mer oligonucleotide mixtures could also be designed using the sequence CMMYPK from the amino acid sequence of F3 shown in Example 4. These short degenerate sequences may be useful to confirm that clones isolated using long oligonucleotides contain the desired DNA sequences coding for the WGL⁺ antigen.

### EXAMPLE 6

### Genetic Engineering of the WGL⁺ (S2) Antigen

The major limitation to the development of commercial vaccines using the WGL⁺ antigen is the limited availability of WGL⁺ material which can be obtained from natural sources. Means by which this limitation can be overcome include the construction by genetic engineering techniques of bacteria, yeast or other readily cultivated cells including mammalian or insect cell lines which synthesize large amounts of all or part of the WGL⁺ antigen. There are several means by which this goal can be achieved but they basically fall into a small number of steps which, by means of example only, are set out below.

In order to identify the recombinant organisms which contain the tick genetic information coding for the protein backbone of the WGL⁺ antigen, appropriate reagents must first be generated. These may be antibodies from animals vaccinated with the purified protective antigen or with partially purified preparations containing that antigen preferably following denaturation of the antigen with a suitable detergent such as SDS. Bacterial, yeast or other cells which synthesize part or all of the WGL⁺ antigen must then be constructed and screened with the antiserum.

Preferably the WGL⁺ antigen is isolated in sufficient quantities in order to determine the amino acid sequence of the protein portion of the antigen or of fragments of the antigen produced as a result of endoproteolytic enzyme digestion using enzymes such as trypsin, endo lys C or pepsin or by chemical cleavage of the peptide using reagents such as cyanogen bromide. Fragments produced as a result of these treatments are separated and isolated using methods known in the art such as fractionation by HPLC reverse phase liquid chromatography or HPLC on columns containing hydrophobic resins, ion exchange resins or size fractionation resins. Preferably reverse phase resins such as C1, C8 or C18 are used singly or consecutively depending on the characteristics of the fragments produced as a result of the enzymatic or chemical treatment chosen.

Fragments of the WGL⁺ peptide produced as a result of these treatments are then analysed on a gas phase amino acid sequenator using methods known in the art. From the amino acid sequence and the known DNA sequences which encode each amino acid, oligonucleotide sequences can be prepared which are complementary to the DNA sequence coding for the antigen and these can be used in hybridisation experiments to identify recombinant organisms containing the DNA coding for the antigen. The DNA sequence of these reacting clones can then be determined to confirm that the sequence is the one of interest. The DNA sequence can then be used to design the best means by which the microorganisms can be engineered to manufacture large amounts of the polypeptide.

### (a) Construction of Gene Libraries

Readily cultivated microorganisms which contain the genetic information coding for the WGL⁺ protective antigen can be constructed using synthesised DNA which is complementary to the RNA isolated from the appropriate developmental stage of ticks or using DNA fragments isolated from any stage of ticks, preferably eggs or larvae as they will not contain bovine blood.

If antibody probes are the only reagents available for detection of the clones containing the WGL⁺ antigen, cDNA or genomic DNA libraries must be constructed in a phage, viral or plasmid system which would result in the expression of the tick antigen or parts thereof. Such vectors include lambda gt11, bacterial plasmids such as pUR290, pUR291, pUR282, pUK270, pUC8, pUC9 or eukaryotic viral vectors such as the baculovirus, pZipNeo, or SV40-based vectors.

If oligonucleotide probes are available, clone libraries can be constructed using cDNA or genomic DNA fragments in a larger range of phage, plasmid and viral systems including, for example, lambda gt10, EMBL vectors, or plasmid vectors such as pBR327, pBR329 or pBR322.

Preferably cDNA libraries are generated since smaller numbers of clones have to be screened and any problems with expression through introns are avoided. Ideally the developmental stage of ticks which synthesise maximum levels of the WGL⁺ antigen are first identified. If antibodies are the only means available to do this, in vitro translation of RNA isolated from ticks of various ages followed by immunoprecipitation of the translation products with antibodies and analysis by SDS gel electrophoresis and fluorography enables the identification of the most suitable stage of ticks for extraction of RNA for construction of cDNA banks. The apparently low abundance of the WGL⁺ protein makes this approach very difficult. If oligonucleotides are available, hybridisation to the RNA isolated from ticks of various ages should enable the identification of the RNA source containing the highest abundance of mRNA coding for the WGL⁺ antigen.

RNA can be isolated from ticks and cDNA synthesized and cloned a number of methods known in the art can be used. The following methods are outlined by means of example only.

### (b) Ethanol precipitation

In the following methods, ethanol precipitation involves adding to the solution of nucleic acid, one tenth of the solution volume of 3M sodium acetate and three to four volumes of absolute ethanol. The mixture is then stored at -20°C for at least 2 hrs or at -70°C or in an ethanol/dry ice bath until the solution becomes viscous. The mixture is then centifuged usually at 12,000xgₐᵥ for at least 10 minutes. The supernatant is carefully removed and the pellet containing the nucleic acid material (as well as other macro-molecules) is used in further manipulations.

### (c) Ethanol precipitation from 2M ammonium acetate

In high salt solutions (e.g. 2M ammonium acetate), the majority of unincorporated deoxynucleotide triphosphates (and other small molecular weight material) will remain in the supernatant after an ethanol precipitation. The procedure is as described above except an equal volume of 4M ammonium acetate is added to the solution instead of the sodium acetate followed by 3-4 volumes of ethanol before cooling as described above.

### (d) Phenol or phenol/chloroform extraction

Phenol or phenol/chloroform extraction involves the addition to the nucleic acid solution of an equal volume of redistilled phenol or a 1:1 (^{v}/v) mixture of phenol and chloroform equilibrated with 0.1M Tris pH8. The contents of the tube are mixed and the phases separated by centrifugation. The upper (aqueous) phase is removed to a fresh tube and the phenol or phenol/chloroform is discarded. Usually the aqueous phase is re-extracted and then extracted with ether to remove remaining phenol. Optionally the phenol or phenol chloroform phase from the first extraction may be re-extracted by addition of TE, mixing and centrifugation. In this case, the two aqueous phases would be combined before ether extraction and further processing.

### (e) PEI cellulose TLC

To monitor incorporation of radioactive dATP into nucleic acids during the various reactions in this procedure, thin layer chromatography on PEI cellulose was performed in 0.75M phosphate buffer pH 3.5. An aliquot of material to be monitored is applied toward one end of a strip of PEI cellulose and, after the chromatogram is resolved, the strip is exposed to an X-ray film. Following development of the autoradiograph, the areas of the PEI cellulose strip containing radioactivity are cut, placed in vials and the radioactivity in each determined by Cherenkov counting in a scintillation counter. The proportion of the radioactive material at the origin of the chromatograph can be used to determine the success of the reaction. This procedure is referred to as PEI cellulose chromatography.

### (f) Extraction of DNA and RNA

High molecular weight DNA and RNA are isolated from ticks picked from the host at different developmental stages. Ticks are homogenised at room temperature in an Omnimixer for 2-3 minutes in a buffer containing guanidine isothiocyanate (4.7M), Sarkosyl (7.4%), Tris (5mM) and 6-mercaptoethanol (70mM) pH 7.4. The homogenate is centrifuged at 4°C at 14 000 x gₐᵥ for 10 minutes. Solid CsCl is added to the homogenate (1g/2.5ml) which is layered onto a CsCl cushion (2.5g/ml) and centrifuged for 48 hours at 25,000 rpm in a SW28 rotor (Beckman). The upper layer is aspirated, the DNA band recovered and the RNA pellet recovered, precipitated with ethanol, washed several times with 70% ethanol and stored in TE at -70°C until used.

Polyadenylated mRNA can be isolated by passage over oligo dT cellulose columns or poly U Sepharose columns using methods described by the manufacturers (Collaborative Research).

### (g) cDNA Synthesis

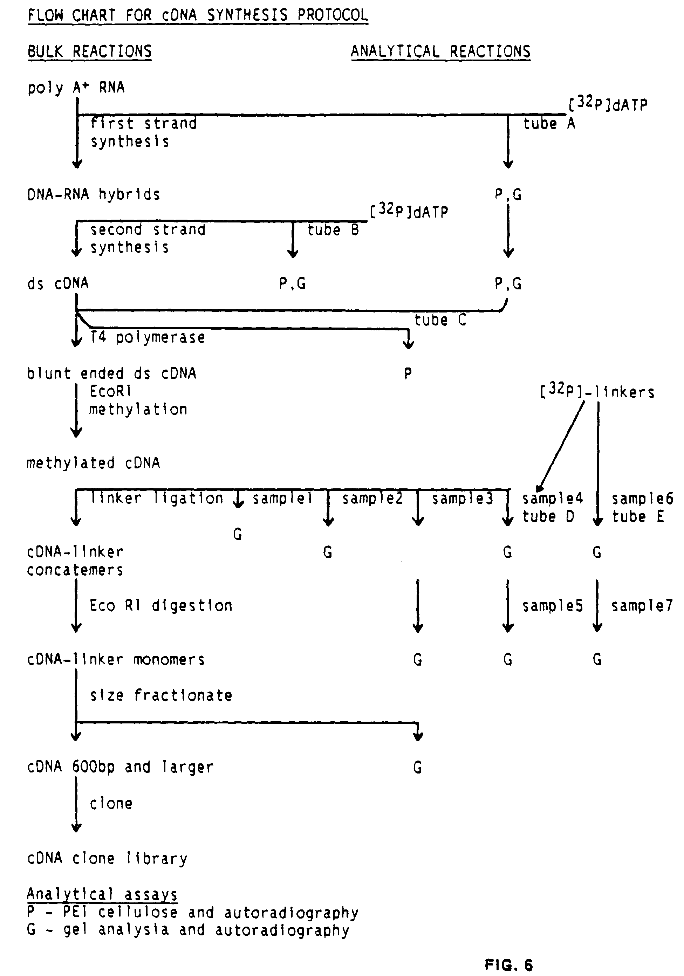

Several methods can be used for the construction of cDNA banks in phage or plasmid vectors. The following method by means of example only is a modification of the "RNase H" method for construction of cDNA banks in lambda gtll. The method is outlined schematically in Fig. 6

### (h) First Strand Synthesis

2µg of poly A⁺RNA is dissolved in TE. Water is added to give a final volume of 25µl. The solution is heated at 70°C for 3 minutes then rapidly cooled on ice. To the cooled solution is added 5µl 10 x 1st Strand Buffer, 5µl 0.1M DTT, 5µl Oligo-dT [Boehringer 100ng/1µl], 1.25µl RNasin [Promega 40U/µl], 2µl BSA (5mg/ml), 5µl 10mM d(GCT)TP, 0.5µl 10mM dATP and 3µl M-MLV Reverse transcriptase [BRL 200U/µl].

2.5µl of the mixture is transferred to tube A (analytical reaction for monitoring synthesis) and 0.2µl [³P]dATP is added (0.2µCi).

To the remaining bulk reaction 0.5µl 50mM dATP is added. The tubes are incubated for 30 minutes at 42°C. 0.25µl 10mM dATP is then added to tube A and the incubations continued for a further 30 minutes. A 0.5µl sample is taken from tube A and ethanol precipitated for gel analysis. A further 0.2µl sample is taken from the tube to be monitored by TLC on PEI cellulose.

If all of the 2µg of RNA added to the reaction was poly A-adenylated, it can be calculated that approximately 30% incorporation of [³P]dATP into nucleic acids is equivalent to 100% efficiency in first strand synthesis. Commonly RNA passaged over Oligo-dT cellulose once yields 6-10% incorporation.

To prepare a sample to monitor the second strand reaction, 2.5µl of the bulk reaction is removed and precipitated with ethanol from 2M ammonium acetate. The sample is washed twice with 70% ethanol then resuspended in 2.5µl 1 x 1st Strand Buffer in tube B.

### (i) Second Strand (RNase H)

A solution of; 28µl of water, 10µl 10 x RNase H Buffer; 1µl 5mg/µl BSA, 1.25µl 10mM d(GCT)TP, 0.5µl 10mM dATP, 1.6µl RNase H [BRL 20U/µl], 5µl DNA Polymerase 1 [holoenzyme (Biolabs) 100 U/µl] and 2µl E. coli DNA ligase, is prepared.

The solution is mixed, then 2.5µl is dispensed into Tube B with 0.2µl [³P]dATP, 1.8µl is dispensed into Tube A and the remainder is dispensed into the bulk reaction tube. 0.75µl of 10mM dATP is added to the bulk reaction tube. The three tubes are incubated at 15°C for 60 minutes, then at 22°C for a further 60 minutes.

A 0.2µl sample from tube 3 is chromatograpned on PEI cellulose to monitor the reaction. A further sample from tube B is ethanol precipitated from 2M ammonium acetate for gel analysis. The tube A sample from the first strand synthesis and the tube B second strand synthesis sample are run on a 1.5% agarose gel to determine the size of the cDNA which has been synthesized.

To prepare a sample to monitor the T₄ polymerase reaction, 0.5µl is taken from the bulk reaction tube and placed in Tube C.

The remaining contents of Tubes A and B are pooled with the bulk reaction. The contents of both the bulk reaction, and Tube C are extracted with phenol/chloroform (1:1), precipitated with ethanol from 2M ammonium acetate and the precipitates are washed twice with 70% ethanol.

### (j) EcoRl Methylation

A solution of 29.5µl of water, 4µl 0.1M DTT, 2µl 10xEcoR1 Methylase buffer, 4µl 1mM 5-adenosyl methonine [Biolabs] and 0.5µl EcoR1 Methylase [Biolabs 20U/µl] is prepared in a fresh tube. 2µl of the mix is dispensed into Tube C and the remainder into the bulk reaction tube. The two tubes are incubated at 37°C for 30 minutes then at 70°C for a further 15 minutes then cooled in ice.

In a fresh tube, the following buffer is prepared: 4µl 10 x TA buffer, 2µl Smg/ml BSA, 1.4µl 0.1M DTT, 2µl T₄ DNA polymerase [Biolabs 1U/µl] and 29.5µl of water. 2µl is added to tube C which is then incubated at 37°C for 10 minutes. 0.5µl of a solution containing 10mM d(GCTA)TP is added to the remainder of the solution and this is added to the bulk reaction tube which is then incubated at 37°C for 50 minutes, 70°C for 15 minutes then ice quenched.

To Tube C 0.2µl of each of 50µM d(GTC)TP, [³P]dATP [0.2µCi] and 5µM dATP are added and incubation is continued at 37°C for a further 50 minutes, after which time 0.2µl of the sample is spotted and chromatographed on PEI cellulose.

0.2µl of 0.2mM dATP represents approximately three times the amount of dATP required to add 2 adenosine residues to the 5' ends of each molecule, assuming that there was a total of 2µg of dscDNA of average size of lkb synthesized after 2nd strand synthesis.

### (k) Kinase -

There is some indication that the kinase step is not necessary and can probably be omitted. To the bulk reaction 20µl 5 x Kinase buffer, 0.2µl 0.1M ATP, and 0.5µl polynucleotide kinase [Biolabs 4U/µl] is added. The mixture is incubated at 37°C for 60 minutes. The reaction is extracted with an equal volume of a phenol/chloroform mixture (1:1), the aqueous phases are pooled, precipitated by ethanol from 2M ammonium acetate then washed twice with 70% ethanol.

### (1) Linker Ligation

To monitor the linker ligation reaction, samples are prepared for agarose and polyacrylamide gel analysis.

| Agarose gel: Samples from bulk reaction (³P cDNA) | |
|---|---|
| Sample 1 | cDNA before ligation to cold linkers |
| Sample 2 | cDNA after ligation to cold linkers |
| Sample 3 | cDNA after ligation to cold linkers and digestion with EcoR1 |

| Polyacrylamide gel: ³P linker samples | |
|---|---|
| Sample 4 | Tube D ³P linkers + cDNA before digestion with Eco R1 |
| Sample 5 | Tube D ³P linkers + cDNA after digestion with Eco R1 |
| Sample 6 | Tube E ³P linkers alone before digestion with Eco R1 |
| Sample 7 | Tube E ³P linkers alone after digestion with Eco R1 |

The ligation mixture is prepared by adding to a fresh tube 9µl EcoR1 linkers [Biolabs 200ng/µl], and 1.7µl of DNA ligase [IBI 3U/µl]. 15µl of ligation mixture is dispensed into the bulk reaction tube mixed quickly, then a 0.25µl sample is removed and frozen on dry ice immediately for agarose gel analysis (Sample 1).

A 1µl sample is taken from the bulk reaction tube and 0.2µl ³P labelled EcoR1 linkers is added (Tube D: cDNA + linkers).

A 1µl sample is taken from the remainder of the ligation mixture and 0.2µl ³P labelled EcoR1 linkers are added (Tube E: linkers alone).

The bulk reaction and tubes D and E are incubated at 25°C for 4 hours. Samples of 0.25µl from the bulk reaction tube and 0.6µl from tubes D and E are removed for agarose or polyacrylamide gel analysis respectively (Samples 2, 4 and 6).

The remainder of the bulk reaction and tubes D and E are heated for 5 hours at 70°C then cooled on ice.

### (m) EcoR1 digestion

To a fresh tube 11µl EcoR1 digestion buffer, 2µl EcoR1 [IBI 18U/µl] and 82µl of water are added. 4µl of the mixture is dispensed into the bulk reaction tube. The three tubes are incubated at 37°C for 60 minutes. A further 2µl aliquot of EcoR1 [36U] is added to the bulk reaction tube and incubation is continued for a further 60 minutes. The remaining samples in tubes D and E are electrophoresed on agarose and acrylamide gels together with the samples taken from tubes D and E above. Autoradiographs of those gels demonstrate whether the reactions have worked.

A 1.4µl sample is removed from the bulk reaction tube (Sample 3). The remainder of the bulk reaction is extracted with phenol/chloroform

A 1% agarose gel is run loaded with 0.25µl each of samples 1, 2 and 3. Samples 4, 5, 6 and 7 are run on a 12% polyacrylamide gel. Both gels are autoradiographed to determine whether all reactions have succeeded.

### (n) Separation of Linkers from cDNA

A 1.2 x 21 cm Sepharose 48 column is equilibrated with 0.1M TEAB. 150µl samples of EcoR1 digested linkered cDNA are loaded on to the column and fractions collected in TEAB buffer (250-500µl). Fractions containing cDNA fragments with sizes greater than 600bp as determined by mobility on agarose or polyacrylamide gels are pooled, evaporated to dryness in a rotary evaporator suspended in TE and ligated to EcoR1 digested and phosphatased lambda gt11 or gt10, packaged in vitro and infected onto suitable host strains such as Y1090 or Y1089 in accordance with suppliers instructions (Promega or Integrated Sciences).

### (o) Screening Clones with Oligonucleotides

From the amino acid sequence of the WGL⁺ protein, peptide fragments derived from chemical cleavage of the WGL⁺ protein or endoproteolytic digestion peptides derived from the WGL⁺ protein, oligonucleotides coding for specific portions of the DNA coding for the protein can be designed and used in hybridisation experiments using procedures known in the art. The DNA sequence of hybridising fragments isolated from the library can then be determined and used to design strategies for engineering the gene for expression of the WGL⁺ protein or portions thereof for incorporation into an effective vaccine.

A cDNA library was constructed in lambda gt 11 using RNA isolated from young adult B microplus which had been feeding on cattle for approximately 16 days. The phage were plated on E.coli strain RY1090 and grown at 37°C for 16 hours. Nitrocellulose filters were placed on the plates and triplicate filters were taken from each plate. The DNA on the filters was denatured and fixed by baking at 80°C under vacumn. The filters were incubated in prehybridization solution for 2-4 hours and then in hybridization solution for 16 hours essentially as described (10). The hybridization solution contained oligonucleotides which had been labelled with ³P using polynucleotide kinase (10) and α³P-ATP (approximately 10⁵cpm/ml of each oligonucleotide used).

For each set of three filters, two were hybridized to the 63-mer oligonucleotide and the remaining replicate filter was hybridized to a mixture of 51-mer, 72-mer, 50-mer, and 53-mer oligonucleotides. Following washing and autoradiography, plaques which gave rise to signals on all three filters were identified, picked and purified to single plaques.

### EXAMPLE 7

### ANALYSIS OF DNA SEQUENCE OF GENE CODING FOR WGL⁺ ANTIGEN

The DNA isolated from one clone will be described in detail. This 1.5Kb lambda gtll clone contained three Eco R1 fragments of approximately 4Kb, 1.5Kb and 0.3Kb. Southern hybridization (10) experiments showed that the 4Kb fragment hybridized to the probes used. This fragment was therefore subcloned into a modified pUC 18 plasmid (giving pBTA 707) in host strain JM101 (recombinant host/plasmid referred to as BTA 1751 ATCC 67548). The 4Kb fragment was then sonicated and subcloned into M13 mp18 for DNA sequence analysis.

M13 sub-clones were sequenced at random and the complete DNA sequence of the 4kb inset compiled by assembly of the sequences of the sub-clones by use of an alignment computer program.

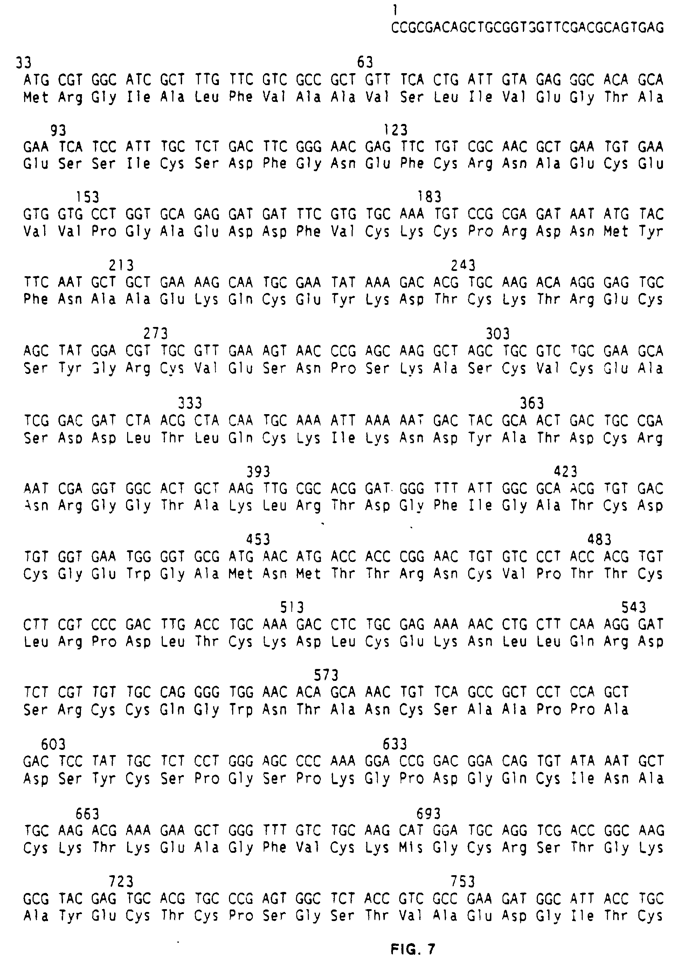

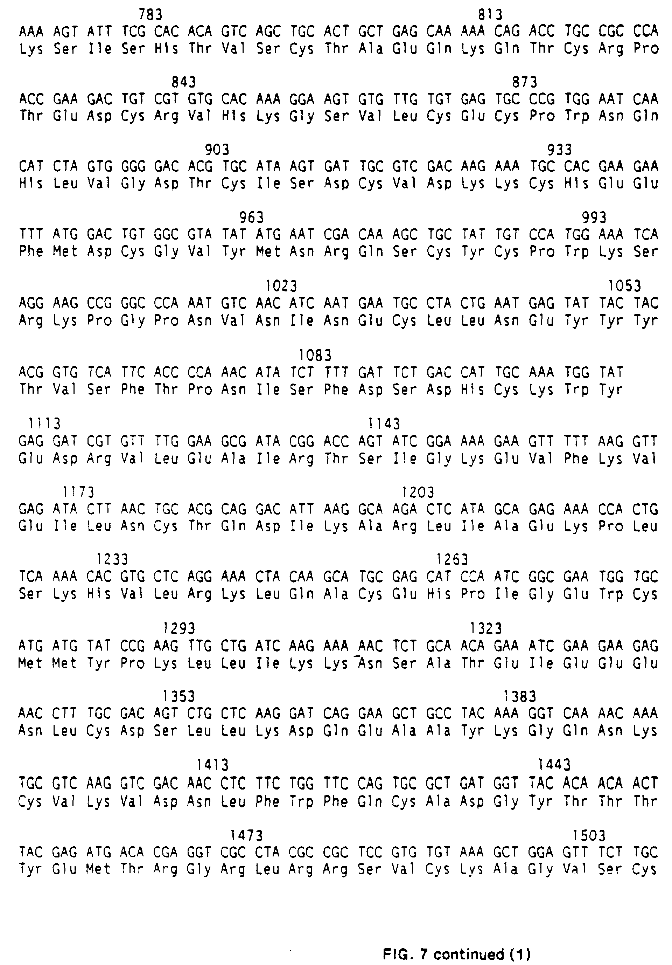

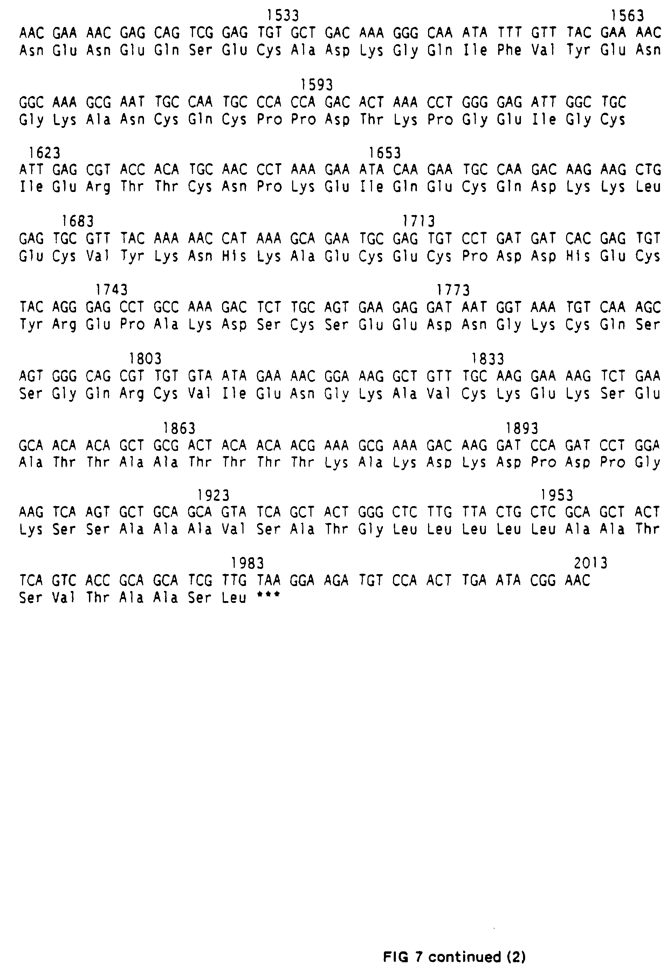

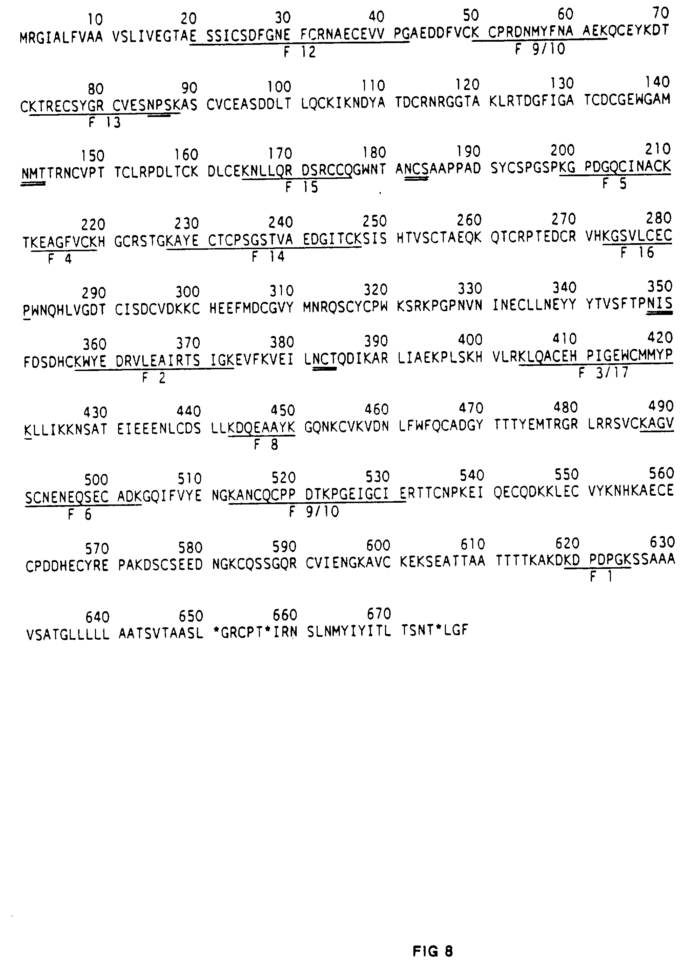

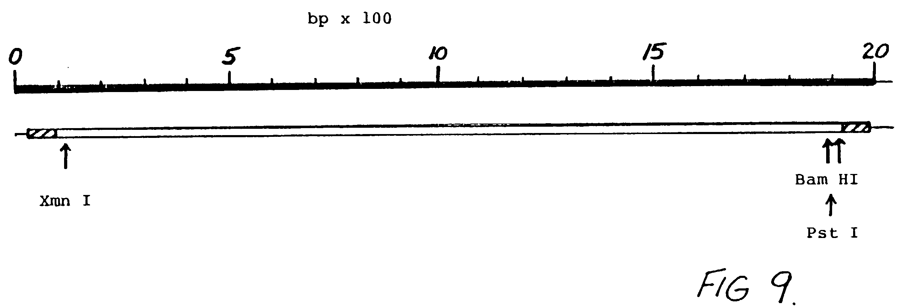

Fig. 7 shows the DNA sequence for the 4kb DNA fragment and the amino acid sequence which can be translated from one region of that DNA sequence into a protein sequence which is identified as the protein backbone of the WGL⁺ antigen. Fig. 8 shows that amino acid sequence using the one letter abbreviation code for amino acids (Table 8).

The peptide fragments identified during the peptide sequence analysis of endo lys-C digest products from the WGL⁺ antigen isolated from ticks are identified in Figs. 7 and 8 by underlines and are tabulated in a summary in Table 9.

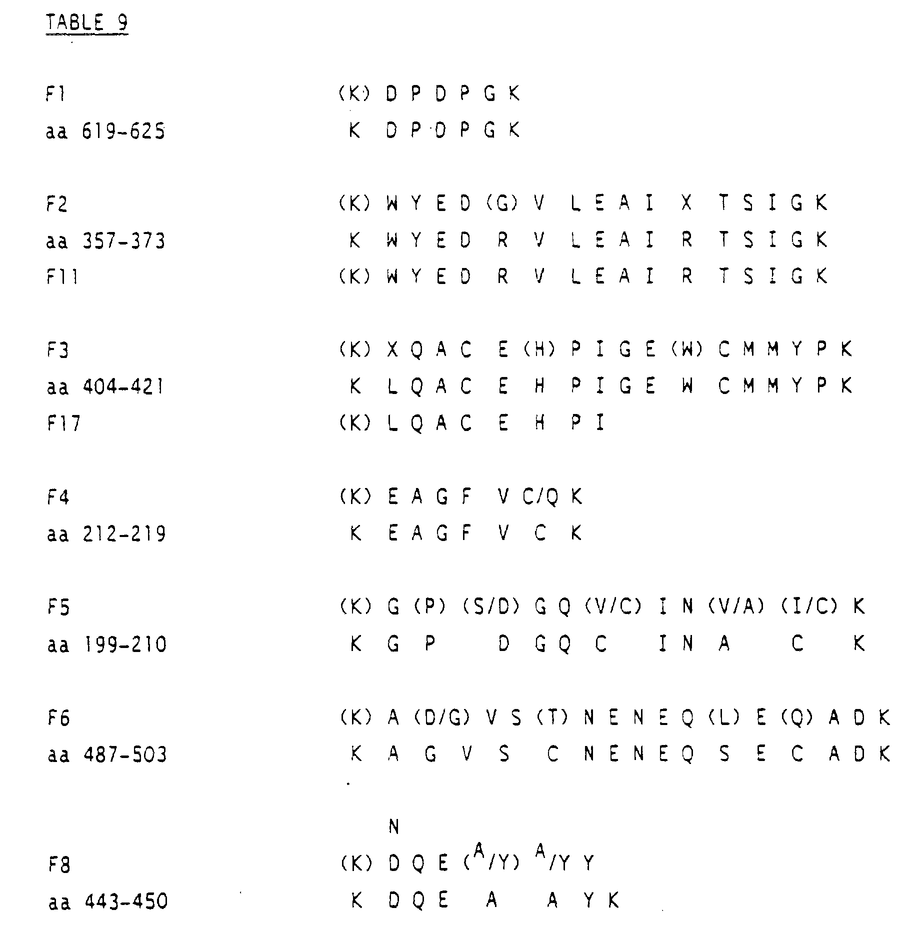

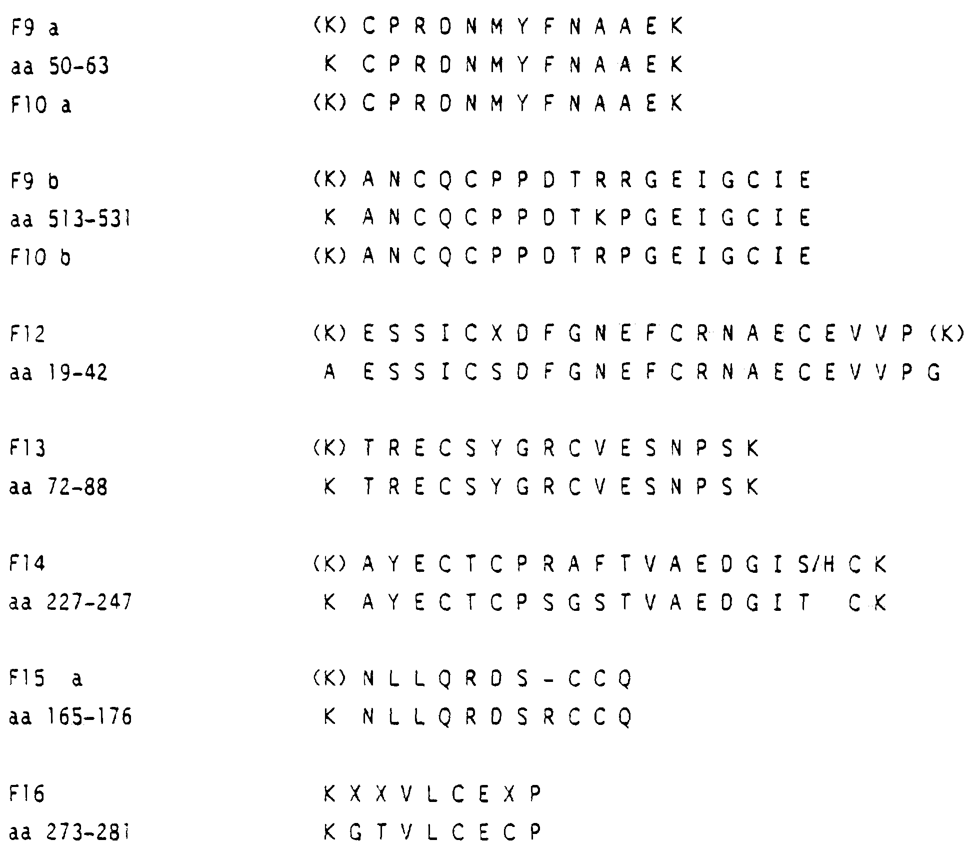

From the DNA sequence and the amino acid sequence deduced from that DNA sequence, it can be seen that the pre-pro-polypeptide of the WGL⁺ antigen consists of 650 amino acids.

The DNA sequence coding for peptide F12 can be identified at the region 90-152bp (Fig. 7) of the DNA sequence and corresponds to amino acids 20-40 in the amino acid sequence (Fig. 8) of the protein. The amino acid preceding the N-terminal glu residue identified in F12 is not a lysine (K) as would be expected if F12 was generated as a result of digestion by endo lys-C. Therefore it is assumed that the F12 peptide fragment was generated by the action of a proteinase other than endo lys-C. The 19 amino acid sequence preceding the F12 N-terminal glu residue begins with a methionine and has hydrophobicity properties which are very similar to leader sequences which precede other secreted and membrane-bound proteins in eukaryote cells (see 9 for review). In addition, the majority of peptide leader sequences are cleaved at positions following A residues (9). It appears therefore that the F12 sequence is the N-terminus of the mature WGL⁺ polypeptide. This then indicates that the protein portion of the mature WGL⁺ polypeptide is 631 amino acids long and which would have a molecular weight of 69 729 daltons.

Assuming that the consensus sequence for N-linked glycosylation is Asn X (Ser or Thr) in ticks as has been reported to be the case in other eukaryotic cells (10) 5 potential sites for N-linked glycosylation can be identified in the mature polypeptide sequence (Fig. 7). Carbohydrate residues added to these residues or to other amino acids in the WGL⁺ antigen produced by ticks would account for the differences in the observed molecular weight for the native antigen compared with that predicted from the DNA sequence.

By comparison of the amino acid sequence (Table 9) with the peptide sequences derived from the fractions from endo lys-C digestion, all of the peptides (F1-17) with the exception of F7 can be identified. In most cases, the amino acids which could not be confidently ascribed during the peptide sequence analysis can be shown to be correct following comparison with the sequence deduced from the DNA sequence.

The amino acid sequence for peptide fragments F1, F11, F13 and F17 all match precisely with the amino acid sequences deduced from the DNA sequence from the corresponding region of DNA (Table 9).

Peptide F2 can be seen to be coded for by the DNA segment 1104-1152bp. Table 9 shows that the G and the X tentatively ascribed to positions 5 and 11 in the F2 peptide sequence are both N. N is very difficult to detect during gas phase sequencing and there was very little material in the sample. Otherwise the match is precise. F2 is the same peptide as Fll and all amino acids were ascribed correctly during the sequence analysis of the Fll peptide fragment.

Peptides F3 and F17 show sequences of the same peptide obtained from two different endo lys-C digests of WGL⁺ (Examples 3 and 4). The amino acid sequence for F17 matches precisely with the translated sequence from amino acids 405 to 412 of the WGL⁺ peptide. When sequencing F3, no amino acid could be ascribed to the first position (L from the DNA sequence) as there was a large amount of background but the rest of the amino acids match precisely with the amino acid sequence derived from the DNA sequence (amino acids 405-421 Fig. 8).

F4 is found at amino acids 213-219 of the WGL⁺ protein (Fig. 8). The sequence matches perfectly and the uncertain ^{C}/Q is shown from the DNA sequence to be C. Carboxy-methylated C migrates with a similar retention time to Q in the HPLC system used to separate the derivatized amino acids following the sequencing reactions.

Very small amounts of material were sequencable in fragment F5 so there were several uncertainties. But it is clear that the sequence obtained corresponds to amino acids 200-210 in Fig. 8. One of the two amino acids tentatively ascribed to each peptide is present in the amino acid sequence derived from the DNA sequence and those ascribed with confidence appear in the expected order.

F6 sequence corresponds to amino acids 488-503 in the WGL⁺ protein sequence. The residues in the sequence derived for the F6 fragment differ from that derived from the DNA sequence. The F6 sequence presented was derived from a mixed sequence in which the amino acids shown to be correct from the DNA sequence were in fact present.

F7 has not been identified with confidence in the amino acid sequence derived from the DNA sequence. As with F6, the F7 sequence was derived from a mixed sequence and very little confidence can be placed in it.

Small amounts of material were present in F8 sample so there were several uncertainties in the sequence. However, the F8 amino acid sequence appears to correspond to amino acids 444-450 in Fig. 8. Again all uncertain residues can be identified in the translated DNA sequence.

F9 and F10 were both mixtures of two amino acid sequences. It is apparent that one of those sequences corresponds to amino acids 51-63 in Fig. 8. In both cases, one of the two amino acids identified during the peptide sequence analysis can be ascribed to the amino acid sequence derived from the DNA sequence in the expected order.

The remaining peptide sequence from F9 and F10 corresponds to amino acids 514-531 in Fig. 8. The R recorded for position 11 in the F9 sequence is P from the DNA sequence which is in agreement with the sequence obtained for F10. The DNA sequence shows K at what would be position 10 of this peptide. The DNA sequence shown is that coding for one molecule of the WGL⁺ antigen and it is likely that different ticks have some variants of the sequence. This point will be expanded when discussing the F14 sequence.

The fragment sequenced as F12 clearly corresponds to amino acids 20-41 in Fig. 8 and, as discussed previously is assumed to be the N-terminal fragment of the mature WGL⁺ peptide so the presumption that lysine preceded the first amino acids sequenced was incorrect in this case. The uncertain residue at position 6 of the peptide fragment is S from the DNA sequence. S is very difficult to detect during gas phase sequencing particularly as in this case, when the preceding amino acid is C and carboxy-methylated-C has a similar retention time to S in the HPLC system used to resolve the derivatized amino acids. Otherwise the F12 peptide sequence matches the sequence derived for WGL⁺ exactly.

F14 is very interesting (amino acids 228-247). The peptide sequence showed RAF for amino acids 8-10 in the peptide, whereas the DNA sequence, when translated, shows SGS in these positions. Both sequences appear to be correct retrospectively so there is a clear discrepancy between the two sequences. The most likely explanation is that both sequences are correct for the molecule (in the case of the cDNA) and the mixture of molecules (in the case of F12) which have been sequenced.

The tick population world wide is genetically diverse as is the case for all organisms which reproduce sexually. Each individual of a population differs subtly from the others in the population and these differences are a consequence of differences in the sequence of the DNA which each individual inherits from its parents. Thus for each gene coding for a particular protein, there are likely to be differences in the sequence among the population of individuals, referred to herein as homologues. In the particular example discussed here, the WGL⁺ protein which was digested in Example 4 to give rise to F12 was extracted from a large number of ticks (60,000 - 70,000). The peptide sequence determined for F12 (and the rest of the peptide fragments sequenced) is that of the majority of the population of WGL⁺ molecules. Among that population of WGL⁺ molecules, it is likely that minor variance (homologues) will exist at a level too low to be detected during the peptide sequence analysis. The cDNA sequence shown in Fig. 7 and the amino acid sequence in Fig. 8 are derived from one cDNA molecule from one individual in the population. This individual may have contained a DNA sequence coding for a minor variant of the WGL⁺ molecule. It is of course understood that other cDNA molecules may be derived from other individuals of the tick population world wide which will similarly vary in some small way from the sequence shown in Fig. 7 but still code for a protein which is essentially the same as that for the WGL⁺ antigen molecule. These homologues are included within the scope of this invention.

If the differences such as the one above are found in regions of the WGL⁺ molecule which are important epitopes for the protective immune response generated against the WGL⁺ molecule following vaccination, it is possible that the ticks with a WGL⁺ product which is a homologues of the sequence shown in Fig. 7 may survive feeding on vaccinated hosts. In this instance it is to be understood that cDNA can be synthesised or DNA isolated from these individuals as described above or by other methods known in the art. In hybridization experiments the 4kb DNA fragment (or parts thereof) can be used as hybridization probes to identify clones containing DNA coding for the WGL⁺ protein from those variants which can then be used to construct bacteria or other micro-organisms which synthesize the variant WGL⁺ antigen to be incorporated into effective vaccine against the variant tick population. This principal extends to isolates of Boophilus microplus and to other species of ticks from anywhere in the world.

The other difference in the sequence of F14 compared with the sequence for WGL⁺ polypeptide derived from the DNA sequence is that the residue at position 18 (S or H in F12 which was ascribed with low confidence) is T from the DNA sequence.

F15 was a mixture of at least three oligopeptides. Among those, one seems to be represented in the polypeptide at amino acids 166-176.

The F16 sequence can be seen in the WGL⁺ amino acid segment 274-281 (Fig. 8). The two uncertain residues in the peptide sequences are X=T in the second position and X=C in the seventh position of F16, both of which were due to the very small amounts of material which were present in this peptide sample.

### COMMENTS ON HYBRIDIZATION PROBES USED

The oligonucleotide probes which were chosen had several shortcomings which can be identified retrospectively. Some of these were due to incorrect choice of bases in the the third codon positions and, of course to the uncertainties in the peptide sequence analysis. The oligonucleotide which was relied upon most heavily was the 63-mer as it was based on the most reliable amino acid sequence obtained at that time. When isolating the clones it was surprising that the hybridization signal with this probe was weaker than expected from theoretical considerations and at one stage there was doubt that the clone isolated coded for the WGL⁺ peptide. This uncertainty was alleviated to some extent by the use of the degenerate oligonucleotide sequences mentioned above as probes. These probes hybridized strongly to the DNA in the clone. The reason for the weaker than expected signal with the 63-mer can now be explained by the variation in the DNA sequence from that expected in this region. A large number of other clones were purified based on the hybridization signal obtained with one or two probes but these all turned out to be unrelated to the WGL⁺ gene by DNA sequence analysis. Therefore the strategy for isolating the clone by using triplicate filters and the use of the highly degenerate oligonucleotide sequences as hybridization probes to confirm the interest in the clone has been vindicated.

### EXAMPLE 8

### Construction of recombinant organisms synthesizing WGL⁺ antigen

The major limitation to the development of a commercial vaccine based on the WGL⁺ antigen or homologues thereof is the limited amount of the antigen which can be obtained from ticks. The means by which this shortage can be overcome include the use of recombinant DNA techniques to engineer bacteria or eukaryote cells to synthesize large amounts of the antigen. The following by means of example only outline some approaches which could be taken.

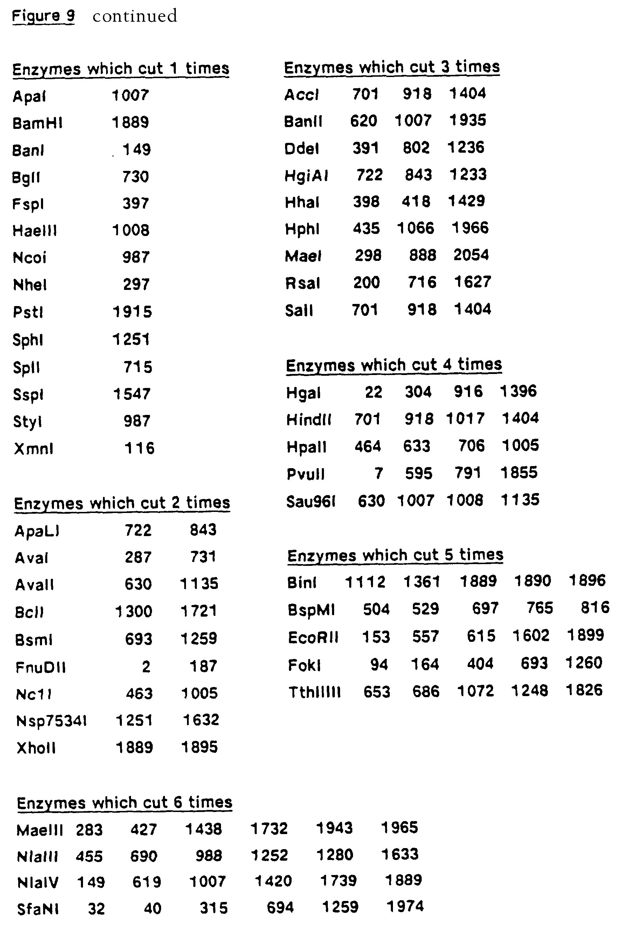

Fig. 9 shows a restriction enzyme map of the gene coding for the WGL⁺ antigen isolated from B. microplus. In order to engineer bacteria which express the gene product at high levels, it would probably be desirable to remove the parts of the molecule which are hydrophobic. These include the hydrophobic leader sequence (amino acids 1-19) which is not found in the mature polypeptide, and the hydrophobic C-terminal sequence (amino acids 630-650) which is likely to be an anchor sequence involved in attaching the antigen to the outer surface of tick cells. In Fig. 9, cleavage sites for the restriction enzymes XmnI (116 bases), PstI (1915 bases) and BamHI (1889 bases) are highlighted. DNA fragments produced by digestion of the WGL⁺ gene with XmnI in addition to BamHI or Pstl will contain the coding region for the majority of the gene without the N-terminal hydrophobic sequence or the C-terminal hydrophobic sequences. These 1773 bp and 1799 bp fragments can be subcloned into a number of plasmids including plasmids pBTA603 and pBTA224 to yield recombinant plasmids which will direct the synthesis of fused proteins containing the majority of the WGL⁺ peptide.

Plasmid pBTA603 has the PL promoter followed by a sequence from the N-terminus for the MS2 polymerase gene containing a multiple clone site vis ATG TCG AAG ACA ACA AAG AAG TTC AAC TCT TTA TCG ATG/GAT CCC Restriction endonuclease BamHl cuts the DNA where indicated (/) to give a 4 base 5' single stranded overhang. When this is filled in with DNA polymerase 1, the sequence MS2 - - TCG ATG GAT C is generated. When this is ligated to Xmnl cut WGL⁺ DNA (Xmnl cuts at the sequence GAANNNNTTC i.e. following base 120) the sequence MS2- - - -TCG ATG GAT CAG TTC TGT - - - WGL⁺ is generated. The plasmid so constructed encodes a protein which contains 15 N terminal amino acids from the MS2 polymerase and the cloning site sequences in place of the N-terminal 11 amino acids of the mature WGL⁺ sequence followed by the WGL⁺ amino acid sequence from amino acids 31 to 620 for the BamHl fragment or 31 to 628 for the Pstl fragment. When transformed into a suitable host such as N4830(10) which contains a mutation (cI^{ts}) in the gene coding for the cI repressor, expression of the fused polypeptide is repressed at temperatures such as 30°C but is active at temperatures such as 42°C. This temperature dependence of expression is advantageous in instances where the fused product is deleterious to the cells. Cells are grown at 30°C to the desired cell density and the temperature is then increased to 42°C to induce the synthesis of the fused protein.
The expression vector pBTA224 was used to generate a strain capable of producing a β-galactosidase-WGL⁺ fusion protein. pBTA224 was derived from pUR292 (EMBO J. 2, 1791-1794 (1983)) by eliminating the EcoR1 site that lies outside of the β-galactosidase-coding region. pBTA224 DNA was cut with the restriction endonucleases Sacl and Pstl, and the resulting 4221 bp fragment was purified by agarose gel e.lectrophoresis. Sacl cuts within the lac Z gene, 1181bps from the 3' end. Pstl cuts pBTA224 at the 3' end of lacZ. A WGL⁺ gene fragment suitable for expression in this vector was prepared by first inserting an Xmnl restriction fragment of about 2Kb (position 116 to past 3' end of WGL⁺ gene) into the vector M13um31 (obtained from International Biotechnologies, Inc.). By cutting the new construct with Sacl and Pstl, a fragment encoding most of the WGL+ and having Sacl and Pstl cohesive ends could be obtained. 
121 of WGL+ sequence 1911
SacI 5' > > PstI
end CGGTACCC AG TTC TGT AGT GCT GCA end
TCGAGCCATGGG TC AAG ACA TCA CG
3' From M13um31 From WGL+ gene
 This fragment ligated to the large pBTA224 Sacl Pstl fragment described above gives.

WGL+
2/3rds lacZ gene   AAC GAG CT   CGGTACCCAG TCC ----
                   TTG CTC GA   GCCATGGGTC AGG ----

The fusion protein expected to be produced after induction with IPTG consists of the first 651 amino acids of β galactosidase, 599 amino acids of WGL⁺ and 19 amino acids that are encoded by other parts of the expression vector, such as the multiple cloning sites. The calculated molecular weight is 143,054 daltons.

The plasmid described above has been designated pBTA708. A suitable E.coli host containing the lacI^{q} gene is JM101. BTA1752 is JM101 transformed with pBTA708.

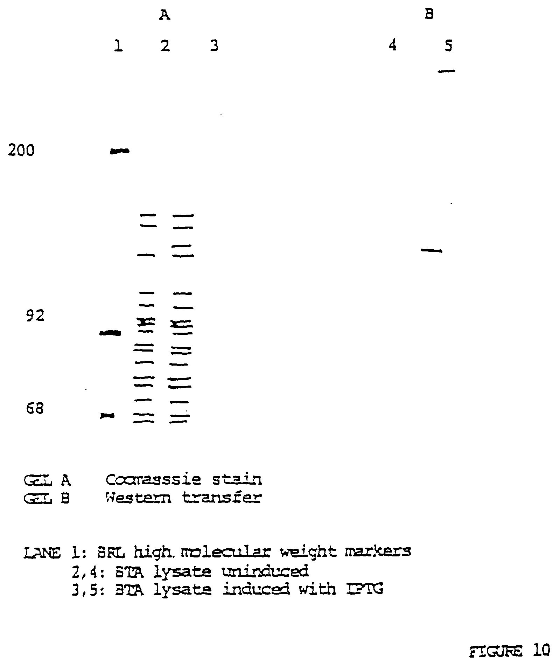

Cell lysates prepared from IPTG induced and control cultures, were analysed by electrophoresis in SDS-polyacrylamide gels. One gel was stained with Coomassie brilliant blue and a band of about the expected size could be visualised (Fig. 10). The band was absent in the non-induced control. A duplicate SDS-polyacrylamide gel was also run and the proteins in the gel were transferred to nitrocellulose paper. The nitrocellulose paper was incubated in BLOTTO (a solution of 5% powdered milk in Tris-saline) for 2 hours, then in BLOTTO containing a 1/500 dilution of serum from a rabbit vaccinated with the fractions GF5 and 6 (see above) for 13 hrs at 4°C, then washed three times with BLOTTO, then incubated in a solution containing goat-anti-rabbit immunoglobulin conjugated to alkaline phosphatase (Promega Biotec). Following incubation for 1hr, the nitrocellulose was removed, washed twice in BLOTTO and incubated in buffer containing Nitro blue tetrazolium and 5-bromo-4-chloro-3-indolyl phosphate. A band appeared where the rabbit antibodies had bound to the β galactosidase-WGL⁺ fused polypeptide synthesized by the bacteria (Fig. 10). The position of the band corresponds to the position of the band seen in the coomassie stained gel.

### Example 9

### Fermentation purification and formulation of vaccines based on the WGL⁺ antigen produced by rDNA techniques

Strains expressing the WGL⁺ antigen or portions thereof are maintained as freeze-dried vials in the production culture collection. Cells from the storage vial are reconstituted and plated out on a selective medium, and the cells from this medium are used to prepare fermentor inocula. The inocula are used to seed fermentors containing a suitable growth medium and the fermentation proceeds under conditions appropriate for the production of the WGL⁺ proteins. At the completion of the fermentation the cells are harvested and the product is released from the cells and undergoes purification. The product is subjected to analyses and quality control, and is stored under conditions appropriate for good stability. The product is formulated for use by combination with other ingredients under conditions of strict hygiene.

The strains produce the WGL⁺ fusion proteins in vivo as insoluble agglomerates termed inclusion bodies and can be produced and purified by the following procedure which is presented by means of example only.

Overnight cultures of BTA1752 is diluted 1:50 into 2 x 1 litre fresh LB (10 g tryptone/5g yeast extract/5g NaCl per litre pH 7.5) in 2 litre baffled flasks and shaken at 30°C until the culture density reached OD 0.3 - 0.4. IPTG is added to a final concentration of 10mM and incubation continued for a further 10 hours. The cells are harvested and resuspended in 20 ml of water per liter of original culture and broken by use of a French Press. The suspension is made 0.1 mM in phenylmethylsulfonyl fluoride (PMSF) and 5% Triton X-100 then centrifuged at 12,000 x gav for 10 minutes. The supernatant is discarded and the pellet resuspended by ultrasound in 50ml 1M NaCl/5% Triton X-100 and recentrifuged. This washing stage is repeated and the pellet finally resuspended using ultrasound in 2.5ml 1M NaCl/5% Triton X-100 per liter original culture.

Purified inclusion bodies are dissolved at.2 mg/ml in 8 M urea/0.1M DTT/0.1M Tris HCl pH 8.0 under nitrogen at 37°C for 2 hrs. The solution is centrifuged at 20,000 x gav for 20 min and the supernatant passed through a 0.1 µm filter. The flow through is passed through a filter with a molecular weight cut-off of 30 kilodaltons and the retained material is applied to a DEAE resin which is poured into a column and washed with 0.1M tris buffer pH8. The column is then resolved with a linear gradient of from 0-5M NaCl in 8M urea 0.1M Tris pH8.0 and the fractions analysed by SDS-Polyacrylamide gel electrophores. Those containing the desired protein are pooled, concentrated and desalted on a XM30 filter. The partially purified protein is emulsified in an adjuvant such as Marcol 52: Montanide 888 (9:1) or Freunds complete or incomplete adjuvant and administered to animals.

### Example 10

### IDENTIFICATION OF DNA SEQUENCES CODING FOR THE WGL⁺ ANTIGEN IN SPECIES OF TICK OTHER THAN BOOPHILUS MICROPLUS

In various countries throughout the world, tick species other than Boophilus microplus are responsible for extensive productivity losses either due to the tick infestation or due to the other parasites which the ticks transmit or a combination of both. It would be highly desirable to develop vaccines against these tick species. This may be achieved by vaccinating animals with the WGL⁺ antigen derived from Boophilus microplus or the other immunogenic protective fractions described in this and Australian patent application No. 45936/85. It may also be possible to vaccinate animals with the WGL⁺ antigen produced by recombinant organisms described herein and elicit an immune response which protects against infestation of animals by other species of tick.

As discussed above, the other species of tick probably contain a molecule which is functionally related to the Boophilus microplus WGL⁺ antigen but which differs in sequence from that shown in Fig. 8. If those differences occur in areas eliciting protective immune responses, then the Boophilus microplus WGL⁺ antigen may not be protective. However, the related gene product from the other species of tick is likely to be protective against those tick species, when incorporated into a vaccine.

One means by which this proposal can be tested is to conduct a series of vaccination/challenge experiments using fractions.derived from homogenates of other ticks and purify the WGL⁺ homologues from the other tick species. These can then be cleaved with proteinases, peptide fragments sequenced, oligonucleotides designed and used to identify recombinant organisms containing the genes in a similar way to that in which the Boophilus microplus WGL⁺ gene has been identified in the present work.

A preferable approach is to construct cDNA or genomic DNA libraries from nucleic acids extracted from other tick species and to use the DNA fragment shown in Fig. 7 or portions thereof as hybridization probes to identify clones containing the homologous gene from the other tick species. Then, engineered recombinant microorganisms synthesizing the homologous gene product could be incorporated into an effective vaccine against the other species of ticks.

In order to demonstrate that this latter approach is feasible and to generate information concerning the conditions under which the hybridization to the clone libraries should be carried out, preliminary "Southern blot" hybridization experiments can be conducted. Briefly by way of example only DNA isolated from a number of species of tick is purified and digested with restriction endonucleases. The DNA fragments so produced are size fractionated by electrophoresis on agarose gels, denatured and transferred to nylon or nitrocellulose filters by capillary action. The filter is, incubated in a prehybridization solution and then in a hybridization solution containing radioactively labelled DNA fragments derived from the WGL⁺ gene coding region. Following hybridization and washing of the filters, they are exposed to X-ray film and the resulting autoradiograph shows exposed areas which correspond to the DNA fragments from the various tick species which have hybridized to the WGL⁺ DNA fragments. There are many variations of protocols for carrying out this procedure which will be known to individuals skilled in the art and the following is detailed by means of example only.

Eggs were obtained from female ticks of the species Rhiphicepalus appendiculatus, Amblyomma variegatim, Boophilus decoloratus and Boophilus microplus. They were incubated in a humidified incubator for 2-4 days then suspended in cold TE buffer and washed. They were then suspended in TE buffer containing 0.5% SDS in a loose fitting glass-homogeniser and gently homogenised to disrupt the eggs. Proteinase K was added to a final concentration of 50µg/ml and the mixture was incubated at 37°C for 1-2 h with gentle shaking. The viscous solution was gently extracted three times with phenol saturated with 0.1M Tris-HCl pH8.0 and then twice with ether (centrifugation at 5,000 x gav for 10 minutes was used to resolve the phases during the phenol extractions). Sodium acetate was. added to 0.3M and 2 volumes of ethanol was slowly added with stirring. The DNA which came out of solution as a fibrous precipitate was removed with a pasteur pipette, washed in ethanol, and gently redissolved in TE. .

Aliquots (generally containing 10µg) of these DNA samples were digested with restriction endonucleases according to the manufacturers instructions. Aliquots of the digest products were fractionated by electrophoresis on a 1.6% agarose gel in SEB buffer (10). The DNA was depurinated with 2 volumes of 0.25M HCl for 15 minutes. The DNA was transferred by capillary action to a nylon membrane (Zetaprobe, Biorad). The filters were incubated in prehybridization in a solution (10) containing herring sperm DNA for 2-4 hours at 55°C. Hybridization was carried out in the same solution containing heat denatured [³P] labelled DNA fragments from the WGL⁺ gene (approximately 10⁵ counts per minute/ml) for 20 hours at 68°C. The filters were washed at 55°C for 30 minutes each in 2 x SSC, 0.1% SDS then three times at 60°C for 15 minutes. After exposure to X-ray film for at least 24 hours the size of the hybridizing fragments could be determined by comparison with marker DNA fragments of known size.

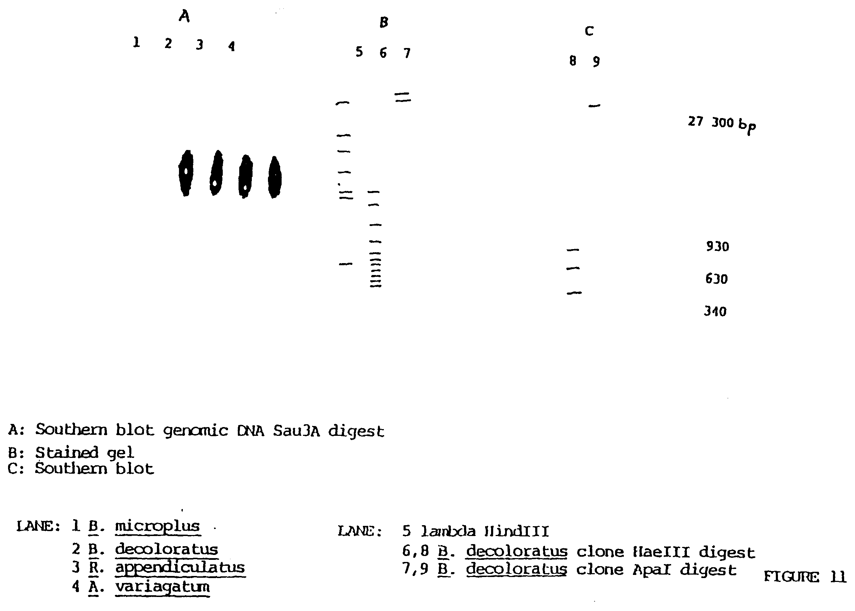

Fig. 11 shows an autoradiogram of one such experiment. DNA was digested with restriction endonuclease Sau 3A. The WGL⁺ DNA clearly hybridizes to the DNA from all four species of ticks. In this experiment, the DNA was not intact so a smear is observed in all cases but hybridization is specific as no hybridization to control DNA on the same gel could be detected.

### Example 11 Isolation of clones coding for WGL⁺ homologous from other tick species

The DNA from each of the species tested possesses sequences which are similar to and homologous with the DNA coding for the WGL⁺ antigen from Boophilus microplus. Clones containing those DNA sequences from other tick species can be isolated by constructing cDNA or genomic DNA libraries for the other tick species and hybridizing Boophilus microplus DNA fragments to those libraries, and purifying recombinant organisms containing the DNA seqences hybridizing to the homologous genes.

More specifically, the genomic DNA isolated from the tick species listed was subjected to partial digestion with the restriction enzyme Sau 3A to give fragments with an average size of 15-20Kb as judged by gel analysis. These were ligated into the 8am HI site of lambda EMBL 3 arms essentially as described by the suppliers (Promega Biotech). The libraries were plated on a restrictive host K62 and incubated overnight at 37°C. The plaques were transferred to triplicate nitrocellulose filters, and the DNA denatured with 1.5M NaCl/0.5M NaOH, neutralised with 3M NaCl/0.5 M Tris HCl pH 7.0. Then the filters were vacuum baked at 80°C. for 2 hours and hybridized to Boophilus microplus DNA probes labelled with ³P. Following autoradiography, plaques which hybridized to the probes on both filters were identified, picked and purified to single plaques by repeated rounds of re hybridization.

DNA was isolated from one plaque from a B. decoloratus genomic library and digested with restriction endonucleases Hae III and Apa 1. The fragments so produced were separated by electrophoresis on 1.6% agarose gels. One gel was stained with an ethidium bromide solution and the bands visualised under ultraviolet light (Fig. 11). A replicate gel was transferred to nylon membrane and hybridized to Boophilus microplus DNA coding for the WGL⁺ antigen. Fig. 11 shows that fragments from the Boophilus decoloratus, Amblyomma variegatum genomic clone hybridize to the WGL⁺ gene.

The bands hybridising in the HaeIII digest are approximately 980, 630, and 340 bp and in the Apa 1 digest 27,300 bp when compared with fragments of DNA from bacteriophage lambda digested with Hind III.

The regions of the DNA in each plaque which codes for portions of the homologous gene for the WGL⁺ antigen from each species of tick are sequenced and engineered for expression in recombinant organisms essentially as described above for the Boophilus microplus WGL⁺ antigen. The same approach can also be taken to isolate cDNA clones from these and other tick species.

The homologous WGL⁺ antigen proteins expressed by the microorganisms are then grown in fermenters, the expression of the recombinant antigen induced and the antigen is purified formulated with an adjuvant or carrier and used to vaccinate animals.

It is understood that this procedure can be equally well applied to any species of tick to isolate clones coding for WGL⁺ related antigens and the WGL⁺ related proteins expressed by the so constructed genetically engineered microorganisms can be used as effective vaccines against a range of tick species which are responsible for productivity losses, morbidity and mortality to domestic animals and man.

In summary, it is clear that the DNA sequence shown in Table 9 codes for one homologue of the tick WGL⁺ polypeptide as 16 of the 17 endo lys-C peptide fragments shown which were generated from the antigen Isolated from ticks can be coded for by that DNA sequence. Evidence has been obtained that homologues of that gene may exist in the tick population and these are included within the scope of this invention whether the homologues originate from Boophilus microplus or other species of ticks found world wide.

The procedures outlined above refer to the WGL⁺ antigen derived from Boophilus microplus. It is clear that this antigen or the equivalent antigen isolated from other tick species may well be effective against other species of Boophilus such as B. annulatus, other tick species such as Haemaphysalis spp, Otobius spp, Rhiphicephalus spp, Ambylomma spp. Dermacentor spp, Ixodes spp and Hyalomma spp, and particular species thereof including Otobius megnini, Rhiphicephalus appendiculatus, Amblyomma variegatum, Haemaphysalis longicornis, Dermacentor andersoni, D. variabilis and Ixodes holocyclus each of which causes significant economic loss throughout the world either as a result of infestation or as vectors of diseases such as Babesia bovis, Babesia bigemina, Anaplasma marginale, Cowdria ruminatum, Theileria parva parva, T. parva lawrencil, T. annulata and T. hirci. The WGL⁺ gene product or the equivalent gene product from the related and other acarines would be expected to provide effective vaccines against the parasites as an extension of the work presented herein.

### DEPOSITION OF MICROORGANISMS

Strain BTA 1751 referred to herein has been deposited with the American Type Culture Collection of 12301 Parklawn Drive Rockville MD 20852 USA in accordance with the provisions of the Budapest Treaty on 26 october 1987 under accession number ATCC 67548.

Strain BTA 1751 has also been deposited with the China Centre for Type Culture Collection under import licence IL-87044 and designated CTCC.

### INDUSTRIAL APPLICATION

The current invention provides a means of vaccinating cattle against infestation with ticks such as Boophilus microplus, Boophilus annulatus; other species such as Haemaphysalis spp, Otobius spp, Rhiphicephalus spp, Ambylomma spp, Dermacentor spp, Ixodes spp and Hyalomma spp; and particular examples thereof including Otobius megnini, Rhiphicephalus appendiculatus, Dermacentor andersoni, D*.* variabilis and Ixodes holocvclus. Further it provides a means of protecting cattle against diseases such as those caused by Babesia bovis, Cowdria ruminatum, Theleria parva parva, T. parva lawrencil, T. annulata and T. hirci. Further it provides diagnostic tools for the identification and quantification of tick antigens.

### REFERENCES

1. Brown, S.J., Shapiro, S.Z. and Askenase, P.W. J. Immunol. 133, 1984, 3319-3325.
2. Ackerman, S., Floyd, M. and Sonenshine, D.E. J. Med. Entomol. 17, 1980, 391-397.
3. McGowan, M.J., Barker, M.J., Homer, J.T., McNew, R.W. and Holscher, K.M. 1971, J. Med. Entomol. 18, 1981, 328.
4. Wikel, S.K., Am. J. Trop. Med. Hyg. 30, 1981, 284.
5. Allen, J.R. and Humphries, S.J. Nature, 280, 1979, 481-493.
6. Johnston, L.A.Y., Kemp, D.H. and Pearson, R.D. Int. J. Parasitol. 16, 27-34, 1986.
7. Kemp, D.H., Agbede, R.I.S., Johnston, L.A.Y. and Gough, J.M. Int. J. Parasitol. 16, 121-130, 1986.
8. Agbede, R.I.S. and Kemp, D.H. Int. J. Parasitol. 16, 35-42, 1986.
9. Briggs M.S. and Gierasch, L.M. (1986), Molecular Mechanisms of Protein Secretion: The Role of the Signal Sequence, pages 110-180 in Advances in Protein Chemistry, vol. 38, Academic Press.
10. Maniatis, T., Fritsch, E.F. and Sambrook, J. (1982), Molecular cloning: A Laboratory Manual (Cold Spring Harbour Laboratory).
11. Kornfeld, R. and Kornfeld, S., 1985, Ann. Rev. Biochem 54, 631-664
12. Van Hemert, F.J., Amons, R., Pluijms, W.J.M., Van Ormondt, H. and Moeller, W. EMBO 3, 1109-1113 1984
13. Willadsen, P., Int. J. Parasitol, 17, 671-677 (1987)
14. Vretblad, P., Biochemica and Biophysica Acta, 434, 169-176 (1976).
15. Sage, H.J. and Green, R.W., in Methods in Enzymology, 28, Guinsburg, V., ed., 332-339 (1972), London Academic Press.

## Claims

1. An isolated antigen, WGL⁺, having substantially the amino acid sequence: 
10 20 30 40 50
MRGIALFVAA VSLIVEGTAE SSICSDFGNE FCRNAECEVV PGAEDDFVCK
60 70 80 90 100
CPRDNMYFNA AEKQCEYKDT CKTRECSYGR CVESNPSKAS CVCEASDDLT
110 120 130 140 150
LQCKIKNDYA TDCRNRGGTA KLRTDGFIGA TCDCGEWGAM NMTTRNCVPT
160 170 180 190 200
TCLRPDLTCK DLCEKNLLQR DSRCCQGWNT ANCSAAPPAD SYCSPGSPKG
210 220 230 240 250
PDGQCINACK TKEAGFVCKH GCRSTGKAYE CTCPSGSTVA EDGITCKSIS
260 270 280 290 300
HTVSCTAEQK QTCRPTEDCR VHKGSVLCEC PWNQHLVGDT CISDCVDKKC
310 320 330 340 350
HEEFMDCGVY MNRQSCYCPW KSRKPGPNVN INECLLNEYY YTVSFTPNIS
360 370 380 390 400
FDSDHCKWYE DRVLEAIRTS IGKEVFKVEI LNCTQDIKAR LIAEKPLSKH
410 420 430 440 450
VLRKLQACEH PIGEWCMMYP KLLIKKNSAT EIEEENLCDS LLKDQEAAYK
460 470 480 490 500
GQNKCVKVDN LFWFQCADGY TTTYEMTRGR LRRSVCKAGV SCNENEQSEC
510 520 530 540 550
ADKGQIFVYE NGKANCQCPP DTKPGEIGCI ERTTCNPKEI QECQDKKLEC
560 570 580 590 600
VYKNHKAECE CPDDHECYRE PAKDSCSEED NGKCQSSGQR CVIENGKAVC
610 620 630 640 650
KEKSEATTAA TTTTKAKDKD PDPGKSSAAA VSATGLLLLL AATSVTAASL
660 670
 *GRCPT*IRN SLNMYIYITL TSNT*LGF, and parts and homologues thereof, which antigen parts and homologues induce immunity to infestation of a mammalian host to which said parts or homologues have been administered, characterized in that said immunity results in the mammalian host producing an immune response which damages the plasma membrane of the gut cells of ticks feeding on said host to such an extent that the majority of said ticks fail to survive to adult stage, or surviving ticks become red in colour and the reproductive capacity of said surviving ticks is substantially decreased or the engorgement weight of said surviving ticks is decreased.

2. An antigen according to claim 1, which antigen has a pI of between 5.30 and 5.67 and a molecular weight of approximately 89 kilodaltons.

3. An antigen according to claim 1 or claim 2 which antigen is a glycoprotein.

4. An antigen according to any one of claims 1 to 3 in substantially homogeneously pure form.

5. An antigen according to any one of claims 1 to 4 wherein said mammalian host is a bovine, horse, deer, goat, dog, cat, sheep or pig.

6. A homologue according to claim 1, wherein the homologue is an antigen of a Boophilus spp, Haemaphysalis spp, Otobius spp, Rhiphicephalus spp, Ambylomma spp, Dermacentor spp, Ixodes spp, or Hyalomma spp.

7. A homologue according to claim 6 wherein the homologue is an antigen of B. annulatus, B. decoloratus, Otobius megnini, Rhiphicephalus appendiculatus, Dermacentor andersoni, D. variabilis, Haemaphysalis longicornis, Ambylomma variegatum or Ixodes holocyclus.

8. An antigen or homologue according to any one of claims 1 to 7 wherein the immunity induced is immunity to infestation by a Haemaphysalis spp, Otobius spp, Rhiphicephalus spp, Ambylomma spp, Dermacentor spp, Ixodes spp or Hyalomma spp.

9. An antigen or homologue according to claim 8 wherein the immunity induced is immunity to infestation by Otobius megnini, Rhiphicephalus appendiculatus, Dermacentor andersoni, D. variabilis, Haemaphysalis longicornis, Ambylomma variegatum or Ixodes holocyclus.

10. An antigen or homologue according to any one of claims 1 to 7 wherein the immunity induced is immunity to infestation by a Boophilus species.

11. An antigen or homologue according to claim 10 wherein the immunity induced is immunity to B. microplus infestation.

12. An antigen or homologue according to claim 10 wherein the immunity induced is immunity to B. annulatus or B. decoloratus infestation.

13. An antigen or homologue according to any one of claims 1 to 12 wherein said antigen or homologue also provides protection against diseases caused by agents such as Babesia bovis, B. bigemia, Anaplasma marginale, Cowdria ruminatum, Theleria parva parva, T. parva lawrencii, T. annulata or T. hirci as a consequence of tick control.

14. An antigen according to any one of claims 1 to 4 comprising a polypeptide having substantially the amino acid sequence: 
ATAAVVRRSEMRGIALFVAAVSLIVEGTAESSICSDFGNEFCRNAECEVVPGAEDDFVCK
CPRDNMYFNAAEKQCEYKDTCKTRECSYGRCVESNPSKASCVCEASDDLTLQCKIKNDYA
TDCRNRGGTAKLRTDGFIGATCDCGEWGAMNMTTRNCVPTTCLRPDLTCKDLCEKNLLQR
DSRCCQGWNTANCSAAPPADSYCSPGSPKGPDGQCINACKTKEAGFVCKHGCRSTGKAYE
CTCPSGSTVAEDGITCKSISHTVSCTAEQKQTCRPTEDCRVHKGTVLCECPWNQHLVGDT
CISDCVDKKCHEEFMDCGVYMNRQSCYCPWKSRKPGPNVNINECLLNEYYYTVSFTPNIS
FDSDHCKWYEDRVLEAIRTSIGKEVFKVEILNCTQDIKARLIAEKPLSKHVLRKLQACEH
PIGEWCMMYPKLLIKKNSATEIEEENLCDSLLKDQEAAYKGQNKCVKVDNLFWFQCADGY
TTTYEMTRGRLRRSVCKAGVSCNENEQSECADKGQIFVYENGKANCQCPPDTKPGEIGCI
ERTTCNPKEIQECQDKKLECVYKNHKAECECPDDHECYREPAKDSCSEEDNGKCQSSGQR
CVIENGKAVCKEKSEATTAATTTTKAKDKDPDPGKSSAAAVSATGLLLLLAATSVTAASL
 *GRCPT*IRNSLNMYIYITLTSNT*LGF, wherein "*" represents a stop codon, or parts, and homologues thereof, which antigen parts and homologues induce immunity to tick infestation of a mammalian host to which said parts or homologues have been administered, characterized in that said immunity results in the mammalian host producing an immune response which damages the plasma membrane of the gut cells of ticks feeding on said host to such an extent that the majority of said ticks fail to survive to adult stage or surviving ticks become red in colour and the reproductive capacity of said surviving ticks is substantially decreased or the engorgement weight of said surviving ticks is decreased.

15. A polypeptide according to claim 14 in glycosylated form.

16. WGL⁺ according to any one of claims 1, 2 or 4 in unglycosylated form.

17. A polypeptide selected from those having the amino acid sequence:

F1 (K) D P D P G K
F2 (K) W Y E D (G) V L E A I (X) T S I G K
F3 (K) (X) Q A C E (H) P I G E (W) C M M Y P K
(C)
F4 (K) E A G F V Q K
(S) (V) (V) (I)
F5 (K) G (P) (D) G Q (C) I N (A) (C) K
(G)
F6 (K) A (D) V S T N E N E Q L E Q A D K
(G)
F7 (K) S (D) T Q (X) I D H I S K
N (A) (A)
F8 (K) D Q E (Y) (Y) Y

F9 [(K) C P C D N M Y F N A A E E I G C I E]
[(K) A N R Q C P P D T R R G K ]
F10 [(K) A P R Q N M Y F N A A E E I G C I E]
[(K) C N C D C P P D T R P G K ]
F11 (K) W Y E D R V L E A I R T S I G K
F12 (K) E S S I C X D F G N E F C R N A E C E V V P
F13 (K) T R E C S Y G R C V E S N P S K
F14 (K) A Y E C T C P R A F T V A E D G I S/H C K
[(K) D E V D N A S/H L V C Q N A ]
F15 [(K) N V L Q S D G C G P Y ]
[(K) C L N P R P/L R L K H/S ]
S
F16 (K) A X V L C E X P
C
G
and
F17 (K) L Q A C E H P I

18. A polypeptide according to claim 17 comprising the amino acid sequence:

F1        K D P D G K
F2, F11   K W Y E D R V L E A I R T S I G K
F3, F17   K L Q A C E H P I G E W C M M Y P K
F4        K E A G F V C K
F5        K G P D G Q C I N A C K
F6        K A G V S C N E N E Q S E C A D K
F8        K D Q E A A Y K
F9, F10   K C P R D N M Y F N A A E K
          K A N C Q C P P D T K P G E I G C I E
          K A N C Q C P P D T R P G E I G C I E
F12       A E S S I C S D F G N E F C R N A E C E V V P G
F13       K T R E C S Y G R C V E S N P S K
F14       K A Y E C T C P S G S T V A E D G I T C K
F15       K N L L Q R D S R C C Q
or
F16       K G T V L C E C P

19. A polynucleotide sequence which acts as a coding sequence for amino acid sequences of an antigen according to claim 1 or claim 2 or of a polypeptide according to any one of claims 14 to 16 with the proviso that the polynucleotide sequence does not include a naturally occurring polynucleotide sequence as it exists in its native environment.

20. A polynucleotide sequence according to claim 19 wherein said sequence is a DNA sequence.

21. A DNA sequence according to claim 20 wherein said DNA sequence is a cDNA sequence.

22. A DNA sequence comprising substantially: 
CCGCGACAGCTGCGGTGGTTCGACGCAGTGAG
ATG CGT GGC ATC GCT TTG TTC GTC GCC GCT GTT TCA CTG ATT GTA
GAG GGC ACA GCA GAA TCA TCC ATT TGC TCT GAC TTC GGG AAC GAG
TTC TGT CGC AAC GCT GAA TGT GAA GTG GTG CCT GGT GCA GAG GAT
GAT TTC GTG TGC AAA TGT CCG CGA GAT AAT ATG TAC TTC AAT GCT
GCT GAA AAG CAA TGC GAA TAT AAA GAC ACG TGC AAG ACA AGG GAG
TGC AGC TAT GGA CGT TGC GTT GAA AGT AAC CCG AGC AAG GCT AGC
TGC GTC TGC GAA GCA TCG GAC GAT CTA ACG CTA CAA TGC AAA ATT
 
AAA AAT GAC TAC GCA ACT GAC TGC CGA AAT CGA GGT GGC ACT GCT
AAG TTG CGC ACG GAT GGG TTT ATT GGC GCA ACG TGT GAC TGT GGT
GAA TGG GGT GCG ATG AAC ATG ACC ACC CGG AAC TGT GTC CCT ACC
ACG TGT CTT CGT CCC GAC TTG ACC TGC AAA GAC CTC TGC GAG AAA
AAC CTG CTT CAA AGG GAT TCT CGT TGT TGC CAG GGG TGG AAC ACA
GCA AAC TGT TCA GCC GCT CCT CCA GCT GAC TCC TAT TGC TCT CCT
GGG AGC CCC AAA GGA CCG GAC GGA CAG TGT ATA AAT GCT TGC AAG
ACG AAA GAA GCT GGG TTT GTC TGC AAG CAT GGA TGC AGG TCG ACC
GGC AAG GCG TAC GAG TGC ACG TGC CCG AGT GGC TCT ACC GTC GCC
GAA GAT GGC ATT ACC TGC AAA AGT ATT TCG CAC ACA GTC AGC TGC
ACT GCT GAG CAA AAA CAG ACC TGC CGC CCA ACC GAA GAC TGT CGT
GTG CAC AAA GGA ACT GTG TTG TGT GAG TGC CCG TGG AAT CAA CAT
CTA GTG GGG GAC ACG TGC ATA AGT GAT TGC GTC GAC AAG AAA TGC
CAC GAA GAA TTT ATG GAC TGT GGC GTA TAT ATG AAT CGA CAA AGC
TGC TAT TGT CCA TGG AAA TCA AGG AAG CCG GGC CCA AAT GTC AAC
ATC AAT GAA TGC CTA CTG AAT GAG TAT TAC TAC ACG GTG TCA TTC
ACC CCA AAC ATA TCT TTT GAT TCT GAC CAT TGC AAA TGG TAT GAG
GAT CGT GTT TTG GAA GCG ATA CGG ACC AGT ATC GGA AAA GAA GTT
TTT AAG GTT GAG ATA CTT AAC TGC ACG CAG GAC ATT AAG GCA AGA
CTC ATA GCA GAG AAA CCA CTG TCA AAA CAC GTG CTC AGG AAA CTA
CAA GCA TGC GAG CAT CCA ATC GGC GAA TGG TGC ATG ATG TAT CCG
AAG TTG CTG ATC AAG AAA AAC TCT GCA ACA GAA ATC GAA GAA GAG
AAC CTT TGC GAC AGT CTG CTC AAG GAT CAG GAA GCT GCC
TAC AAA GGT CAA AAC AAA TGC GTC AAG GTC GAC AAC CTC TTC TGG
TTC CAG TGC GCT GAT GGT TAC ACA ACA ACT TAC GAG ATG ACA CGA
GGT CGC CTA CGC CGC TCC GTG TGT AAA GCT GGA GTT TCT TGC AAC
GAA AAC GAG CAG TCG GAG TGT GCT GAC AAA GGG CAA ATA TTT GTT
TAC GAA AAC GGC AAA GCG AAT TGC CAA TGC CCA CCA GAC ACT AAA
CCT GGG GAG ATT GGC TGC ATT GAG CGT ACC ACA TGC AAC CCT AAA
GAA ATA CAA GAA TGC CAA GAC AAG AAG CTG GAG TGC GTT TAC AAA
AAC CAT AAA GCA GAA TGC GAG TGT CCT GAT GAT CAC GAG TGT TAC
AGG GAG CCT GCC AAA GAC TCT TGC AGT GAA GAG GAT AAT GGT AAA
TGT CAA AGC AGT GGG CAG CGT TGT GTA ATA GAA AAC GGA AAG GCT
GTT TGC AAG GAA AAG TCT GAA GCA ACA ACA GCT GCG ACT ACA ACA
ACG AAA GCG AAA GAC AAG GAT CCA GAT CCT GGA AAG TCA AGT GCT
GCA GCA GTA TCA GCT ACT GGG CTC TTG TTA CTG CTC GCA GCT ACT
TCA GTC ACC GCA GCA TCG TTG TAA GGA AGA TGT CCA ACT TGA ATA
CGG AAC
 GCTTGAATATGTATATATACATCACGCTTACATCGAACACCTAGCTTGGTTT or parts thereof encoding polypeptides which induce immunity to tick infestation of a mammalian host to which said polypeptide has been administered, characterized in that said immunity results in the mammalian host producing an immune response which damages the plasma membrane of the gut cells of ticks feeding on said host to such an extent that the majority of said ticks fail to survive to adult stage or surviving ticks become red in colour and the reproductive capacity of said surviving ticks is substantially decreased or the engorgement weight of said surviving ticks is decreased, and excluding the sequence as it exists in its native environment.

23. A hybridization probe selected from:

20-mer    5' T T A C C T G G A T C T G G A T C C T T 3'
50-mer    TTA CCA ATG GAT GTA CAA ATA GCT TCA AGG ACA CCA
          TCT TCG TAC CAC TT
                                                                  T
53-mer    TTTGGGTACATCATACACCATTCACCAATTGGGTGTTCACAAGCCTGAGG
          CTT                                                     AC
72-mer    5' TTT AGG TAC AAC CTC ACA TTC AGC ATT CCT ACA AAA
          TTC ATT ACC GAA ATC AAA ACA AAT ACT ACT CTC CTT 3'
51-mer    5' CTT CGA CGG ATT GGA TTC GAC GCA TCT GCC ATA GCT
          ACA TTC CCT CGT CTT 3'
or
63-mer    5' CTT GCA ATG GAT TCC ATC CTC GGC GAC AGT GAA AGC
          TCT AGG GCA AGT GCA CTC ATA AGC CTT 3'

24. A recombinant DNA molecule comprising at least one DNA sequence according to any one of claims 20 to 22 and vector DNA.

25. A recombinant DNA molecule according to claim 24 wherein said vector DNA comprises phage, viral or plasmid DNA.

26. A recombinant DNA molecule according to claim 24 or claim 25 wherein said vector DNA comprises lambda gt11, pUR290, pUR291, pUR282, pUK270, pUC8, pUC9, baculovirus, pZipNeo an SV40 based vector, αgt10, an EMBL vector, pBR327, pBR329 or pBR329 containing a par locus.

27. pBTA 707.

28. A recombinant DNA molecule according to any one of claims 24 to 27 which recombinant DNA molecule additionally comprises an expression control sequence.

29. A recombinant DNA molecule according to claim 28 wherein said expression control sequence comprises a promoter and a translation start signal.

30. A transformant comprising a host cell line transformed with at least one recombinant DNA molecule according to any one of claims 24 to 29.

31. A transformant according to claim 30 wherein said host comprises a bacterial, yeast, mammalian or insect cell line.

32. A transformant according to claim 31 wherein said transformant host is Y 1090 or or Y 1089 or JM 101.

33. A transformant as identified by accession number ATCC 67548.

34. An expression product of a host cell line grown in vitro comprising an antigen according to claim 1 or claim 2 or a polypeptide according to claim 14 or claim 15.

35. A vaccine comprising at least one antigen or homologue according to any one of claims 1 to 12 or a polypeptide according to any one of claims 14 to 16 or an expression product according to claim 34 and a carrier, adjuvant, immunopotentiator or diluent.

36. A vaccine according to claim 35 comprising WGL⁺ according to claim 1 in glycosylated or unglycosylated form.

37. An idiotype antibody raised against an antigen or homologue according to any one of claims 1 to 12 or a polypeptide according to any one of claims 14 to 16.

38. An anti-idiotype antibody raised against an antibody according to claim 37.

39. A vaccine comprising an idiotype or an anti-idiotype antibody according to claim 37 or claim 38 respectively.

40. A process for the preparation of an antigen or homologue according to any one of claims 1 to 12 or a polypeptide according to any one of claims 14 to 16 which process comprises subjecting said antigen, homologue or said polypeptide to a chromatographic step performed on wheat germ lectin or on a lectin having the same or similar terminal sugar specificity as wheat germ lectin whereby said antigen, homologue or polypeptide is bound to said lectin and then eluted therefrom.

41. A process for the preparation of an antigen or homologue according to claim 40 said process comprising extracting membrane enriched fractions obtained from homogenised ticks with detergent and subjecting the solubilised material to wheat germ lectin sepharose chromatography and elution with N-acetylglucosamine or chromatography using a lectin having the same or similar terminal sugar specificity to wheat germ lectin.

42. A process according to claim 41 wherein said detergent is selected from NP40 or an NP40 derivative, Zwittergent 3-14 or SDS.

43. A process according to claim 42 which process additionally comprises Concanavalin A-Sepharose chromatography and elution with methyl-a-D-mannopyranoside.

44. A process according to claim 43 which process additionally comprises a preparative isoelectric focussing step.

45. A process according to claim 44 which process additionally comprises size exclusion chromatography.

46. A process according to any one of claims 41 to 45 which process comprises:
preparation of a homogenate of ticks;
centrifugation to produce membrane enriched fractions;
treatment of said membrane fractions with detergent;
chromatography of the detergent soluble material on wheat germ lectin affinity columns or on columns with the same or similar terminal sugar specificity to wheat germ lectin;
separation of lectin binding antigens by isoelectricfocussing in buffer containing detergent;
chromatography of said antigens by size exclusion HPLC in buffer containing detergent;
chromatography of resulting fractions by HPLC chromatography;
and analysis of resulting fractions by SDS-PAGE.

47. A process according to claim 46 wherein said membranes are treated with Zwittergent 3-14.

48. A process according to claim 46 or claim 47 wherein said wheat germ lectin columns are wheat germ lectin sepharose 6B columns.

49. A process according to any one of claims 46 to 48 wherein said buffers contain Zwittergent 3-14 as detergent.

50. A process according to any one of claims 46 to 49 wherein said size exclusion HPLC is on Bio-Sil Tsk 4000 and PP 300 SW columns in series.

51. An antigen produced by a process according to any one of claims 40 to 50.

52. A process for the preparation of a recombinant DNA molecule according to any one of claims 24 to 29 which process comprises inserting a DNA sequence according to any one of claim 20 to 22 into vector DNA.

53. A process for the preparation of a transformant cell line according to any one of claims 30 to 33 which process comprises inserting a recombinant DNA molecule according to any one of claims 24 to 29 into a host cell line.

54. A process for the biosynthesis of an antigen, WGL⁺, or a part or homologue thereof, which is capable of inducing immunity to tick infestation of a mammalian host to which said antigen part or homologue has been administered characterized in that said immunity results in the mammaliam host producing an immune response which damages the plasma membrane of the gut cells of ticks feeding on said host to such an extent that the majority of said ticks fail to survive to adult stage or surviving ticks become red in colour and the reproductive capacity of said surviving ticks is substantially decreased or the engorgement weight of surviving ticks is decreased, or a polypeptide according to any one of claims 14 to 16 which process comprises providing a recombinant DNA molecule according to any one of claims 24 to 29; transforming a host cell line with said recombinant DNA molecule so that said host is capable of expressing a proteinaceous product comprising said antigen or polypeptide; culturing said host to obtain said expression; and collecting said proteinaceous product.

55. A process for the biosynthesis of an antigen according to claim 54 wherein said antigen is formed as an inclusion body.

56. An isolated antigen LL⁺ having a pI of 4.8 to 5.0, a molecular weight of 80-90 kD and an amino acid composition as follows:
| | |
|---|---|
| Asp | 11.0 |
| Glu | 10.3 |
| Ser | 7.4 |
| Gly | 10.5 |
| His | 2.9 |
| Arg | 5.2 |
| Thr | 5.6 |
| Ala | 6.8 |
| Pro | 5.2 |
| Tyr | 3.9 |
| Val | 6.5 |
| Met | 2.9 |
| Cys | 0.5 |
| Ile | 4.5 |
| Leu | 8.8 |
| Phe | 4.0 |
| Lys | 3.8 |

57. LL⁺ according to claim 56 in unglycosylated form.

58. An expression product of a transformant host transformed with a recombinant DNA molecule encoding LL⁺ as hereinbefore defined, said expression product comprising LL⁺ in glycosylated or unglycosylated form.

59. An epitope of an antigen according to any one of claims 1 to 4 which epitope induces immunity to infestation of a mammalian host to which said epitope has been administered, characterized in that said immunity results in the mammalian host producing an immune response which damages the plasma membrane of the gut cells of ticks feeding on said host to such an extent that the majority of said ticks fail to survive to adult stage, or surviving ticks become red in colour and the reproductive capacity of said surviving ticks is substantially decreased or the engorgement weight of said surviving ticks is decreased.

60. An epitope according to claim 59 when produced synthetically or as a result of chemical or enzymatic cleavage of the antigen.

## Patentansprüche

1. Isoliertes Antigen WGL⁺, das im wesentlichen die Aminosäuresequenz: 
10 20 30 40 50
MRGIALFVAA VSLIVEGTAE SSICSDFGNE FCRNAECEVV PGAEDDFVCK
60 70 80 90 100
CPRDNMYFNA AEKQCEYKDT CKTRECSYGR CVESNPSKAS CVCEASDDLT
110 120 130 140 150
LQCKIKNDYA TDCRNRGGTA KLRTDGFIGA TCDCGEWGAM NMTTRNCVPT
160 170 180 190 200
TCLRPDLTCK DLCEKNLLQR DSRCCQGWNT ANCSAAPPAD SYCSPGSPKG
210 220 230 240 250
PDGQCINACK TKEAGFVCKH GCRSTGKAYE CTCPSGSTVA EDGITCKSIS
260 270 280 290 300
HTVSCTAEQK QTCRPTEDCR VHKGSVLCEC PWNQHLVGDT CISDCVDKKC
310 320 330 340 350
HEEFMDCGVY MNRQSCYCPW KSRKPGPNVN INECLLNEYY YTVSFTPNIS
360 370 380 390 400
FDSDHCKWYE DRVLEAIRTS IGKEVFKVEI LNCTQDIKAR LIAEKPLSKH
410 420 430 440 450
VLRKLQACEH PIGEWCMMYP KLLIKKNSAT EIEEENLCDS LLKDQEAAYK
460 470 480 490 500
GQNKCVKVDN LFWFQCADGY TTTYEMTRGR LRRSVCKAGV SCNENEQSEC
510 520 530 540 550
ADKGQIFVYE NGKANCQCPP DTKPGEIGCI ERTTCNPKEI QECQDKKLEC
560 570 580 590 600
VYKNHKAECE CPDDHECYRE PAKDSCSEED NGKCQSSGQR CVIENGKAVC
610 620 630 640 650
KEKSEATTAA TTTTKAKDKD PDPGKSSAAA VSATGLLLLL AATSVTAASL
660 670
 *GRCPT*IRN SLNMYIYITL TSNT*LGF aufweist, sowie Teile und Homologe davon, welche Antigenteile und Homologe Immunität gegen den Befall eines Säugerwirts induzieren, dem die Teile oder Homologe verabreicht wurden, dadurch gekennzeichnet, daß als Ergebnis der Immunität der Säugerwirt eine Immunantwort produziert, welche die Plasmamembran der Darmzellen von Zecken, die sich von dem Wirt ernähren, in einem solchem Grad schädigt, daß die Mehrheit der Zecken nicht bis zum adulten Stadium überlebt, oder die überlebenden Zecken eine rote Farbe annehmen und die Reproduktionsfähigkeit der überlebenden Zecken wesentlich vermindert ist oder das Gewicht der überlebenden Zecken im vollgesaugten Zustand ("engorgement weight") verringert ist.

2. Antigen nach Anspruch 1, welches Antigen einen pl von 5,30 bis 5,67 und ein Molekulargewicht von etwa 89 Kilodalton aufweist.

3. Antigen nach Anspruch 1 oder Anspruch 2, welches Antigen ein Glycoprotein ist.

4. Antigen nach irgendeinem der Ansprüche 1 bis 3 in im wesentlichen homogener reiner Form.

5. Antigen nach irgendeinem der Ansprüche 1 bis 4, wobei der Säugerwirt ein(e) Rind, Pferd, Hirsch, Ziege, Hund, Katze, Schaf oder Schwein ist.

6. Homologes nach Anspruch 1, wobei das Homologe ein Antigen eines Boophilus spp, Haemaphysalis spp, Otobius spp, Rhiphicephalus spp, Ambylomma spp, Dermacentor spp, Ixodes spp oder Hyalomma spp ist.

7. Homologes nach Anspruch 6, wobei das Homologe ein Antigen von B. annulatus, B. decoloratus, Otobius megnini, Rhiphicephalus appendiculatus, Dermacentor andersoni, D. variabilis, Haemaphysalis longicornis, Ambylomma variegatum oder Ixodes holocyclus ist.

8. Antigen oder Homologes nach irgendeinem der Ansprüche 1 bis 7, wobei die induzierte Immunität eine Immunität gegen den Befall durch einen Haemaphysalis spp, Otobius spp, Rhiphicephalus spp, Ambylomma spp, Dermacentor spp, Ixodes spp oder Hyalomma spp ist.

9. Antigen oder Homologes nach Anspruch 8, wobei die induzierte Immunität eine Immunität gegen den Befall durch Otobius megnini, Rhiphicephalus appendiculatus, Dermacentor andersoni, D. variabilis, Haemaphysalis longicornis, Ambylomma variegatum oder Ixodes holocyclus ist.

10. Antigen oder Homologes nach irgendeinem der Ansprüche 1 bis 7, wobei die induzierte Immunität eine Immunität gegen den Befall durch eine Boophilus-Spezies ist.

11. Antigen oder Homologes nach Anspruch 10, wobei die induzierte Immunität eine Immunität gegen den Befall durch B. microplus ist.

12. Antigen oder Homologes nach Anspruch 10, wobei die induzierte Immunität eine Immunität gegen den Befall durch B. annulatus oder B. decoloratus ist.

13. Antigen oder Homologes nach irgendeinem der Ansprüche 1 bis 12, wobei das Antigen oder Homologe auch Schutz verleiht gegen Krankheiten, die von Agenzien wie Babesia bovis, B. bigemia, Anaplasma marginale, Cowdria ruminatum, Theleria parva parva, T. parva Lawrencii T. annulata oder T. hirci als Folge einer Zeckenbekämpfung verursacht werden.

14. Antigen nach irgendeinem der Ansprüche 1 bis 4, umfassend ein Polypeptid, das im wesentlichen die Aminosäuresequenz: 
ATAAVVRRSEMRGIALFVAAVSLIVEGTAESSICSDFGNEFCRNAECEVVPGAEDDFVCK
CPRDNMYFNAAEKQCEYKDTCKTRECSYGRCVESNPSKASCVCEASDDLTLQCKIKNDYA
TDCRNRGGTAKLRTDGFIGATCDCGEWGAMNMTTRNCVPTTCLRPDLTCKDLCEKNLLQR
DSRCCQGWNTANCSAAPPADSYCSPGSPKGPDGQCINACKTKEAGFVCKHGCRSTGKAYE
CTCPSGSTVAEDGITCKSISHTVSCTAEQKQTCRPTEDCRVHKGTVLCECPWNQHLVGDT
CISDCVDKKCHEEFMDCGVYMNRQSCYCPWKSRKPGPNVNINECLLNEYYYTVSFTPNIS
FDSDHCKWYEDRVLEAIRTSIGKEVFKVEILNCTQDIKARLIAEKPLSKHVLRKLQACEH
PIGEWCMMYPKLLIKKNSATEIEEENLCDSLLKDQEAAYKGQNKCVKVDNLFWFQCADGY
TTTYEMTRGRLRRSVCKAGVSCNENEQSECADKGQIFVYENGKANCQCPPDTKPGEIGCI
ERTTCNPKEIQECQDKKLECVYKNHKAECECPDDHECYREPAKDSCSEEDNGKCQSSGQR
CVIENGKAVCKEKSEATTAATTTTKAKDKDPDPGKSSAAAVSATGLLLLLAATSVTAASL
 *GRCPT*IRNSLNMYIYITLTSNT*LGF aufweist, worin "*" ein Stopcodon darstellt, oder Teile und Homologe davon, welche Antigenteile und Homologe Immunität gegen den Zeckenbefall eines Säugerwirts induzieren, dem diese Teile oder Homologe verabreicht wurden, dadurch gekennzeichnet, daß als Ergebnis der Immunität der Säugerwirt eine Immunantwort produziert, welche die Plasmamembran der Darmzellen von Zecken, die sich von dem Wirt ernähren, in einem solchem Grad schädigt, daß die Mehrheit der Zecken nicht bis zum adulten Stadium überlebt, oder die überlebenden Zecken eine rote Farbe annehmen und die Reproduktionsfähigkeit der überlebenden Zecken wesentlich vermindert ist oder das Gewicht der überlebenden Zecken im vollgesaugten Zustand ("engorgement weight") verringert ist.

15. Polypeptid nach Anspruch 14 in glycosylierter Form.

16. WGL⁺ nach irgendeinem der Ansprüche 1, 2 oder 4 in unglycosylierter Form.

17. Polypeptid, ausgewählt aus denjenigen mit der Aminosäuresequenz:

F1 (K) D P D P G K
F2 (K) W Y E D (G) V L E A I (X) T S I G K
F3 (K) (X) Q A C E (H) P I G E (W) C M M Y P K
(C)
F4 (K) E A G F V Q K
(S) (V) (V) (I)
F5 (K) G (P) (D) G Q (C) I N (A) (C) K
(G)
F6 (K) A (D) V S T N E N E Q L E Q A D K
(G)
F7 (K) S (D) T Q (X) I D H I S K
N (A) (A)
F8 (K) D Q E (Y) (Y) Y

F9 [(K) C P C D N M Y F N A A E E I G C I E]
[(K) A N R Q C P P D T R R G K ]
F10 [(K) A P R Q N M Y F N A A E E I G C I E]
[(K) C N C D C P P D T R P G K ]
F11 (K) W Y E D R V L E A I R T S I G K
F12 (K) E S S I C X D F G N E F C R N A E C E V V P
F13 (K) T R E C S Y G R C V E S N P S K
F14 (K) A Y E C T C P R A F T V A E D G I S/H C K
[(K) D E V D N A S/H L V C Q N A ]
F15 [(K) N V L Q S D G C G P Y ]
[(K) C L N P R P/L R L K H/S ]
S
F16 (K) A X V L C E X P
C
G

und

F17 (K) L Q A C E H P I

18. Polypeptid nach Anspruch 17, umfassend die Aminosäuresequenz:

F1         K D P D G K
F2, F11    K W Y E D R V L E A I R T S I G K
F3, F17    K L Q A C E H P I G E W C M M Y P K
F4         K E A G F V C K
F5         K G P D G Q C I N A C K
F6         K A G V S C N E N E Q S E C A D K
F8         K D Q E A A Y K
F9, F10    K C P R D N M Y F N A A E K
           K A N C Q C P P D T K P G E I G C I E
           K A N C Q C P P D T R P G E I G C I E
F12        A E S S I C S D F G N E F C R N A E C E V V P G
F13        K T R E C S Y G R C V E S N P S K
F14        K A Y E C T C P S G S T V A E D G I T C K
F15        K N L L Q R D S R C C Q

oder

F16        K G T V L C E C P

19. Polynukleotidsequenz, die als kodierende Sequenz für Aminosäuresequenzen eines Antigens nach Anspruch 1 oder Anspruch 2 oder eines Polypeptids nach irgendeinem der Ansprüche 14 bis 16 dient, mit der Maßgabe, daß die Polynukleotidsequenz keine in der Natur vorkommende Polynukleotidsequenz, wie sie in ihrer natürlichen Umgebung existiert, umfaßt.

20. Polynukleotidsequenz nach Anspruch 19, wobei die Sequenz eine DNA-Sequenz ist.

21. DNA-Sequenz nach Anspruch 20, wobei die DNA-Sequenz eine cDNA-Sequenz ist.

22. DNA-Sequenz, welche im wesentlichen umfaßt: 
CCGCGACAGCTGCGGTGGTTCGACGCAGTGAG
ATG CGT GGC ATC GCT TTG TTC GTC GCC GCT GTT TCA CTG ATT GTA
GAG GGC ACA GCA GAA TCA TCC ATT TGC TCT GAC TTC GGG AAC GAG
TTC TGT CGC AAC GCT GAA TGT GAA GTG GTG CCT GGT GCA GAG GAT
GAT TTC GTG TGC AAA TGT CCG CGA GAT AAT ATG TAC TTC AAT GCT
GCT GAA AAG CAA TGC GAA TAT AAA GAC ACG TGC AAG ACA AGG GAG
TGC AGC TAT GGA CGT TGC GTT GAA AGT AAC CCG AGC AAG GCT AGC
TGC GTC TGC GAA GCA TCG GAC GAT CTA ACG CTA CAA TGC AAA ATT
AAA AAT GAC TAC GCA ACT GAC TGC CGA AAT CGA GGT GGC ACT GCT
AAG TTG CGC ACG GAT GGG TTT ATT GGC GCA ACG TGT GAC TGT GGT
GAA TGG GGT GCG ATG AAC ATG ACC ACC CGG AAC TGT GTC CCT ACC
ACG TGT CTT CGT CCC GAC TTG ACC TGC AAA GAC CTC TGC GAG AAA
AAC CTG CTT CAA AGG GAT TCT CGT TGT TGC CAG GGG TGG AAC ACA
GCA AAC TGT TCA GCC GCT CCT CCA GCT GAC TCC TAT TGC TCT CCT
GGG AGC CCC AAA GGA CCG GAC GGA CAG TGT ATA AAT GCT TGC AAG
ACG AAA GAA GCT GGG TTT GTC TGC AAG CAT GGA TGC AGG TCG ACC
GGC AAG GCG TAC GAG TGC ACG TGC CCG AGT GGC TCT ACC GTC GCC
GAA GAT GGC ATT ACC TGC AAA AGT ATT TCG CAC ACA GTC AGC TGC
ACT GCT GAG CAA AAA CAG ACC TGC CGC CCA ACC GAA GAC TGT CGT
GTG CAC AAA GGA ACT GTG TTG TGT GAG TGC CCG TGG AAT CAA CAT
CTA GTG GGG GAC ACG TGC ATA AGT GAT TGC GTC GAC AAG AAA TGC
CAC GAA GAA TTT ATG GAC TGT GGC GTA TAT ATG AAT CGA CAA AGC
TGC TAT TGT CCA TGG AAA TCA AGG AAG CCG GGC CCA AAT GTC AAC
ATC AAT GAA TGC CTA CTG AAT GAG TAT TAC TAC ACG GTG TCA TTC
ACC CCA AAC ATA TCT TTT GAT TCT GAC CAT TGC AAA TGG TAT GAG
GAT CGT GTT TTG GAA GCG ATA CGG ACC AGT ATC GGA AAA GAA GTT
TTT AAG GTT GAG ATA CTT AAC TGC ACG CAG GAC ATT AAG GCA AGA
CTC ATA GCA GAG AAA CCA CTG TCA AAA CAC GTG CTC AGG AAA CTA
CAA GCA TGC GAG CAT CCA ATC GGC GAA TGG TGC ATG ATG TAT CCG
AAG TTG CTG ATC AAG AAA AAC TCT GCA ACA GAA ATC GAA GAA GAG
AAC CTT TGC GAC AGT CTG CTC AAG GAT CAG GAA GCT GCC
TAC AAA GGT CAA AAC AAA TGC GTC AAG GTC GAC AAC CTC TTC TGG
TTC CAG TGC GCT GAT GGT TAC ACA ACA ACT TAC GAG ATG ACA CGA
GGT CGC CTA CGC CGC TCC GTG TGT AAA GCT GGA GTT TCT TGC AAC
GAA AAC GAG CAG TCG GAG TGT GCT GAC AAA GGG CAA ATA TTT GTT
 
TAC GAA AAC GGC AAA GCG AAT TGC CAA TGC CCA CCA GAC ACT AAA
CCT GGG GAG ATT GGC TGC ATT GAG CGT ACC ACA TGC AAC CCT AAA
GAA ATA CAA GAA TGC CAA GAC AAG AAG CTG GAG TGC GTT TAC AAA
AAC CAT AAA GCA GAA TGC GAG TGT CCT GAT GAT CAC GAG TGT TAC
AGG GAG CCT GCC AAA GAC TCT TGC AGT GAA GAG GAT AAT GGT AAA
TGT CAA AGC AGT GGG CAG CGT TGT GTA ATA GAA AAC GGA AAG GCT
GTT TGC AAG GAA AAG TCT GAA GCA ACA ACA GCT GCG ACT ACA ACA
ACG AAA GCG AAA GAC AAG GAT CCA GAT CCT GGA AAG TCA AGT GCT
GCA GCA GTA TCA GCT ACT GGG CTC TTG TTA CTG CTC GCA GCT ACT
TCA GTC ACC GCA GCA TCG TTG TAA GGA AGA TGT CCA ACT TGA ATA
CGG AAC

GCTTGAATATGTATATATACATCACGCTTACATCGAACACCTAGCTTGGTTT
 oder Teile davon, kodierend für Polypeptide, die Immunität gegen den Zeckenbefall eines Säugerwirts induzieren, dem das Polypeptid verabreicht wurde, dadurch gekennzeichnet, daß als Ergebnis der Immunität der Säugerwirt eine Immunantwort produziert, welche die Plasmamembran der Darmzellen von Zecken, die sich von dem Wirt ernähren, in einem solchem Grad schädigt, daß die Mehrheit der Zecken nicht bis zum adulten Stadium überlebt, oder die überlebenden Zecken eine rote Farbe annehmen und die Reproduktionsfähigkeit der überlebenden Zecken wesentlich vermindert ist oder das Gewicht der überlebenden Zecken im vollgesaugten Zustand ("engorgement weight") verringert ist, und die Sequenz, wie sie in ihrer natürlichen Umgebung existiert, ausgeschlossen ist.

23. Hybridisierungssonde, ausgewählt aus:

20-mer 5' T T A C C T G G A T C T G G A T C C T T 3'
50-mer TTA CCA ATG GAT GTA CAA ATA GCT TCA AGG ACA CCA
TCT TCG TAC CAC TT

T
53-mer TTTGGGTACATCATACACCATTCACCAATTGGGTGTTCACAAGCCTGAGG
CTT
AC

72-mer 5' TTT AGG TAC AAC CTC ACA TTC AGC ATT CCT ACA AAA
TTC ATT ACC GAA ATC AAA ACA AAT ACT ACT CTC CTT 3'
51-mer 5' CTT CGA CGG ATT GGA TTC GAC GCA TCT GCC ATA GCT
ACA TTC CCT CGT CTT 3'
oder
63-mer 5' CTT GCA ATG GAT TCC ATC CTC GGC GAC AGT GAA AGC
TCT AGG GCA AGT GCA CTC ATA AGC CTT 3'

24. Rekombinantes DNA-Molekül, das mindestens eine DNA-Sequenz nach irgendeinem der Ansprüche 20 bis 22 und Vektor-DNA umfaßt.

25. Rekombinantes DNA-Molekül nach Anspruch 24, worin die Vektor-DNA Phagen-, Virus- oder Plasmid-DNA umfaßt.

26. Rekombinantes DNA-Molekül nach Anspruch 24 oder Anspruch 25, worin die Vektor-DNA lambda gt11, pUR290, pUR291, pUR282, pUK270, pUC8, pUC9, Baculovirus, pZipNeo, einen Vektor auf SV40-Basis, αgt10, einen EMBL-Vektor, pBR327, pBR329 oder pBR329 mit einem par-Locus umfaßt.

27. pBTA 707.

28. Rekombinantes DNA-Molekül nach irgendeinem der Ansprüche 24 bis 27, welches rekombinante DNA-Molekül ferner eine Expressions-Kontrollsequenz umfaßt.

29. Rekombinantes DNA-Molekül nach Anspruch 28, worin die Expressions-Kontrollsequenz einen Promotor und ein Translations-Startsignal umfaßt.

30. Transformant, umfassend eine Wirts-Zellinie, die mit mindestens einem rekombinanten DNA-Molekül nach irgendeinem der Ansprüche 24 bis 29 transformiert wurde.

31. Transformant nach Anspruch 30, wobei der Wirt eine Bakterien-, Hefe-, Säuger- oder Insekten-Zellinie umfaßt.

32. Transformant nach Anspruch 31, wobei der transformierte Wirt Y 1090 oder Y 1089 oder JM 101 ist.

33. Transformant, wie durch die Hinterlegungsnummer ATCC 67548 identifiziert.

34. Expressionsprodukt einer in vitro gezüchteten Wirts-Zellinie, das ein Antigen nach Anspruch 1 oder Anspruch 2 oder ein Polypeptid nach Anspruch 14 oder Anspruch 15 umfaßt.

35. Impfstoff, der mindestens ein Antigen oder Homologes nach irgendeinem der Ansprüche 1 bis 12 oder ein Polypeptid nach irgendeinem der Ansprüche 14 bis 16 oder ein Expressionsprodukt nach Anspruch 34 und ein(en) Träger, Adjuvans, Immunverstärker oder Verdünnungsmittel umfaßt.

36. Impfstoff nach Anspruch 35, der WGL⁺ nach Anspruch 1 in glycosylierter oder unglycosylierter Form umfaßt.

37. Idiotypischer Antikörper gegen ein Antigen oder Homologes nach irgendeinem der Ansprüche 1 bis 12 oder ein Polypeptid nach irgendeinem der Ansprüche 14 bis 16.

38. Anti-idiotypischer Antikörper gegen einen Antikörper nach Anspruch 37.

39. Impfstoff, der einen idiotypischen oder anti-idiotypischen Antikörper nach Anspruch 37 bzw. Anspruch 38 umfaßt.

40. Verfahren zur Herstellung eines Antigens oder Homologen nach irgendeinem der Ansprüche 1 bis 12 oder eines Polypeptids nach irgendeinem der Ansprüche 14 bis 16, welches Verfahren umfaßt, daß man das Antigen, Homologe oder Polypeptid einem Chromatographieschritt, der auf Weizenkeim-Lektin oder einem Lektin mit der gleichen oder einer ähnlichen Spezifität für endständige Zucker wie Weizenkeim-Lektin durchgeführt wird, unterzieht, wodurch das Antigen, Homologe oder Polypeptid an das Lektin gebunden und dann davon eluiert wird.

41. Verfahren zur Herstellung eines Antigens oder Homologen nach Anspruch 40, welches Verfahren umfaßt, daß man membran-angereicherte Fraktionen, die aus homogenisierten Zecken erhalten wurden, mit Detergens extrahiert und das solubilisierte Material einer Weizenkeim-Lektin-Sepharose-Chromatographie und Elution mit N-Acetylglucosamin oder einer Chromatographie unter Verwendung eines Lektins mit der gleichen oder einer ähnlichen Spezifität für endständige Zucker wie Weizenkeim-Lektin unterwirft.

42. Verfahren nach Anspruch 41, worin das Detergens aus NP40 oder einem NP40-Derivat, Zwittergent 3-14 oder SDS ausgewählt ist.

43. Verfahren nach Anspruch 42, welches Verfahren ferner Concanavalin A-Sepharose-Chromatographie und Elution mit Methyl-α-D-Mannopyranosid umfaßt.

44. Verfahren nach Anspruch 43, welches Verfahren ferner einen präparativen Schritt isoelektrischer Fokussierung umfaßt.

45. Verfahren nach Anspruch 44, welches Verfahren ferner Größenausschluß-Chromatographie umfaßt.

46. Verfahren nach irgendeinem der Ansprüche 41 bis 45, welches Verfahren umfaßt:
Herstellung eines Zeckenhomogenats;
Zentrifugation, um membran-angereicherte Fraktionen herzustellen;
Behandlung der Membran-Fraktionen mit Detergens;
Chromatographie des detergens-löslichen Materials auf Weizenkeim-Lektin-Affinitätssäulen oder auf Säulen mit der gleichen oder einer ähnlichen Spezifität für endständige Zucker wie Weizenkeim-Lektin;
Abtrennung von lektin-bindenden Antigenen durch isoelektrische Fokussierung in detergens-enthaltendem Puffer;
Chromatographie der Antigene durch Größenausschluß-HPLC in detergens-enthaltendem Puffer;
Chromatographie der resultierenden Fraktionen durch HPLC-Chromatographie;
und Analyse der resultierenden Fraktionen durch SDS-PAGE.

47. Verfahren nach Anspruch 46, worin die Membranen mit Zwittergent 3-14 behandelt werden.

48. Verfahren nach Anspruch 46 oder Anspruch 47, worin die Weizenkeim-Lektin-Säulen Weizenkeim-Lektin-Sepharose 6B-Säulen sind.

49. Verfahren nach irgendeinem der Ansprüche 46 bis 48, worin die Puffer Zwittergent 3-14 als Detergens enthalten.

50. Verfahren nach irgendeinem der Ansprüche 46 bis 49, worin die Größenausschluß-HPLC auf hintereinander angeordneten Bio-Sil Tsk 4000-und PP 300 SW-Säulen stattfindet.

51. Antigen, erzeugt durch ein Verfahren nach irgendeinem der Ansprüche 40 bis 50.

52. Verfahren zur Herstellung eines rekombinanten DNA-Moleküls nach irgendeinem der Ansprüche 24 bis 29, welches Verfahren das Inserieren einer DNA-Sequenz nach irgendeinem der Ansprüche 20 bis 22 in Vektor-DNA umfaßt.

53. Verfahren zur Herstellung einer Transformanten-Zellinie nach irgendeinem der Ansprüche 30 bis 33, welches Verfahren das Inserieren eines rekombinanten DNA-Moleküls nach irgendeinem der Ansprüche 24 bis 29 in eine Wirts-Zellinie umfaßt.

54. Verfahren zur Biosynthese eines Antigens WGL⁺, oder eines Teils oder Homologen davon, der oder das in der Lage ist, immunität gegen den Zeckenbefall eines Säugerwirts zu induzieren, dem der Antigenteil oder das Homologe verabreicht wurde, dadurch gekennzeichnet, daß als Ergebnis der Immunität der Säugerwirt eine Immunantwort, welche die Plasmamembran der Darmzellen von Zecken, die sich von dem Wirt ernähren, in einem solchem Grad schädigt, daß die Mehrheit der Zecken nicht bis zum adulten Stadium überlebt, oder die überlebenden Zecken eine rote Farbe annehmen und die Reproduktionsfähigkeit der überlebenden Zecken wesentlich vermindert ist oder das Gewicht der überlebenden Zecken im vollgesaugten Zustand ("engorgement weight") verringert ist, oder ein Polypeptid nach irgendeinem der Ansprüche 14 bis 16 produziert, welches Verfahren umfaßt das Bereitstellen eines rekombinantes DNA-Moleküls nach irgendeinem der Ansprüche 24 bis 29;
das Transformieren einer Wirts-Zellinie mit dem rekombinanten DNA-Molekül, so daß der Wirt in der Lage ist, ein proteinhaltiges Produkt, welches das Antigen oder Polypeptid umfaßt, zu exprimieren;
das Züchten des Wirts in Kultur, um die Expression zu erhalten;
und die Gewinnung des proteinhaltigen Produkts.

55. Verfahren zur Biosynthese eines Antigens nach Anspruchs 54, worin das Antigen als Einschlußkörper gebildet wird.

56. Isoliertes Antigen LL⁺, das einen pl von 4,8 bis 5,0, ein Molekulargewicht von 80-90 kD und eine Aminosäurezusammensetzung wie folgt aufweist:
| | |
|---|---|
| Asp | 11.0 |
| Glu | 10.3 |
| Ser | 7.4 |
| Gly | 10.5 |
| His | 2.9 |
| Arg | 5.2 |
| Thr | 5.6 |
| Ala | 6.8 |
| Pro | 5.2 |
| Tyr | 3.9 |
| Val | 6.5 |
| Met | 2.9 |
| Cys | 0.5 |
| Ile | 4.5 |
| Leu | 8.8 |
| Phe | 4.0 |
| Lys | 3.8 |

57. LL⁺ nach Anspruch 56 in unglycosylierter Form.

58. Expressionsprodukt eines Transformanten-Wirts, der mit einem rekombinanten DNA-Molekül, das für LL⁺ wie oben definiert kodiert, transformiert wurde, wobei das Expressionsprodukt LL⁺ in glycosylierter oder unglycosylierter Form umfaßt.

59. Epitop eines Antigens nach irgendeinem der Ansprüche 1 bis 4, welches Epitop Immunität gegen den Befall eines Säugerwirts induziert, dem das Epitop verabreicht wurde, dadurch gekennzeichnet, daß als Ergebnis der Immunität der Säugerwirt eine Immunantwort produziert, welche die Plasmamembran der Darmzellen von Zecken, die sich von dem Wirt ernähren, in einem solchem Grad schädigt, daß die Mehrheit der Zecken nicht bis zum adulten Stadium überlebt, oder die überlebenden Zecken eine rote Farbe annehmen und die Reproduktionsfähigkeit der überlebenden Zecken wesentlich vermindert ist oder das Gewicht der überlebenden Zecken im vollgesaugten Zustand ("engorgement weight") verringert ist.

60. Epitop nach Anspruch 59 als synthetisches Produkt oder als Ergebnis einer chemischen oder enzymatischen Spaltung des Antigens.

## Revendications

1. Antigène isolé, WGL⁺, comportant sensiblement la séquence d'aminoacides qui suit : 
10 20 30 40 50
MRGIALFVAA VSLIVEGTAE SSICSDFGNE FCRNAECEVV PGAEDDFVCK
60 70 80 90 100
CPRDNMYFNA AEKQCEYKDT CKTRECSYGR CVESNPSKAS CVCEASDDLT
110 120 130 140 150
LQCKIKNDYA TDCRNRGGTA KLRTDGFIGA TCDCGEWGAM NMTTRNCVPT
160 170 180 190 200
TCLRPDLTCK DLCEKNLLQR DSRCCQGWNT ANCSAAPPAD SYCSPGSPKG
210 220 230 240 250
PDGQCINACK TKEAGFVCKH GCRSTGKAYE CTCPSGSTVA EDGITCKSIS
260 270 280 290 300
HTVSCTAEQK QTCRPTEDCR VHKGSVLCEC PWNQHLVGDT CISDCVDKKC
310 320 330 340 350
HEEFMDCGVY MNRQSCYCPW KSRKPGPNVN INECLLNEYY YTVSFTPNIS
360 370 380 390 400
FDSDHCKWYE DRVLEAIRTS IGKEVFKVEI LNCTQDIKAR LIAEKPLSKH
410 420 430 440 450
VLRKLQACEH PIGEWCMMYP KLLIKKNSAT EIEEENLCDS LLKDQEAAYK
460 470 480 490 500
GQNKCVKVDN LFWFQCADGY TTTYEMTRGR LRRSVCKAGV SCNENEQSEC
510 520 530 540 550
ADKGQIFVYE NGKANCQCPP DTKPGEIGCI ERTTCNPKEI QECQDKKLEC
560 570 580 590 600
VYKNHKAECE CPDDHECYRE PAKDSCSEED NGKCQSSGQR CVIENGKAVC
610 620 630 640 650
KEKSEATTAA TTTTKAKDKD PDPGKSSAAA VSATGLLLLL AATSVTAASL
660 670
 *GRCPT*IRN SLNMYIYITL TSNT*LGF, et ses parties et homologues, lesquelles parties et homologues de l'antigène induisent l'immunité vis-à-vis de l'infestation d'un hôte mammalien auquel on a administré lesdites parties ou homologues, caractérisé en ce que ladite immunité a pour résultat que l'hôte mammalien produit une réponse immunitaire qui endommage la membrane plasmatique des cellules de l'intestin d'ixodes s'alimentant sur ledit hôte avec une ampleur telle que la majorité desdits ixodes ne survivent pas jusqu'à l'age adulte, ou que les ixodes survivants virent au rouge et que le pouvoir reproducteur desdits ixodes survivants soit sensiblement diminué ou que le poids de gonflement desdits ixodes survivants soit diminué.

2. Antigène suivant la revendication 1, caractérisé en ce qu'il possède un pI compris entre 5,30 et 5,67 et un poids moléculaire d'approximativement 89 kilodaltons.

3. Antigène suivant la revendication 1 ou la revendication 2, caractérisé en ce qu'il est une glycoprotéine.

4. Antigène suivant l'une quelconque des revendications 1 à 3 sensiblement sous une forme pure de manière homogène.

5. Antigène suivant l'une quelconque des revendications 1 à 4, caractérisé en ce que l'hôte mammalien est un bovin, un cheval, un cerf, une chèvre, un chien, un chat, un mouton ou un porc.

6. Homologue suivant la revendication 1, caractérisé en ce que l'homologue est un antigène de Boophilus spp, Haemaphysalis spp, Otobius spp, Rhiphicephalus spp, Ambylomma spp, Dermacentor spp, Ixodes spp ou Hyalomma spp.

7. Homologue suivant la revendication 6, caractérisé en ce que ce qu'il est un antigène de B. annulatus, B. decoloratus, Otobius megnini, Rhiphicephalus appendiculatus, Dermacentor andersoni, D. variabilis, Haemaphysalis longicornis, Ambylomma variegatum ou Ixodes holocyclus.

8. antigène ou homologue suivant l'une quelconque des revendications 1 à 7, caractérisé en ce que l'immunité induite est une immunité vis-à-vis de l'infestation par Haemaphysalis spp, Otobius spp, Rhiphicephalus spp, Ambylomma spp, Dermacentor spp, Ixodes spp ou Hyalomma spp.

9. Antigène ou homologue suivant la revendication 8, caractérisé en ce que l'immunité induite est une immunité vis-à-vis de l'infestation par Otobius megnini, Rhiphicephalus appendiculatus, Dermacentor andersoni, D. variabilis, Haemaphysalis longicornis, Ambylomma variegatum ou Ixodes holocyclus.

10. Antigène ou homologue suivant l'une quelconque des revendications 1 à 7, caractérisé en ce que l'immunité induite est une immunité vis-à-vis d'une infestation par une espèce Boophilus.

11. Antigène ou homologue suivant la revendication 10 dans lequel l'immunité induite est l'immunité vis-à-vis d'une infestation de B. microplus.

12. Antigène ou homologue suivant la revendication 10, dans lequel l'immunité induite est l'immunité vis-à-vis d'une infestation par B. annulatus ou B. decoloratus.

13. Antigène ou homologue suivant l'une quelconque des revendications 1 à 12, caractérisé en ce que cet antigène ou cet homologue confère également une protection contre des maladies provoquées par des agents tels que les suivants : Babesia bovis. B. bigemia, Anaplasma marginale, Cowdria ruminatum, Theleria parva parva, T. parva lawrencii, T. annulata ou T. hirci, par suite de la destruction des ixodes.

14. Antigène suivant l'une quelconque des revendications 1 à 4, comprenant un polypeptide possédant sensiblement la séquence d'aminoacides ci-dessous : 
ATAAVVRRSEMRGIALFVAAVSLIVEGTAESSICSDFGNEFCRNAECEVVPGAEDDFVCK
CPRDNMYFNAAEKQCEYKDTCKTRECSYGRCVESNPSKASCVCEASDDLTLQCKIKNDYA
TDCRNRGGTAKLRTDGFIGATCDCGEWGAMNMTTRNCVPTTCLRPDLTCKDLCEKNLLQR
DSRCCQGWNTANCSAAPPADSYCSPGSPKGPDGQCINACKTKEAGFVCKHGCRSTGKAYE
CTCPSGSTVAEDGITCKSISHTVSCTAEQKQTCRPTEDCRVHKGTVLCECPWNQHLVGDT
CISDCVDKKCHEEFMDCGVYMNRQSCYCPWKSRKPGPNVNINECLLNEYYYTVSFTPNIS
FDSDHCKWYEDRVLEAIRTSIGKEVFKVEILNCTQDIKARLIAEKPLSKHVLRKLQACEH
PIGEWCMMYPKLLIKKNSATEIEEENLCDSLLKDQEAAYKGQNKCVKVDNLFWFQCADGY
TTTYEMTRGRLRRSVCKAGVSCNENEQSECADKGQIFVYENGKANCQCPPDTKPGEIGCI
ERTTCNPKEIQECQDKKLECVYKNHKAECECPDDHECYREPAKDSCSEEDNGKCQSSGQR
CVIENGKAVCKEKSEATTAATTTTKAKDKDPDPGKSSAAAVSATGLLLLLAATSVTAASL
 *GRCPT*IRNSLNMYIYITLTSNT*LGF, où "*" représente un codon d'arrêt, ou des parties et des homologues de cet antigène, lesquelles parties et homologues de l'antigène induisent une immunité vis-à-vis de l'infestation d'ixodes d'un hôte mammalien auquel on a administré lesdites parties ou lesdits homologues, caractérisé en ce que ladite immunité a pour résultat que l'hôte mammalien produit une réponse immunitaire qui endommage la membrane plasmatique des cellules de l'intestin d'ixodes s'alimentant sur ledit hôte avec une ampleur telle que la majorité desdits ixodes ne survivent pas jusqu'à l'age adulte, ou que les ixodes survivants virent au rouge et que le pouvoir reproducteur desdits ixodes survivants soit sensiblement diminué ou que le poids de gonflement desdits ixodes survivants soit diminué.

15. Polypeptide suivant la revendication 14, sous forme glycosylée.

16. WGL⁺ suivant l'une quelconque des revendications 1, 2 et 4, sous forme non glycosylée.

17. Polypeptide choisi parmi ceux possédant la séquence d'aminoacides ci-dessous :

F1 (K) D P D P G K
F2 (K) W Y E D (G) V L E A I (X) T S I G K
F3 (K) (X) Q A C E (H) P I G E (W) C M M Y P K
(C)
F4 (K) E A G F V Q K
(S) (V) (V) (I)
F5 (K) G (P) (D) G Q (C) I N (A) (C) K
(G)
F6 (K) A (D) V S T N E N E Q L E Q A D K
(G)
F7 (K) S (D) T Q (X) I D H I S K
N (A) (A)
F8 (K) D Q E (Y) (Y) Y

F9 [(K) C P C D N M Y F N A A E E I G C I E]
[(K) A N R Q C P P D T R R G K ]
F10 [(K) A P R Q N M Y F N A A E E I G C I E]
[(K) C N C D C P P D T R P G K ]
F11 (K) W Y E D R V L E A I R T S I G K
F12 (K) E S S I C X D F G N E F C R N A E C E V V P
F13 (K) T R E C S Y G R C V E S N P S K
F14 (K) A Y E C T C P R A F T V A E D G I S/H C K
[(K) D E V D N A S/H L V C Q N A ]
F15 [(K) N V L Q S D G C G P Y ]
[(K) C L N P R P/L R L K H/S ]
S
F16 (K) A X V L C E X P
C
G

et
F17 (K) L Q A C E H P I

18. Polypeptide suivant la revendication 17, comprenant la séquence d'aminoacides ci-dessous :

F1        K D P D G K
F2, F11   K W Y E D R V L E A I R T S I G K
F3, F17   K L Q A C E H P I G E W C M M Y P K
F4        K E A G F V C K
F5        K G P D G Q C I N A C K
F6        K A G V S C N E N E Q S E C A D K
F8        K D Q E A A Y K
F9, F10   K C P R D N M Y F N A A E K
          K·A N C Q C P P D T K P G E I G C I E
          K A N C Q C P P D T R P G E I G C I E
F12       A E S S I C S D F G N E F C R N A E C E V V P G
F13       K T R E C S Y G R C V E S N P S K
F14       K A Y E C T C P S G S T V A E D G I T C K
F15       K N L L Q R D S R C C Q

ou
F16       K G T V L C E C P

19. Séquence de polynucléotides qui agit comme une séquence de codage de séquences d'aminoacides d'un antigène suivant la revendication 1 ou la revendication 2 ou d'un polypeptide suivant l'une quelconque des revendications 14 à 16, avec la condition que la séquence des polynucléotides ne comprenne pas de séquence de polynucléotides se présentant à l'état naturel comme existant dans son environnement d'origine.

20. Séquence de polynucléotides suivant la revendication 19, où cette séquence est une séquence d'ADN.

21. Séquence d'ADN suivant la revendication 20, où cette séquence d'ADN est une séquence d'ADNc.

22. Séquence d'ADN essentiellement constituée de : 
CCGCGACAGCTGCGGTGGTTCGACGCAGTGAG
ATG CGT GGC ATC GCT TTG TTC GTC GCC GCT GTT TCA CTG ATT GTA
GAG GGC ACA GCA GAA TCA TCC ATT TGC TCT GAC TTC GGG AAC GAG
TTC TGT CGC AAC GCT GAA TGT GAA GTG GTG CCT GGT GCA GAG GAT
GAT TTC GTG TGC AAA TGT CCG CGA GAT AAT ATG TAC TTC AAT GCT
GCT GAA AAG CAA TGC GAA TAT AAA GAC ACG TGC AAG ACA AGG GAG
TGC AGC TAT GGA CGT TGC GTT GAA AGT AAC CCG AGC AAG GCT AGC
TGC GTC TGC GAA GCA TCG GAC GAT CTA ACG CTA CAA TGC AAA ATT
AAA AAT GAC TAC GCA ACT GAC TGC CGA AAT CGA GGT GGC ACT GCT
AAG TTG CGC ACG GAT GGG TTT ATT GGC GCA ACG TGT GAC TGT GGT
GAA TGG GGT GCG ATG AAC ATG ACC ACC CGG AAC TGT GTC CCT ACC
ACG TGT CTT CGT CCC GAC TTG ACC TGC AAA GAC CTC TGC GAG AAA
AAC CTG CTT CAA AGG GAT TCT CGT TGT TGC CAG GGG TGG AAC ACA
GCA AAC TGT TCA GCC GCT CCT CCA GCT GAC TCC TAT TGC TCT CCT
GGG AGC CCC AAA GGA CCG GAC GGA CAG TGT ATA AAT GCT TGC AAG
ACG AAA GAA GCT GGG TTT GTC TGC AAG CAT GGA TGC AGG TCG ACC
GGC AAG GCG TAC GAG TGC ACG TGC CCG AGT GGC TCT ACC GTC GCC
GAA GAT GGC ATT ACC TGC AAA AGT ATT TCG CAC ACA GTC AGC TGC
ACT GCT GAG CAA AAA CAG ACC TGC CGC CCA ACC GAA GAC TGT CGT
GTG CAC AAA GGA ACT GTG TTG TGT GAG TGC CCG TGG AAT CAA CAT
CTA GTG GGG GAC ACG TGC ATA AGT GAT TGC GTC GAC AAG AAA TGC
CAC GAA GAA TTT ATG GAC TGT GGC GTA TAT ATG AAT CGA CAA AGC
TGC TAT TGT CCA TGG AAA TCA AGG AAG CCG GGC CCA AAT GTC AAC
ATC AAT GAA TGC CTA CTG AAT GAG TAT TAC TAC ACG GTG TCA TTC
ACC CCA AAC ATA TCT TTT GAT TCT GAC CAT TGC AAA TGG TAT GAG
GAT CGT GTT TTG GAA GCG ATA CGG ACC AGT ATC GGA AAA GAA GTT
TTT AAG GTT GAG ATA CTT AAC TGC ACG CAG GAC ATT AAG GCA AGA
CTC ATA GCA GAG AAA CCA CTG TCA AAA CAC GTG CTC AGG AAA CTA
CAA GCA TGC GAG CAT CCA ATC GGC GAA TGG TGC ATG ATG TAT CCG
AAG TTG CTG ATC AAG AAA AAC TCT GCA ACA GAA ATC GAA GAA GAG
AAC CTT TGC GAC AGT CTG CTC AAG GAT CAG GAA GCT GCC
TAC AAA GGT CAA AAC AAA TGC GTC AAG GTC GAC AAC CTC TTC TGG
 
TTC CAG TGC GCT GAT GGT TAC ACA ACA ACT TAC GAG ATG ACA CGA
GGT CGC CTA CGC CGC TCC GTG TGT AAA GCT GGA GTT TCT TGC AAC
GAA AAC GAG CAG TCG GAG TGT GCT GAC AAA GGG CAA ATA TTT GTT
TAC GAA AAC GGC AAA GCG AAT TGC CAA TGC CCA CCA GAC ACT AAA
CCT GGG GAG ATT GGC TGC ATT GAG CGT ACC ACA TGC AAC CCT AAA
GAA ATA CAA GAA TGC CAA GAC AAG AAG CTG GAG TGC GTT TAC AAA
AAC CAT AAA GCA GAA TGC GAG TGT CCT GAT GAT CAC GAG TGT TAC
AGG GAG CCT GCC AAA GAC TCT TGC AGT GAA GAG GAT AAT GGT AAA
TGT CAA AGC AGT GGG CAG CGT TGT GTA ATA GAA AAC GGA AAG GCT
GTT TGC AAG GAA AAG TCT GAA GCA ACA ACA GCT GCG ACT ACA ACA
ACG AAA GCG AAA GAC AAG GAT CCA GAT CCT GGA AAG TCA AGT GCT
GCA GCA GTA TCA GCT ACT GGG CTC TTG TTA CTG CTC GCA GCT ACT
TCA GTC ACC GCA GCA TCG TTG TAA GGA AGA TGT CCA ACT TGA ATA
CGG AAC
 ou de parties de cette séquence d'ADN encodant des polypeptides qui induisent une immunité vis-à-vis de l'infestation d'ixodes d'un hôte mammalien auquel on a administré le polypeptide en question, caractérisé en ce que ladite immunité a pour résultat que l'hôte mammalien produit une réponse immunitaire qui endommage la membrane plasmatique des cellules de l'intestin d'ixodes s'alimentant sur ledit hôte avec une ampleur telle que la majorité desdits ixodes ne survivent pas jusqu'à l'age adulte, ou que les ixodes survivants virent au rouge et que le pouvoir reproducteur desdits ixodes survivants soit sensiblement diminué ou que le poids de gonflement desdits ixodes survivants soit diminué et à l'exclusion de la séquence telle qu'elle existe dans son environnement d'origine.

23. Sonde d'hybridation choisie parmi les suivantes :

20-mer 5' T T A C C T G G A T C T G G A T C C T T 3'
50-mer TTA CCA ATG GAT GTA CAA ATA GCT TCA AGG ACA CCA
TCT TCG TAC CAC TT
T
53-mer TTTGGGTACATCATACACCATTCACCAATTGGGTGTTCACAAGCCTGAGG
CTT AC
72-mer 5' TTT AGG TAC AAC CTC ACA TTC AGC ATT CCT ACA AAA
TTC ATT ACC GAA ATC AAA ACA AAT ACT ACT CTC CTT 3'
51-mer 5' CTT CGA CGG ATT GGA TTC GAC GCA TCT GCC ATA GCT
ACA TTC CCT CGT CTT 3'
ou
63-mer 5' CTT GCA ATG GAT TCC ATC CTC GGC GAC AGT GAA AGC
TCT AGG GCA AGT GCA CTC ATA AGC CTT 3'

24. Molécule d'ADN recombinant comprenant au moins une séquence d'ADN suivant l'une quelconque des revendications 20 à 22 et l'ADN vecteur.

25. Molécule d'ADN recombinant suivant la revendication 24, caractérisée en ce que ledit ADN vecteur comprend de l'ADN phagique, viral ou plasmidique.

26. Molécule d'ADN recombinant suivant la revendication 24 ou la revendication 25, caractérisée en ce que ledit ADN vecteur comprend lambda gt11, pUR290, pUR291, pUR282, pUK270, pUC8, pUC9, baculovirus, pZipNeo, un vecteur à base de SV40, αgt10, un vecteur d'EMBL, pBR327, pBR329 ou pBR329 contenant un par locus.

27. pBTA 707.

28. Molécule d'ADN recombinant suivant l'une quelconque des revendication 24 à 27, caractérisée en ce que cette molécule d'ADN recombinant comprend en outre une séquence de commande de l'expression.

29. Molécule d'ADN recombinant suivant la revendication 28, caractérisée en ce que ladite séquence de commande de l'expression comprend un promoteur et un signal d'amorce ou de départ de la traduction.

30. Un transformant comprenant une lignée de cellules d'hôte transformée avec au moins une molécule d'ADN recombinant suivant l'une quelconque des revendications 24 à 29.

31. Transformant suivant la revendication 30, caractérisé en ce que l'hôte précité est constitué par une lignée de cellules bactériennes, de levures, mammalienne ou d'insectes.

32. Transformant suivant la revendication 31, caractérisé en ce que l'hôte transformant est Y 1090, ou Y 1089, ou JM 101.

33. Transformant tel qu'identifié sous le numéro d'accès ATCC 67548.

34. Produit d'expression d'une lignée de cellules d'hôte cultivées in vitro, comprenant un antigène suivant la revendication 1 ou la revendication 2, ou un polypeptide suivant la revendication 14 ou la revendication 15.

35. Vaccin comprenant au moins un antigène ou un homologue suivant l'une quelconque des revendications 1 à 12, ou un polypeptide suivant l'une quelconque des revendications 14 à 16 ou un produit d'expression suivant la revendication 34 et un véhicule ou support, adjuvant, immunopotentialisateur, ou diluant.

36. Vaccin suivant la revendication 35, comprenant WGL⁺ suivant la revendication 1 sous forme glycosylée ou non glycosylée.

37. Anticorps idiotype éveillé contre ou vis-à-vis d'un antigène ou homologue suivant l'une quelconque des revendications 1 à 12, ou un polypeptide suivant l'une quelconque des revendications 14 à 16.

38. Anticorps anti-idiotype éveillé contre ou vis-à-vis d'un anticorps suivant la revendication 37.

39. Vaccin comprenant un anticorps idiotype ou un anticorps anti-idiotype suivant la revendication 37 ou la revendication 38, respectivement.

40. Procédé de préparation d'un antigène ou d'un homologue suivant l'une quelconque des revendications 1 à 12, ou d'un polypeptide suivant l'une quelconque des revendications 14 à 16, lequel procédé se caractérise en ce que l'on soumet ledit antigène, homologue ou ledit polypeptide à une étape chromatographique entreprise sur de la lectine de germes de blé, ou sur une lectine possédant une saccharospécificité terminale identique ou similaire à celle de la lectine de germes de blé, si bien que ledit antigène, homologue, ou polypeptide est lié à ladite lectine et en est ensuite élué.

41. Procédé de préparation d'un antigène ou d'un homologue suivant la revendication 40, caractérisé en ce qu'il comprend l'extraction de fractions membrano-enrichies, obtenues à partir d'ixodes homogénéisés avec du détergent et la soumission de la matière solubilisée à une chromatographie sur sépharose-lectine de germes de blé et l'élution avec de la N-acétylglucosamine, ou à une chromatographie utilisant une lectine possédant une saccharospécificité terminale identique ou similaire à celle de la lectine de germes de blé.

42. Procédé suivant la revendication 41, caractérisé en ce que l'on choisit le détergent parmi le NP40 ou un dérivé de NP40, le Zwittergent 3-14 ou le DSS.

43. Procédé suivant la revendication 42, caractérisé en ce qu'il comprend, en outre, une chromatographie sur sépharose-concanavaline A et l'élution avec du méthyl-a-D-mannopyranoside.

44. Procédé suivant la revendication 43, caractérisé en ce qu'il comprend, en outre, une étape de focalisation iso-électrique de préparation.

45. Procédé suivant la revendication 44, caractérisé en ce qu'il comprend, en outre, une chromatographie à exclusion de calibres.

46. Procédé suivant l'une quelconque des revendications 41 à 45, caractérisé en ce qu'il comprend :
la préparation d'un homogénéisat d'ixodes,
la centrifugation pour produire des fractions membrano-enrichies,
le traitement desdites fractions membrano-enrichies par un détergent,
la chromatographie de la matière soluble dans le détergent sur des colonnes d'affinité vis-à-vis de la lectine de germes de blé, ou sur des colonnes avec une saccharospécificité terminale identique ou similaire à celle de la lectine de germes de blé,
la séparation des antigènes liant la lectine par focalisation iso-électrique dans du détergent contenant un tampon,
la chromatographie desdits antigènes par HPLC à exclusion de calibres dans du détergent contenant un tampon,
la chromatographie des fractions obtenues par HPLC et
l'analyse des fractions résultantes par EGPA-DSS (SDS-PAGE).

47. Procédé suivant la revendication 46, caractérisé en ce que l'on traite lesdites membranes par du Zwittergent 3-14.

48. Procédé suivant la revendication 46 ou la revendication 47, caractérisé en ce que lesdites colonnes de lectine de germes de blé sont des colonnes de sépharose 6B-lectine de germes de blé.

49. Procédé suivant l'une quelconque des revendications 46 à 48, caractérisé en ce que lesdits tampons contiennent du Zwittergent 3-14 à titre de détergent.

50. Procédé suivant l'une quelconque des revendications 46 à 49, caractérisé en ce que ladite HPLC à exclusion de calibres s'effectue en séries sur des colonnes de Bio-Sil Tsk 4000 et PP 300 SW.

51. Antigène produit par mise en oeuvre d'un procédé suivant l'une quelconque des revendications 40 à 50.

52. Procédé de préparation d'une molécule d'ADN recombinant suivant l'une quelconque des revendications 24 à 29, caractérisé en ce qu'il comprend l'insertion d'une séquence d'ADN suivant l'une quelconque des revendications 20 à 22 dans un ADN vecteur.

53. Procédé de préparation d'une lignée de cellules transformante suivant l'une quelconque des revendications 30 à 33, caractérisé en ce qu'il comprend l'insertion d'une molécule d'ADN recombinant suivant l'une quelconque des revendications 24 à 29 dans une lignée de cellules d'hôte.

54. Procédé de biosynthèse d'un antigène, WGL⁺, ou d'une partie ou d'un homologue de celui-ci, qui est capable d'induire une immunité vis-à-vis d'une infestation d'ixodes d'un hôte mammalien auquel ladite partie ou ledit homologue d'antigène a été administré, caractérisé en ce que ladite immunité a pour résultat que l'hôte mammalien produit une réponse immunitaire qui endommage la membrane plasmatique des cellules de l'intestin d'ixodes s'alimentant sur ledit hôte avec une ampleur telle que la majorité desdits ixodes ne survivent pas jusqu'à l'age adulte, ou que les ixodes survivants virent au rouge et que le pouvoir reproducteur desdits ixodes survivants soit sensiblement diminué ou que le poids de gonflement desdits ixodes survivants soit diminué, ou d'un polypeptide suivant l'une quelconque des revendications 14 à 16, procédé caractérisé en ce que l'on fournit une molécule d'ADN recombinant suivant l'une quelconque des revendications 24 à 29, on transforme une lignée de cellules d'hôte à l'aide de ladite molécule d'ADN recombinant en sorte que ledit hôte soit capable d'exprimer un produit protéinique comprenant ledit antigène ou ledit polypeptide, on cultive ledit hôte pour obtenir ladite expression et on recueille ledit produit protéinique.

55. Procédé de biosynthèse d'un antigène suivant la revendication 54, caractérisé en ce que ledit antigène est formé sous forme d'un corps d'inclusion.

56. Antigène LL⁺ isolé, possédant un pI de 4,8 à 5,0, un poids moléculaire de 80 à 90 kD et la composition d'aminoacides ci-dessous :
| | |
|---|---|
| Asp | 11,0 |
| Glu | 10,3 |
| Ser | 7,4 |
| Gly | 10,5 |
| His | 2,9 |
| Arg | 5,2 |
| Thr | 5,6 |
| Ala | 6,8 |
| Pro | 5,2 |
| Tyr | 3,9 |
| Val | 6,5 |
| Met | 2,9 |
| Cys | 0,5 |
| Ile | 4,5 |
| Leu | 8,8 |
| Phe | 4,0 |
| Lys | 3,8 |

57. LL⁺ suivant la revendication 56 sous forme non glycosylée.

58. Produit d'expression d'un hôte transformant transformé à l'aide d'une molécule d'ADN recombinant encodant LL⁺ comme précédemment défini, ledit produit d'expression comprenant LL⁺ sous forme glycosylée ou non glycosylée.

59. Epitope d'un antigène suivant l'une quelconque des revendications 1 à 4, lequel épitope induit l'immunité vis-à-vis d'une infestation d'un hôte mammalien auquel on a administré ledit épitope, caractérisé en ce que ladite immunité a pour résultat que l'hôte mammalien produit une réponse immunitaire qui endommage la membrane plasmatique des cellules de l'intestin d'ixodes s'alimentant sur ledit hôte avec une ampleur telle que la majorité desdits ixodes ne survivent pas jusqu'à l'age adulte, ou que les ixodes survivants virent au rouge et que le pouvoir reproducteur desdits ixodes survivants soit sensiblement diminué ou que le poids de gonflement desdits ixodes survivants soit diminué.

60. Epitope suivant la revendication 59, lorsqu'il est produit par voie synthétique ou par suite d'une scission ou dissociation chimique ou enzymatique de l'antigène.
